# EUROPEAN PATENT SPECIFICATION

(11) **EP 2 324 024 B1**
(45) Date of publication and mention of the grant of the patent: **26.09.2012**
(21) Application number: 09762885.3
(22) Date of filing: 10.06.2009
(51) Int. Cl.: C07D 471/14, A61K 31/519, A61P 35/00

(54) **DIAZACARBAZOLES AND METHODS OF USE**
DIAZACARBAZOLE UND ANWENDUNGSVERFAHREN
DIAZACARBAZOLES ET PROCÉDÉS D'UTILISATION

(30) Priority: 11.06.2008 US 60750 P; 28.01.2009 US 148004 P
(43) Date of publication of application: 25.05.2011
(73) Proprietor: Genentech, Inc., South San Francisco, CA 94080 (US)
(72) Inventor: DYKE, Hazel, Joan, Essex CM195TR (GB); GANCIA, Emanuela, Essex CM195TR (GB); GAZZARD, Lewis, J., Belmont CA 94002 (US); GOODACRE, Simon, Charles, Essex CM195TR (GB); LYSSIKATOS, Joseph, P., Piedmont CA 94611 (US); MACLEOD, Calum, Essex CM195TR (GB); WILLIAMS, Karen, Essex CM195TR (GB); CHEN, Huifen, Burlingame CA 94010 (US)
(74) Representative: Kiddle, Simon John
(86) International application number: PCT/US2009/003481
(87) International publication number: WO 2009/151589

(56) References cited:
- WO-A-01/68648
- WO-A-2007/044779
- WO-A-2009/004329
- US-A1- 2006 264 493
- M. HAYAKAWA ET. AL.: "Synthesis and biological evaluation of pyrido[3',2':4,5]furo[3,2-d]pyrimidine derivatives as novel PI3K kinase p110alpha inhibitors." BIOORGANIC AND MEDICINAL CHEMISTRY LETTERS, vol. 17, 2007, pages 2438-2442, XP002546651
- I. A. BHATTI ET. AL.: "Pyrolysis of 1-substituted pyrazoles and chloroform at 550°C: formation of alpha-carboline from 1-benzylpyrazoles." JOURNAL OF THE CHEMICAL SOCIETY, PERKIN TRANSACTIONS I, vol. 1997, 1997, pages 3581-3585, XP002546649
- R. H. BAKEHAR ET. AL.: "Synthesis of 3,8,9-trisubstituted 1,7,9-triaza-fluorene-6-carboxylic acid derivatives as a new class of insulin secretagogues." BIOORGANIC AND MEDICINAL CHEMISTRY, vol. 15, 2007, pages 5950-5964, XP002546650

## Description

The invention relates to 1,5-diazacarbazole compounds which are useful as kinase inhibitors, more specifically useful as checkpoint kinase 1 (chk1) inhibitors, thus useful as cancer therapeutics. The invention also relates to compositions, more specifically pharmaceutical compositions comprising these compounds and medical uses of the compounds for treating various forms of cancer and hyperproliferative disorders.

Individual cells replicate by making an exact copy of their chromosomes, and then segregating these into separate cells. This cycle of DNA replication, chromosome separation and division is regulated by mechanisms within the cell that maintain the order of the steps and ensure that each step is precisely carried out. Involved in these processes are the cell cycle checkpoints (Hartwell et al., Science, Nov. 3, 1989, 246(4930):629-34) where cells may arrest to ensure DNA repair mechanisms have time to operate prior to continuing through the cycle into mitosis. There are two such checkpoints in the cell cycle - the G1/S checkpoint that is regulated by p53 and the G2/M checkpoint that is monitored by the serine/threonine kinase checkpoint kinase 1 (chk1).

Chk 1 and Chk2 are structurally unrelated yet functionally overlapping serine/threonine kinases activated in response to genotoxic stimuli (reviewed in Bartek et al., Nat. Rev. Mol. Cell Biol. 2001, vol. 2, pp. 877-886). Chk1 and Chk2 relay the checkpoint signals from the ATM and ATR, which phosphorylate and activate them. Chk2 is a stable protein expressed throughout the cell cycle, activated mainly by ATM in response to double-strand DNA breaks (DSBs). In contrast, Chk1 protein expression is largely restricted to S and G2 phases. In response to DNA damage, ChK1 is phosphorylated and activated by ATM/ATR, resulting in cell cycle arrest in the S and G2/M phases to allow for repair of DNA damage (reviewed in Cancer Cell, Bartek and Lukas, Volume 3, Issue 5, May 2003, Pages 421-429. Inhibition of Chk1 has been shown to abrogate cell cycle arrest leading to enhanced tumor cell death following DNA damage by a range of chemotherapeutics. Cells lacking intact G 1 checkpoints are particularly dependent on S and G2/M checkpoints and are therefore expected to be more sensitive to chemotherapeutic treatment in the presence of a chk 1 inhibitor, whereas normal cells with functional G 1 checkpoints would be predicted to undergo less cell death.

WO 2007/044779 describes compounds having activity as AlK inhibitors and c-KIT inhibitors. Bhatti et al (J. Chem. Soc., Perkin Trans. 1, 1997, 3581-3585) describes reactions involving the pyrolysis of 1-substituted pyrazoles. Bahekar et al (Bioorganic & Medicinal Chemistry 15 (2007) 5950-5964) discloses the synthesis of 3,8,9-trisubstituted-1,7,9-triaza-fluorene-6-carboxylic acid derivatives and their properties as insulin secretagogues. WO 01/68648 describes substituted beta-carbolines as IKB-kinase inhibitors. US 2006/0264493 discloses tetracyclic pyrazole derivatives. WO 2009/004329 relates to 9H-pyrimido[4,5-b]indoles, 9H-pyrido[4',3':4,5]pyrrolo[2,3-d)pyridine compounds Hayakawa et al (Bioorganic & Medicinal Chemistry Letters, 17 (2007) 2438-2442) discloses the synthesis and biological evaluation ofpyrido[3',2':4,5]furo[3,2-d]pyrimidine derivatives as novel PI3 kinase p110α inhibitors.

The invention relates generally to 1,5-diazacarbazoles of Formula (**I**), (**I-a)**, (**I-b**), (**I-c**), (**I-d**), (**I-e**), (**I-f**), (**I-g**) and (**I-h**) (and/or solvates, hydrates and/or salts thereof) with kinase inhibitory activity, more specifically with chk1 inhibitory activity. The compounds of the present invention are useful as inhibitors of Glycogen Synthase Kinase 3 (GSK-3), KDR kinase, and FMS-like tyrosine kinase 3 (FLT3). Accordingly, the compounds of the invention and compositions thereof are useful in the treatment of hyperproliferative disorders such as cancer. 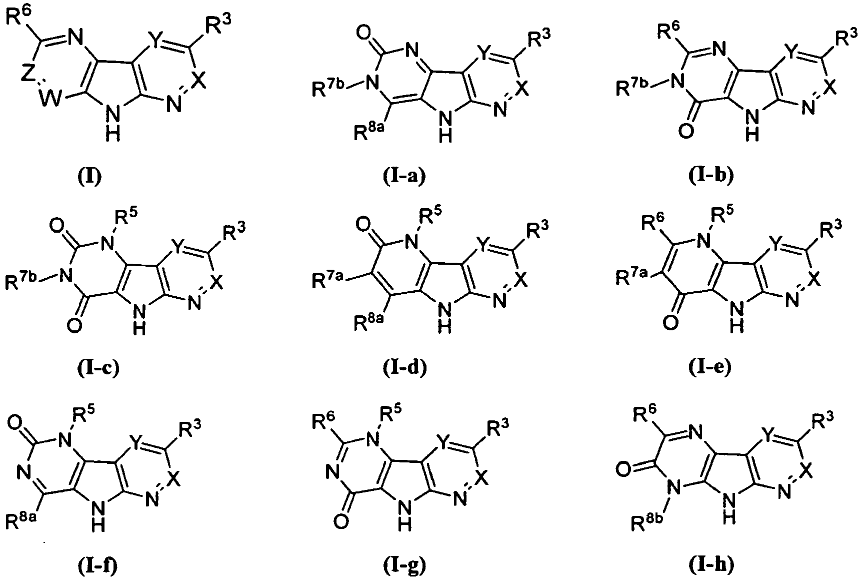
- X: is CR² or N;
- Y: is CR⁴ or N;
- Z: is CR^{7a} or N;
- W: is CR^{8a} or N; provided that (i) Z and W are not both N at the same time and (ii) X and Y are not both N at the same time;
- R²: is H, halo, CN, CF₃, -OCF₃, OH, -NO_{2,} C₁-C₅ alkyl, -O(C₁-C₅ alkyl), -S(C₁-C₅ alkyl), or N(R²²)₂;
- R³: is H, halo, -O-R⁹, -N(R²²)-R⁹, - S(O)ₚ-R⁹, or R⁹; p is 0, 1 or 2;
- R⁴: is H, halo, CN, CF₃, -OCF₃, OH, -NO₂, -(CR¹⁴R¹⁵)ₙC(=Y')OR¹¹, -(CR¹⁴R¹⁵)ₙC(=Y')NR¹¹R¹², -(CR¹⁴R¹⁵)ₙNR¹¹R¹², -(CR¹⁴R¹⁵)ₙOR¹¹, -(CR¹⁴R¹⁵)ₙS(O)ₚR¹¹, -(CR¹⁴R¹⁵)ₙNR¹²C(=Y')R¹¹, -(CR¹⁴R¹⁵)ₙNR¹²C(=Y')OR¹¹, -(CR¹⁴R¹⁵)ₙNR¹²C(=Y')NR¹¹R¹², -(CR¹⁴R¹⁵)ₙNR¹²SO₂R¹¹, -(CR¹⁴R¹⁵)ₙOC(=Y')R¹¹, -(CR¹⁴R¹⁵)ₙOC(=Y')NR¹¹R¹², -(CR¹⁴R¹⁵)ₙS(O)₂NR¹¹R¹², C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, or 5- or 6-membered heteroaryl wherein said alkyl, alkenyl, alkynyl, cycloalkyl, heterocyclyl, aryl and heteroaryl are optionally substituted with one to four R¹³ groups;
- each n: is independently 0-5;
- R⁵: is H, CN, CF₃, OH, -(CR¹⁴R¹⁵)ₙC(=Y')OR¹¹, -(CR¹⁴R¹⁵)ₙC(=Y')NR¹¹R¹², -(CR¹⁴R¹⁵)nNR¹²C(=Y')R¹¹, -(CR¹⁴R¹⁵)ₙNR¹¹R¹², -(CR¹⁴R¹⁵)ₙOR¹¹, -(CR¹⁴R¹⁵)ₙS(O)ₚR¹¹, -(CR¹⁴R¹⁵)ₙNR¹²C(=Y')OR¹¹, -(CR¹⁴R¹⁵)ₙNR¹²C(=Y')NR¹¹R¹², -(CR¹⁴R¹⁵)ₙNR¹²SO₂R¹¹, -(CR¹⁴R¹⁵)ₙOC(=Y')R¹¹, -(CR¹⁴R¹⁵)ₙOC(=Y')NR¹¹R¹², -(CR¹⁴R¹⁵)ₙS(O)₂NR¹¹R¹², C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, 5- or 6-membered heteroaryl wherein the said alkyl, alkenyl, alkynyl, cycloalkyl, heterocyclyl, aryl and heteroaryl are optionally substituted with one to four R¹³ groups;
- R⁶: is H, CN, -CF₃, -OCF₃, halo, -C(=Y')OR¹¹, -C(=Y')NR¹¹R¹², -OR¹¹, -OC(=Y')R¹¹, -NR¹¹R¹², -NR¹²C(=Y')R¹¹, -NR¹²C(=Y')NR¹¹R¹², -NR¹²S(O)_{q}R¹¹, -SR¹¹, -S(O)R¹¹, -S(O)₂R¹¹, -OC(=Y')NR¹¹R¹², -S(O)₂NR¹¹R¹², -S(O)₂(OR¹¹), -SC(=Y')R", -SC(=Y')OR¹¹, -SC(=Y')NR¹¹R¹², C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, or 5- or 6-membered heteroaryl wherein said alkyl, alkenyl, alkynyl, cycloalkyl, heterocyclyl, aryl and heteroaryl are optionally substituted by one to four R¹³ groups;
- R^{7a}: is H, halo, -CN, -OH, -NO₂, -O(C₁-C₃ alkyl), or C₁-C₄ alkyl, wherein each said alkyl is optionally substituted with one to three groups selected from halo, N(R²²)₂ or OR²²;
- R^{7b}: is H, -OH, -CN, -O(C₁-C₃ alkyl), or C₁-C₄ alkyl, wherein each said alkyl is optionally substituted with one to three groups selected from halo, N(R²²)₂ or OR²²;
- R^{8a}: is H, halo, -CN, -NO₂, -N(R²²)₂, -OH, -O(C₁-C₃ alkyl), or C₁-C₃ alkyl, wherein each said alkyl is optionally substituted with one to three halo groups;
- R^{8b}: is H, -OH, -CN, -O(C₁-C₃ alkyl), or C₁-C₃ alkyl, wherein each said alkyl is optionally substituted with one to three halo groups;
- each R⁹: is independently C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, 5- or 6-membered heteroaryl, wherein each member of R⁹ is independently substituted with one to three R¹⁰ groups;
- each R¹⁰: is independently H, CN, -CF₃, -OCF₃, -NO₂, halo, R", -OR¹¹, -NR¹²C(=Y')R¹¹, -NR¹²C(=NR¹²)R¹¹, -NR¹²S(O)_{q}R¹¹, -SR¹¹, -NR¹¹R¹², oxo, -C(=Y')OR¹¹, -C(=Y')NR¹¹R¹², -S(O)_{q}R¹¹, -NR¹²C(=Y')OR¹¹, -NR¹²C(=Y')NR¹¹R¹², -OC(=Y')R¹¹, -OC(=Y')NR¹¹R¹², or -S(O)₂NR¹¹R¹²;
- each q: independently is 1 or 2;
- R¹¹ and R¹²: are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R¹³ groups, wherein two geminal R¹³ groups are optionally taken together with the atom to which they are attached to form a 3-6 membered ring having additional 0-2 heteroatoms selected from O, S, and N, said ring being optionally substituted with one to four R¹⁸ groups;
- R¹¹ and R¹²: are optionally taken together with the attached N atom to form a 4-7 membered ring having additional 0-2 heteroatoms selected from O, S, and N, said ring being optionally substituted with one to four R¹³ groups;
- each R¹³: is independently halo, CN, CF₃, -OCF₃, -NO₂, oxo, -(CR¹⁴R¹⁵)ₙC(=Y')R¹⁶, -(CR¹⁴R¹⁵)ₙC(=Y')OR¹⁶, -(CR¹⁴R¹⁵)ₙC(=Y')NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙSR¹⁶, -(CR¹⁴R¹⁵)ₙNR¹⁶C(=Y')R¹⁷, -(CR¹⁴R¹⁵)ₙNR¹⁶C(=Y')OR¹⁷, -(CR¹⁴R¹⁵)ₙNR¹⁷C(=Y')NR¹⁶R¹⁷, -(CR¹⁴R¹⁵)ₙNR¹⁷SO₂R¹⁶, -(CR¹⁴R¹⁵)ₙOC(=Y')R¹⁶, -(CR¹⁴R¹⁵)ₙOC(=Y')NR¹⁶R¹⁷, -(CR¹⁴R¹⁵)ₙS(O)R¹⁶, -(CR¹⁴R¹⁵)ₙS(O)₂R¹⁶, -(CR¹⁴R¹⁵)ₙS(O)₂NR¹⁶R¹⁷, or R¹⁶;
- R¹⁴: and R¹⁵ are independently selected from H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R¹⁸ groups;
- R¹⁶ and R¹⁷: are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R¹⁸ groups;
- R¹⁶ and R¹⁷: are optionally taken together with the attached N atom to form a 5-6 membered ring having additional 0-2 heteroatoms selected from O, S, and N, said ring being optionally substituted with one to four R¹⁸ groups;
- each R¹⁸: is independently H, C₁-C₁₂-alkyl, C₃-C₁₂-cycloalkyl, heterocyclyl monocycle having 3 to 7 ring members or a bicycle having 6 to 10 ring members, aryl, 5- or 6-membered heteroaryl, halo, CN, CF₃, -OCF₃, -NO₂, oxo, -(CR¹⁹R²⁰)ₙ C(=Y')R²³, -(CR¹⁹R²⁰)ₙC(=Y')OR²³, -(CR¹⁹R²⁰)ₙ C(=Y')NR²³R²⁴, -(CR¹⁹R²⁰)ₙNR²³R²⁴, -(CR¹⁹R²⁰)ₙOR²³, -(CR¹⁹R²⁰)ₙ-SR²³, -(CR¹⁹R²⁰)ₙNR²⁴C(=Y')R²³, -(CR¹⁹R²⁰)ₙ NR²⁴C(=Y')OR²³, -(CR¹⁹R²⁰)ₙNR¹²C(=Y')NR²³R²⁴, -(CR¹⁹R²⁰)ₙNR²⁴SO₂R²³, -(CR¹⁹R²⁰)ₙOC(-Y')R²³, -(CR¹⁹R²⁰)ₙ OC(=Y')NR²¹R²¹, -(CR¹⁹R²⁰)ₙS(O)R²³, -(CR¹⁹R²⁰)ₙS(O)₂R²³, or -(CR¹⁹R²⁰)ₙS(O)₂NR²³R²⁴, wherein said alkyl, cycloalkyl, heterocyclyl, aryl, and heteroaryl are optionally substituted with one to four R²¹ groups;
- R¹⁹ and R²⁰: are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R²⁵ groups;
- R²³ and R²⁴: are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R²¹ groups;
- R²³ and R²⁴: are optionally taken together with the attached N atom to form a 5-6 membered ring having additional 0-2 heteroatoms selected from O, S, and N, said ring being optionally substituted with one to four R²¹ groups;
- each R²¹: is independently H, C₁-C₁₂-alkyl, C₃-C₁₂-cycloalkyl, heterocyclyl monocycle having 3 to 7 ring members or a bicycle having 6 to 10 ring members, aryl, 5- or 6-membered heteroaryl, halo, CN, CF₃, -OCF₃, -NO₂, oxo, -C(=Y')R²⁵, -C(=Y')OR²⁵, -C(=Y')NR²⁵R²⁶, -NR²⁵R²⁶, -OR²⁵, -SR²⁵, -NR²⁶C(=Y')R²⁵, -NR²⁶C(=Y')OR²⁵, -NR²²C(=Y')NR²⁵R²⁶, -NR²⁶SO₂R²⁵, -OC(=Y')R²⁵, -OC(=Y')NR²⁵R²⁶, -S(O)R²⁵, -S(O)₂R²⁵, or -S(O)₂NR²⁵R²⁶, wherein said alkyl, cycloalkyl, heterocyclyl, aryl, and 5- or 6-membered heteroaryl are optionally substituted with one to four R²⁵ groups;
- each R²⁵ and R²⁶: is independently H, C₁-C₁₂-alkyl, C₃-C₁₂-cycloalkyl, heterocyclyl monocycle having 3 to 7 ring members or a bicycle having 6 to 10 ring members, aryl, or 5- or 6- membered heteroaryl, wherein said alkyl, cycloalkyl, heterocyclyl, aryl, or heteroaryl is optionally substituted with one to four groups selected from halo, -CN, -OCF₃, -CF₃, -NO₂, -C₁-C₆ alkyl, -OH, oxo, -SH, -O(C₁-C₆ alkyl), -S(C₁-C₆ alkyl), -NH₂, -NH(C₁-C₆ alkyl), -N(C₁-C₆ alkyl)₂, -SO₂(C₁-C₆ alkyl), -CO₂H, -CO₂(C₁-C₆ alkyl), -C(O)NH₂, -C(O)NH(C₁-C₆ alkyl), -C(O)N(C₁-C₆ alkyl, -N(C₁-C₆ alkyl)C(O)(C₁-C₆ alkyl), -NHC(O)(C₁-C₆ alkyl), -NHSO₂(C₁-C₆ alkyl), -N(C₁-C₆ alkyl)SO₂(C₁-C₆ alkyl), -SO₂NH₂, -SO₂NH(C₁-C₆ alkyl), -SO₂N(C₁-C₆ alkyl, -OC(O)NH₂, -OC(O)NH(C₁-C₆ alkyl), -OC(O)N(C₁-C₆ alkyl)₂, -NHC(O)NH(C₁-C₆ alkyl), -NHC(O)N(C₁-C₆ alkyl)₂, -N(C₁-C₆ alkyl)C(O)NH(C₁-C₆ alkyl), -N(C₁-C₆ alkyl)C(O)N(C₁-C₆ alkyl, -NHC(O)NH(C₁-C₆ alkyl), -NHC(O)N(C₁-C₆ alkyl)₂, -NHC(O)O(C₁-C₆ alkyl), and -N(C₁-C₆ alkyl)C(O)O(C₁-C₆ alkyl);
- R²⁵ and R²⁶: are optionally taken together with the attached N atom to form a 5-6 membered ring having additional 0-2 heteroatoms selected from O, S, and N, said ring being optionally substituted with one to four groups selected from halo, -CN, -OCF₃, -CF₃, -NO₂, -C₁-C₆ alkyl, -OH, oxo, -SH, -O(C₁-C₆ alkyl), -S(C₁-C₆ alkyl), -NH₂, -NH(C₁-C₆ alkyl), -N(C₁-C₆ alkyl)₂, -SO₂(C₁-C₆ alkyl), -CO₂H, -CO₂(C₁-C₆ alkyl), -C(O)NH₂, -C(O)NH(C₁-C₆ alkyl), -C(O)N(C₁-C₆ alkyl, -N(C₁-C₆ alkyl)C(O)(C₁-C₆ alkyl), -NHC(O)(C₁-C₆ alkyl), -NHSO₂(C₁-C₆ alkyl), -N(C₁-C₆ alkyl)SO₂(C₁-C₆ alkyl), -SO₂NH₂, -SO₂NH(C₁-C₆ alkyl), -SO₂N(C₁-C₆ alkyl)₂, -OC(O)NH₂, -OC(O)NH(C₁-C₆ alkyl), -OC(O)N(C₁-C₆ alkyl)₂, -NHC(O)NH(C₁-C₆ alkyl), -NHC(O)N(C₁-C₆ alkyl)₂, -N(C₁-C₆ alkyl)C(O)NH(C₁-C₆ alkyl), -N(C₁-C₆ alkyl)C(O)N(C₁-C₆ alkyl)₂, -NHC(O)NH(C₁-C₆ alkyl), -NHC(O)N(C₁-C₆ alkyl)₂, -NHC(O)O(C₁-C₆ alkyl), and -N(C₁-C₆ alkyl)C(O)O(C₁-C₆ alkyl);
- Y': is independently O, NR²², or S; and
- each R²²: is independently H or C₁-C₅ alkyl.

The present invention includes a composition (e.g., a pharmaceutical composition) comprising a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) and a carrier (a pharmaceutically acceptable carrier). The present invention also includes a composition (e.g., a pharmaceutical composition) comprising a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) and a carrier (a pharmaceutically acceptable carrier), further comprising a second chemotherapeutic agent. The present compositions are therefore useful for inhibiting abnormal cell growth or treating a hyperproliferative disorder in a mammal (e.g., human), such as cancer.

The present invention includes medical uses of the compounds in methods of inhibiting abnormal cell growth or treating a hyperproliferative disorder in a mammal (e.g., human) such as cancer comprising administering to said mammal a therapeutically effective amount of a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) or a composition thereof, alone or in combination with a second chemotherapeutic agent.

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described. In the event that one or more of the incorporated literature, patents, and similar materials differs from or contradicts this application, including but not limited to defined terms, term usage, described techniques, or the like, this application controls.

The term "alkyl" as used herein refers to a saturated linear or branched-chain monovalent hydrocarbon radical of one to twelve carbon atoms. Examples of alkyl groups include, but are not limited to, methyl (Me, -CH₃), ethyl (Et, -CH₂CH₃), 1-propyl (*n*-Pr, *n*-propyl, -CH₂CH₂CH₃), 2-propyl (*i*-Pr, *i*-propyl, -CH(CH₃)₂), 1-butyl (*n-*Bu, *n*-butyl, -CH₂CH₂CH₂CH₃), 2-methyl-1-propyl (*i*-Bu, *i*-butyl, -CH₂CH(CH₃)₂), 2-butyl (*s*-Bu, *s*-butyl, -CH(CH₃)CH₂CH₃), 2-methyl-2-propyl (*t*-Bu, *t*-butyl, -C(CH₃)₃), 1-pentyl (*n*-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂ CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl, 1-octyl, and the like.

The term "alkenyl" refers to linear or branched-chain monovalent hydrocarbon radical of two to twelve carbon atoms with at least one site of unsaturation, i.e., a carbon-carbon, sp² double bond, wherein the alkenyl radical includes radicals having "cis" and "trans" orientations, or alternatively, "*E*" and "*Z*" orientations. Examples include, but are not limited to, ethylenyl or vinyl (-CH=CH₂), allyl (-CH₂CH=CH₂), and the like.

The term "alkynyl" refers to a linear or branched monovalent hydrocarbon radical of two to twelve carbon atoms with at least one site of unsaturation, i.e., a carbon-carbon, sp triple bond. Examples include, but are not limited to, ethynyl (-C≡CH), propynyl (propargyl, -CH₂C≡CH), and the like.

The term "cycloalkyl" refers to a monovalent non-aromatic, saturated or partially unsaturated ring having 3 to 12 carbon atoms as a monocyclic ring or 6 to 12 carbon atoms as a bicyclic ring. Bicyclic carbocycles having 6 to 12 atoms can be arranged, for example, as a bicyclo [4,5], [5,5], [5,6] or [6,6] system, and bicyclic carbocycles having 9 or 10 ring atoms can be arranged as a bicyclo [5,6] or [6,6] system, or as bridged systems such as bicyclo[2.2.1]heptane, bicyclo[2.2.2]octane and bicyclo[3.2.2]nonane. Examples of monocyclic carbocycles include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl, and the like.

"Aryl" means a monovalent aromatic hydrocarbon radical of 6-14 carbon atoms derived by the removal of one hydrogen atom from a single carbon atom of a parent aromatic ring system. Some aryl groups are represented in the exemplary structures as "Ar". Aryl includes bicyclic radicals comprising an aromatic ring fused to a saturated, partially unsaturated ring, or aromatic carbocyclic or heterocyclic ring. Typical aryl groups include, but are not limited to, radicals derived from benzene (phenyl), substituted benzenes, naphthalene, anthracene, indenyl, indanyl, 1,2-dihydronapthalene, 1,2,3,4-tetrahydronapthyl, and the like.

The terms "heterocycle," "heterocyclyl" and "heterocyclic ring" are used interchangeably herein and refer to a saturated or a partially unsaturated (i.e., having one or more double bonds within the ring) carbocyclic radical of 3 to 14 ring atoms in which at least one ring atom is a heteroatom selected from nitrogen, oxygen and sulfur, the remaining ring atoms being C, where one or more ring atoms is optionally substituted independently with one or more substituents described below. A heterocycle may be a monocycle having 3 to 7 ring members (2 to 6 carbon atoms and 1 to 4 heteroatoms selected from N, O, and S) or a bicycle having 6 to 10 ring members (4 to 9 carbon atoms and 1 to 6 heteroatoms selected from N, O, and S), for example: a bicyclo [4,5], [5,5], [5,6], or [6,6] system or a bridged [2.1.1], [2.2.1], [2.2.2] or [3.2.2] system. Heterocycles are described in Paquette, Leo A.; "Principles of Modem Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. (1960) 82:5566. "Heterocyclyl" also includes radicals where heterocycle radicals are fused with a saturated, partially unsaturated ring, or aromatic carbocyclic or heterocyclic ring. Examples of heterocyclic rings include, but are not limited to, pyrrolidinyl, tetrahydrofuranyl, dihydrofuranyl, tetrahydrothienyl, tetrahydropyranyl, dihydropyranyl, tetrahydrothiopyranyl, piperidinyl, morpholinyl, thiomorpholinyl, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, pyrazolidinylimidazolinyl, imidazolidinyl, 3-azabicyco[3.1.0]hexanyl, 3-azabicyclo[4.1.0]heptanyl, and azabicyclo[2.2.2]hexanyl. Spiro moieties are also included within the scope of this definition. Examples of a heterocyclic group wherein ring atoms are substituted with oxo (=O) moieties are pyrimidinonyl and 1,1-dioxo-thiomorpholinyl.

The term "heteroaryl" refers to a monovalent aromatic radical of 5- or 6- membered rings, and includes fused ring systems (at least one of which is aromatic) of 5-16 atoms, containing one or more heteroatoms independently selected from nitrogen, oxygen, and sulfur. Examples of heteroaryl groups are pyridinyl (including, for example, 2-hydroxypyridinyl), imidazolyl, imidazopyridinyl, pyrimidinyl (including, for example, 4-hydroxypyrimidinyl), pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl.

The heterocycle or heteroaryl groups may be carbon (carbon-linked) or nitrogen (nitrogen-linked) attached where such is possible. By way of example and not limitation, carbon bonded heterocycles or heteroaryls are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiophene, tetrahydrothiophene, pyrrole or pyrrolidine, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline.

By way of example and not limitation, nitrogen bonded heterocycles or heteroaryls are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, 2-oxo-1,2-dihydropyridine, or 4-oxo-1,4-dihydropyridine; position 2 of a isoindole, or isoindoline; position 4 of a morpholine; and position 9 of a carbazole, or β-carboline.

The term "halo" refers to F, Cl, Br or I. The heteroatoms present in heteroaryl or heterocyclyl include the oxidized forms such as N⁺ →O⁻, S(O) and S(O)₂.

The terms "treat" and "treatment" refer to both therapeutic treatment and prophylactic or preventative measures, wherein the object is to prevent or slow down (lessen) an undesired physiological change or disorder, such as the development or spread of cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The phrase "therapeutically effective amount" means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can be measured, for example, by assessing the time to disease progression (TTP) and/or determining the response rate (RR).

The terms "abnormal cell growth" and "hyperproliferative disorder" are used interchangeably in this application. "Abnormal cell growth", as used herein, unless otherwise indicated, refers to cell growth that is independent of normal regulatory mechanisms. This includes, for example, the abnormal growth of: (1) tumor cells (tumors) that proliferate by expressing a mutated tyrosine kinase or overexpression of a receptor tyrosine kinase; (2) benign and malignant cells of other proliferative diseases in which aberrant tyrosine kinase activation occurs; (3) any tumors that proliferate by receptor tyrosine kinases; (4) any tumors that proliferate by aberrant serine/threonine kinase activation; and (5) benign and malignant cells of other proliferative diseases in which aberrant serine/threonine kinase activation occurs.

The terms "cancer" and "cancerous" refer to or describe the physiological condition in mammals that is typically characterized by unregulated cell growth. A "tumor" comprises one or more cancerous cells. Tumors include solid and liquid tumors. Examples of cancer include, but are not limited to, carcinoma, lymphoma, blastoma, sarcoma, myeloma, and leukemia or lymphoid malignancies. More particular examples of such cancers include squamous cell cancer (e.g., epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, malignant brain tumors, melanoma, endometrial or uterine carcinoma, salivary gland carcinoma, kidney or renal cancer, prostate cancer, vulval cancer, thyroid cancer, hepatic carcinoma, anal carcinoma, penile carcinoma, head and neck cancer, as well as acute myelogenous leukemia (AML).

A "chemotherapeutic agent" is a chemical compound useful in the treatment of cancer. Examples of chemotherapeutic agents include Erlotinib (TARCEVA®, Genentech/OSI Pharm.), Bortezomib (VELCADE®, Millennium Pharm.), Fulvestrant (FASLODEX®, AstraZeneca), Sutent (SU11248, Pfizer), Letrozole (FEMARA®, Novartis), Imatinib mesylate (GLEEVEC®, Novartis), PTK787/ZK 222584 (Novartis), Oxaliplatin (Eloxatin®, Sanofi), Leucovorin, Rapamycin (Sirolimus, RAPAMUNE®, Wyeth), Lapatinib (TYKERB®, GSK572016, Glaxo Smith Kline), Lonafamib (SCH 66336), Sorafenib (BAY43-9006, Bayer Labs), and Gefitinib (IRESSA®, AstraZeneca), AG1478, AG1571 (SU 5271; Sugen), alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; bestrabucil; bisantrene; edatraxate; defofamine; demecolcine; diaziquone; elfomithine; elliptinium acetate; an epothilone; etoglucid; gallium nitrate; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; dacarbazine; mannomustine; mitobronitol; mitolactol; pipobroman; gacytosine; arabinoside ("Ara-C"); chloranmbucil; 6-thioguanine; mercaptopurine; ifosfamide; mitoxantrone; novantrone; edatrexate; daunomycin; aminopterin; capecitabine (XELODA®); ibandronate; CPT-11; difluoromethylornithine (DMFO); and pharmaceutically acceptable salts, acids and derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY 117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) antiandrogens such as flutamide, nilutamide, bicalutamide, leuprolide, and goserelin; as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN® rIL-2; a topoisomerase 1 inhibitor such as LURTOTECAN®; ABARELIX® rmRH; (ix) anti-angiogenic agents such as bevacizumab (AVASTIN®, Genentech); and (x) pharmaceutically acceptable salts, acids and derivatives of any of the above.

Other examples of "chemotherapeutic agents" that can be used in combination with the present compounds include inhibitors of MEK (MAP kinase kinase), such as XL518 (Exelixis, Inc.) and AZD6244 (Astrazeneca); inhibitors of Raf, such as XL281 (Exelixis, Inc.), PLX4032 (Plexxikon), and ISIS5132 (Isis Pharmaceuticals); inhibitors of mTor (mammalian target of rapamycin), such as rapamycin, AP23573 (Ariad Pharmaceuticals), temsirolimus (Wyeth Pharmaceuticals) and RAD001 (Novartis); inhibitors of PI3K (phosphoinositide-3 kinase), such as SF-1126 (PI3K inhibitor, Semafore Pharmaceuticals), BEZ-235 (PI3K inhibitor, Novartis), XL-147 (PI3K inhibitor, Exelixis, Inc.), and GDC-0941 (Genentech); inhibitors of cMet, such as PHA665752 (Pfizer), XL-880 (Exelixis, Inc.), ARQ-197 (ArQule), and CE-355621; and pharmaceutically acceptable salts, acids and derivatives of any of the above.

Examples of a "chemotherapeutic agent" also include a DNA damaging agent such as thiotepa and CYTOXAN® cyclosphosphamide; alkylating agents (for example cis-platin; carboplatin; cyclophosphamide; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; busulphan; nitrosoureas such as carmustine, chlorozotocin, fotemustine, lomustine, nimustine, and ranimnustine; and temozolomide); antimetabolites (for example antifolates such as fluoropyrimidines like 5-fluorouracil (5-FU) and tegafur, raltitrexed, methotrexate, cytosine arabinoside, hydroxyurea and GEMZAR® (gemcitabine); antitumour antibiotics such as the enediyne antibiotics (e.g., calicheamicin, especially calicheamicin gammaII and calicheamicin omegaI1 (Angew Chem. Intl. Ed. Engl. (1994) 33:183-186); anthracyclines like adriamycin; dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin, epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, and zorubicin; antimitotic agents (for example vinca alkaloids like vincristine, vinblastine, vindesine and NAVELBINE® (vinorelbine) and taxoids like taxoids, e.g., TAXOL® (paclitaxel; Bristol-Myers Squibb Oncology, Princeton, N.J.), ABRAXANE™ (Cremophor-free), albumin-engineered nanoparticle formulations of paclitaxel (American Pharmaceutical Partners, Schaumberg, Illinois), and TAXOTERE® (doxetaxel; Rhône-Poulenc Rorer, Antony, France); topoisomerase inhibitors (for example RFS 2000, epipodophyllotoxins like etoposide and teniposide, amsacrine, a camptothecin (including the synthetic analog topotecan), and irinotecan and SN-38) and cytodifferentiating agents (for example retinoids such as all-trans retinoic acid, 13-cis retinoic acid and fenretinide); and pharmaceutically acceptable salts, acids and derivatives of any of the above.

A "chemotherapeutic agent" also includes an agent that modulates the apoptotic response including inhibitors of IAP (inhibitor of apoptosis proteins) such as AEG40826 (Aegera Therapeutics); and inhibitors of bcl-2 such as GX15-070 (Gemin X Biotechnologies), CNDO103 (Apogossypol; Coronado Biosciences), HA14-1 (ethyl 2-amino-6-bromo-4-(1-cyano-2-ethoxy-2-oxoethyl)-4*H*-chromene-3-carboxylate), AT101 (Ascenta Therapeutics), ABT-737 and ABT-263 (Abbott); and pharmaceutically acceptable salts, acids and derivatives of any of the above.

The term "prodrug" as used in this application refers to a precursor or derivative form of a compound of the invention that is capable of being enzymatically or hydrolytically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, ester-containing prodrugs, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs, optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, compounds of the invention and chemotherapeutic agents such as described above.

A "metabolite" is a product produced through metabolism in the body of a specified compound or salt thereof. Metabolites of a compound may be identified using routine techniques known in the art and their activities determined using tests such as those described herein. Such products may result for example from the oxidation, hydroxylation, reduction, hydrolysis, amidation, deamidation, esterification, deesterification, enzymatic cleavage, and the like, of the administered compound. Accordingly, the invention includes metabolites of compounds of the invention, including compounds produced by a process comprising contacting a compound of this invention with a mammal for a period of time sufficient to yield a metabolic product thereof.

A "liposome" is a small vesicle composed of various types of lipids, phospholipids and/or surfactant which is useful for delivery of a drug (such as chk inhibitors disclosed herein and, optionally, a chemotherapeutic agent) to a mammal. The components of the liposome are commonly arranged in a bilayer formation, similar to the lipid arrangement of biological membranes.

The term "package insert" is used to refer to instructions customarily included in commercial packages of therapeutic products, that contain information about the indications, usage, dosage, administration, contraindications and/or warnings concerning the use of such therapeutic products.

The term "chiral" refers to molecules which have the property of non-superimposability of the mirror image partner, while the term "achiral" refers to molecules which are superimposable on their mirror image partner.

The term "stereoisomer" refers to compounds which have identical chemical constitution and connectivity, but different orientations of their atoms in space that cannot be interconverted by rotation about single bonds.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as crystallization, electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or *R* and *S*, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons. For example, any reference to a structure of 2-hydroxypyridine include its tautomer 2-oxo-1,2-dihydropyridine, also known as 2-pyridone, and vice versa.

The phrase "pharmaceutically acceptable salt" as used herein, refers to pharmaceutically acceptable organic or inorganic salts of a compound of the invention. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate", ethanesulfonate, benzenesulfonate, *p*-toluenesulfonate, pamoate (i.e., 1,1'-methylene-*bis*-(2-hydroxy-3-naphthoate)) salts, alkali metal (e.g., sodium and potassium) salts, alkaline earth metal (e.g., magnesium) salts, and ammonium salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt may be prepared by any suitable method available in the art, for example, treatment of the free base with an inorganic acid, such as hydrochloric acid, hydrobromic acid, sulfuric acid, nitric acid, phosphoric acid and the like, or with an organic acid, such as acetic acid, methanesulfonic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as *p*-toluenesulfonic acid or ethanesulfonic acid, or the like.

If the compound of the invention is an acid, the desired pharmaceutically acceptable salt may be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include, but are not limited to, organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

The phrase "pharmaceutically acceptable" indicates that the substance or composition must be compatible chemically and/or toxicologically, with the other ingredients comprising a formulation, and/or the mammal being treated therewith.

A "solvate" refers to an association or complex of one or more solvent molecules and a compound of the invention. Examples of solvents that form solvates include, but are not limited to, water, isopropanol, ethanol, methanol, DMSO, ethyl acetate, acetic acid, and ethanolamine. The term "hydrate" refers to the complex where the solvent molecule is water.

The term "protecting group" refers to a substituent that is commonly employed to block or protect a particular functionality while reacting other functional groups on the compound. For example, an "amino-protecting group" is a substituent attached to an amino group that blocks or protects the amino functionality in the compound. Suitable amino-protecting groups include acetyl, trifluoroacetyl, t-butoxycarbonyl (BOC), benzyloxycarbonyl (CBZ), 2-(trimethylsilyl)ethoxymethyl (SEM) and 9-fluorenylmethylenoxycarbonyl (Fmoc). Similarly, a "hydroxy-protecting group" refers to a substituent of a hydroxy group that blocks or protects the hydroxy functionality. Suitable protecting groups include acetyl and *t-*butyldimethylsilyl. A "carboxy-protecting group" refers to a substituent of the carboxy group that blocks or protects the carboxy functionality. Common carboxy-protecting groups include phenylsulfonylethyl, cyanoethyl, 2-(trimethylsilyl)ethyl, 2-(trimethylsilyl)ethoxymethyl, 2-(*p-*toluenesulfonyl)ethyl, 2-(*p*-nitrophenylsulfenyl)ethyl, 2-(diphenylphosphino)-ethyl, nitroethyl and the like. For a general description of protecting groups and their use, see T. W. Greene, Protective Groups in Organic Synthesis, John Wiley & Sons, New York, 1991.

The terms "compound of this invention," and "compounds of the present invention", and "compounds of Formula (**I**), (**I-a**), (**I-b**), (**I-c**), (**I-d**), (**I-e**), (**I-f**), (**I-g**) or (**I-h**)", "compounds of Formula (**I**), (**I-a**), (**I-b**), (**I-c**), (**I-d**), (**I-e**), (**1-f**), (**I-g**) and/or (**I-h**)", unless otherwise indicated, include compounds of Formula (**I**), (**I-a**), (**I-b**), (**I-c**), (**I-d**), (**I-e**), (**I-f**), (**I-g**) and/or (**I-h**), and stereoisomers, geometric isomers, tautomers, solvates, metabolites, salts (e.g., pharmaceutically acceptable salts) and prodrugs thereof. Unless otherwise stated, structures depicted herein are also meant to include compounds that differ only in the presence of one or more isotopically enriched atoms. For example, compounds of Formula (**I**), (**I-a**), (**I-b**), (**I-c**), (**I-d**), (**I-e**), (**I-f**), (**I-g**) or (**I-h**), wherein one or more hydrogen atoms are replaced deuterium or tritium, or one or more carbon atoms are replaced by a ¹³C- or ¹⁴C-enriched carbon are within the scope of this invention.

The present invention provides 1,5-diazacarbazoles of Formula (**I**), (**I-a**), (**I-b**), (**I-c**), (**I-d**), (**I-e**), (**I-f**), (**I-g**) and/or (**I-h**) (and/or solvates, hydrates and/or salts thereof) as described above with kinase inhibitory activity, such as chk1, GSK-3, KDR and/or FLT3 inhibitory activities. The present compounds are particularly useful as chk1 kinase inhibitors.

In certain embodiments of the present invention, X is CR², and all other variables are as defined in Formula (**I**), (**I-a**), (**I-b**), (**I-c**), (**I-d**), (**I-e**), (**I-f**), (**I-g**), or (**I-h**). In certain embodiments of the present invention, R² is H, CF₃, C₁-C₅ alkyl, or O(C₁-C₅ alkyl), and all other variables are as defined in Formula (**I**), (**I-a**), (**I-b**), (**I-c**), (**I-d**), (**I-e**), (**I-f**), (**I-g**), or (**I-h**). In certain embodiments of the present invention, R² is H, CF₃, C₁-C₃ alkyl, or O(C₁-C₃ alkyl), and all other variables are as defined in Formula (**I**), (**I-a**), (**I-b**), (**I-c**), (**I-d**), (**I-e**), (**I-f**), (**I-g**), or (**I-h**). In certain embodiments of the present invention, R² is H, and all other variables are as defined in Formula (**I**), (**I-a**), (**I-b**), (**I-c**), (**I-d**), (**I-e**), (**I-f**), (**I-g**), or (**I-h**).

In certain embodiments of the present invention, X is N, and all other variables are as defined in Formula (**I**), (**I-a**), (**I-b**), (**I-c**), (**I-d**), (**I-e**), (**I-f**), (**I-g**), or (**I-h**).

In certain embodiments of the present invention, Y is CR⁴, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above. In certain embodiments of the present invention, R⁴ is H, and all other variables are as defined in Formula (**I**), (**I-a**), (**I-b**), (**I-c**), (**I-d**), (**I-e**), (**I-f**), (**I-g**), or (I-h), or as defined in any one of the embodiments above.

In certain embodiments of the present invention, Y is N, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, Z is CR^{7a}, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above. In certain embodiments of the present invention, Z is CR^{7a} wherein R^{7a} is H, or C₁-C₄ alkyl optionally substituted with one to three halo groups or OH; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above. In certain embodiments of the present invention, Z is CR^{7a} wherein R^{7a} is H, methyl or ethyl; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, Z is CH (i.e., Z is CR^{7a} wherein R^{7a} is H); and all other variables are as defined in Formula (**I**), (**I-a**), (**I-b**), (**I-c**), (**I-d**), (**I-e**), (**If**), (**I-g**), or (**I-h**), or as defined in any one of the embodiments above.

In certain embodiments of the present invention, Z is N, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, W is CR^{8a}, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above. In certain embodiments of the present invention, W is CR^{8a} wherein R⁸ is H, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above. In certain embodiments of the present invention, W is CR^{8a} wherein R⁸ is CH₃, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, W is N, and all other variables are as defined in Formula (**I**), or as defined in any one of the embodiments above.

In certain embodiments of the present invention, R³ is halo, -O-R⁹, N(R²²)-R⁹, - S(O)ₚ-R⁹, or R⁹; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R³ is halo, and all other variables are as defined in Formula (**I**), (**I-a**), (**I-b**), (**I-c**), (**I-d**), (**I-e**), (**I-f**), (**I-g**), or (**I-b**), or as defined in any one of the embodiments above. In certain embodiments of the present invention, R³ is Br, and all other variables are as defined in Formula (**I**), (**I-a**), (**I-b**), (**I-c**), (**I-d**), (**I-e**), (**I-f**), (**I-g**), or (I-h), or as defined in any one of the embodiments above.

In certain embodiments of the present invention, R⁹ is alkynyl, cycloalkyl, heterocyclyl, aryl, or heteroaryl and wherein each member of R⁹ is independently substituted with one to three R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁹ is C₂-C₃ alkynyl, C₆ cycloalkyl, 5-6 membered heterocyclyl having 1 to 2 nitrogen ring atoms, C₆ aryl, or 5-6 membered monocyclic or 8-10 membered bicyclic heteroaryl and wherein each member of R⁹ is independently substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁹ is C₂-C₃ alkynyl, C₆ aryl, or 5-6 membered monocyclic or 8-10 membered bicyclic heteroaryl having 1 to 2 ring atoms selected from N, O and S; and wherein each member of R⁹ is independently substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁹ is propynyl, phenyl, pyrazolyl, furanyl, thienyl, pyridyl, imidazolyl, pyrimidinyl, or benzothienyl, wherein each member of R⁹ is independently substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁹ is cyclohexyl or piperidinyl, and wherein each member of R⁹ is independently substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R³ is R⁹, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R³ is R⁹ wherein R⁹ is alkynyl, cycloalkyl, heterocyclyl, aryl, or heteroaryl and wherein each member of R⁹ is independently substituted with one to three R¹⁰ groups; and all other variables are as defined in Formula (**I**), (**I-a**), (**I-b**), (**I-c**), **(I-d**), (**I-e**), (**I-f**), (**I-g**), or (**I-h**), or as defined in any one of the embodiments above.

In certain embodiments of the present invention, R³ is R⁹ wherein R⁹ is C₂-C₃ alkynyl, C₆ cycloalkyl, 5-6 membered heterocyclyl, C₆ aryl, or 5-6 membered monocyclic or 8-10 membered bicyclic heteroaryl having 1 to 2 ring atoms selected from N, O and S; and wherein each member of R⁹ is independently substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R³ is R⁹ wherein R⁹ is C₂-C₃ alkynyl, C₆ aryl, or 5-6 membered monocyclic or 8-10 membered bicyclic heteroaryl having 1 to 2 ring atoms selected from N, O and S; and wherein each member of R⁹ is independently substituted with one to two R¹⁰ groups; and all other variables are as defined in Formula (**I**), **(I-a**), (**I-b**), (**I-c**), (**I-d**), (**I-e**), (**I-f**), (**I-g**), or (**I-h**), or as defined in any one of the embodiments above.

In certain embodiments of the present invention, R³ is R⁹ wherein R⁹ is propynyl, phenyl, pyrazolyl, furanyl, thienyl, pyridyl, imidazolyl, pyrimidinyl, or benzothienyl, wherein each member of R⁹ is independently substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R³ is R⁹ wherein R⁹ is cyclohexyl or piperidinyl, wherein each member of R⁹ is independently substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R³ is phenyl substituted with one to two R¹⁰ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R³ is phenyl substituted with one to two groups selected from halo, R¹¹, -OR¹¹, CN, -CF₃, -OCF₃, -NR¹²C(=O)R¹¹, -NR¹²S(O)_{q}R¹¹, -SR¹¹, -NR¹¹R¹², -C(O)NR¹¹R¹², -S(O)_{q}R¹¹, or -S(O)₂NR¹¹R¹², wherein R¹¹ and R¹² are optionally taken together with the attached N atom to form a 4-7 membered ring having additional 0-2 heteroatoms selected from O, S, and N, said ring being optionally substituted with one to four R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is H, halo, R¹¹, -OR¹¹, CN, -CF₃, -OCF₃, -NR¹²C(=O)R¹¹, -NR¹²S(O)_{q}R¹¹, -SR¹¹, -NR¹¹R¹², -C(=O)NR¹¹R¹², oxo, -S(O)_{q}R¹¹, or -S(O)₂NR¹¹R¹², wherein R¹¹ and R¹² are optionally taken together with the attached N atom to form a 4-7 membered ring having additional 0-2 heteroatoms selected from O, S, and N, said ring being optionally substituted with one to four R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is halo, R¹¹, -OR¹¹, CN, -CF₃, -OCF₃, -NR¹²C(=O)R¹¹, -NR¹²S(O)_{q}R¹¹, -S¹¹, -NR¹¹R¹², -C(=O)NR¹¹R¹², oxo, -S(O)_{q}R¹¹, or -S(O)₂NR¹¹R¹², wherein R¹¹ and R¹² are optionally taken together with the attached N atom to form a 4-7 membered ring having additional 0-2 heteroatoms selected from O, S, and N, said ring being optionally substituted with one to four R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is halo; CN; -CF₃; -CF₃; -NR¹²C(O)R¹¹ wherein R¹² is H and R¹¹ is C₁-C₄ alkyl; -NR¹²S(O)₂R¹¹ wherein R¹² is H and R¹¹ is C₁-C₄ alkyl; -SR¹¹ wherein R¹¹ is H or C₁-C₄ alkyl; -NR¹¹R¹² wherein R¹¹ and R¹² are independently H or C₁-C₄ alkyl and R¹¹ and R¹² are optionally taken together with the attached N atom to form a 5-6-membered ring having additional 0-2 heteroatoms selected from O, S, and N, said ring being optionally substituted with one R²² group; -C(=Y')NR¹¹R¹² wherein R¹¹ and R¹² are independently H or C₁-C₄ alkyl; oxo; -S(O)₂R¹¹ wherein R¹¹ is C₁-C₄ alkyl, C₅-C₆ cycloalkyl or a 5-6 membered heterocyclyl having 1 to 2 heteroatoms selected from N and O; or -S(O)₂NR¹¹R¹² wherein R¹¹ and R¹² are independently H or C₁-C₄ alkyl; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is F, Cl, CN, -CF₃, -OCF₃, -OH, -NHC(O)CH₃, -NHS(O)₂CH₃, -SCH₃, -NH₂, -N(Et)₂, -C(O)NH₂, -C(O)NH(p-methoxybenzyl), -C(O)N(Et)₂, oxo, -S(O)₂CH₃, -S(O)₂N(CH₃)₂, *N*-morpholinyl, *N*-piperidinyl, or N-piperazinyl, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is R¹¹, and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is R¹¹ wherein R¹¹ is alkyl or heterocyclyl, wherein said alkyl and heterocyclyl are optionally substituted with one to four R¹³ groups, wherein two geminal R¹³ groups are optionally taken together with the atom to which they are attached to form a 3-6 membered ring having additional 0-2 heteroatom selected from O, S, and N, said ring being optionally substituted with one to four R¹⁸ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is R¹¹ wherein R¹¹ is C₁-C₆ alkyl, or 5-6 membered monocyclic or 8-10 membered bicyclic heterocyclyl having 1 to 2 heteroatoms selected from N and O, wherein said alkyl and heterocyclyl are optionally substituted with one to four R¹³ groups, wherein two geminal R¹³ groups are optionally taken together with the atom to which they are attached to form a six-membered ring having 0-2 heteroatom selected from O, S, and N, said ring being optionally substituted with one to four R¹⁸ groups; and all other variables are as defined in Formula (I), (I-a), (**I-b**), (I-c), (I-d), (I-e), (I-f), (I-g), or (I-h), or as defined in any one of the embodiments above.

In certain embodiments of the present invention, R¹⁰ is R¹¹ wherein R¹¹ is C₁-C₆ alkyl, or 5-6 membered monocyclic or 8-10 membered bicyclic heterocyclyl having 1 to 2 heteroatoms selected from N and O, wherein said alkyl and heterocyclyl are optionally substituted with one to two R¹³ groups and wherein each R¹³ is independently halo, CN, CF₃, -OCF₃, oxo, -(CR¹⁴R¹⁵)ₙC(O)OR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷, or R¹⁶; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is R¹¹ wherein R¹¹ is C₁-C₆ alkyl, wherein alkyl is optionally substituted with one to two R¹³ groups and wherein each R¹³ is independently halo, CN, CF₃, -OCF₃, oxo, -(CR¹⁴R¹⁵)ₙC(O)OR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷ -(CR¹⁴R¹⁵)ₙS(O)₂NR¹⁶R¹⁷, or R¹⁶; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is R¹¹ wherein R¹¹ is methyl, ethyl, *i*-butyl, *t*-butyl, CH₂R²⁷ wherein R²⁷ is OH, CH₂OH, piperazinyl, piperidinyl, or morpholinyl wherein piperazinyl, piperidinyl is optionally substituted with methyl or ethyl; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is R¹¹ wherein R¹¹ is 5-6 membered monocyclic or 8-10 membered bicyclic heterocyclyl having 1 to 2 heteroatoms selected from N and O, wherein said alkyl and heterocyclyl are optionally substituted with one to two R¹³ groups and wherein each R¹³ is independently halo, CN, CF₃, -OCF₃, oxo, -(CR¹⁴R¹⁵)ₙC(O)OR¹⁶, -(CR¹⁴R¹⁵)ₙ(CO)NR¹⁶R¹⁷, -(CR¹⁴)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷, or R¹⁶ ; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is -OR¹¹; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is -OR¹¹ wherein R¹¹ is H, alkyl or heterocyclyl, wherein said alkyl or heterocyclyl is optionally substituted with one to four R¹³ groups and all other variables are as defined in Formula (**I**), (**I-a**), (**I-b**), (**I-c**), (**I-d**), (**I-e**), (**If**), (**I-g**), or (**I-h**), or as defined in any one of the embodiments above.

In certain embodiments of the present invention, R¹⁰ is -OR¹¹ wherein R¹¹ is H, C₁-C₄ alkyl, or 5-6 membered monocyclic or 8-10 membered bicyclic heterocyclyl having 1 to 2 nitrogen atoms, wherein said alkyl or heterocyclyl is optionally substituted with one to four R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R¹⁰ is -OR¹¹ wherein R¹¹ is H, C₁-C₄ alkyl, or 5-6 membered monocyclic or 8-10 membered bicyclic heterocyclyl having 1 to 2 nitrogen atoms, wherein said alkyl or heterocyclyl is optionally substituted with one to two R¹³ groups, wherein each R¹³ is independently halo, CN, CF₃, -OCF₃, oxo, -(CR¹⁴R¹⁵)ₙC(O)OR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)NR¹⁶C(O)R¹⁷, -(CR¹⁴R¹⁵)ₙS(O)₂NR¹⁶R¹⁷, or R¹⁶ ; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁵ is H, -(CR¹⁴R¹⁵)ₙC(O)NR¹¹R¹², -(CR¹⁴R¹⁵)ₙN¹²C(O)R¹¹, -(CR¹⁴R¹⁵)ₙNR¹¹R¹², -(CR¹⁴R¹⁵)ₙOR¹¹, alkyl, or heterocyclyl, wherein the said alkyl or heterocyclyl is optionally substituted with one to four R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁵ is H; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁵ is -(CR¹⁴R¹⁵)ₙC(O)NR¹¹R¹², -(CR¹⁴R¹⁵)ₙNR¹²C(O)R¹¹, -(CR¹⁴R¹⁵)ₙR¹¹R¹², -(CR¹⁴R¹⁵)ₙOR¹¹, C₁-C₆ alkyl, or 5-6 membered monocyclic heterocyclyl having 1 to 2 nitrogen atoms, wherein said alkyl or heterocyclyl is optionally substituted with one to two R¹³ groups; wherein R¹⁴ and R¹⁵ are H; n is 0-2; each R¹¹ is independently H, C₁-C₄ alkyl or 5-6 membered monocyclic heterocyclyl having 1 to 2 nitrogen atoms, wherein said alkyl or heterocyclyl is optionally substituted with one to two R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁵ is -(CR¹⁴R¹⁵)ₙC(O)NR¹¹R¹², -(CR¹⁴R¹⁵)ₙNR¹²C(O)R¹¹, -(CR¹⁴R¹⁵)ₙNR¹¹R¹², -(CR¹⁴R¹⁵)ₙOR¹¹, C₁-C₆alkyl, or 5-6 membered monocyclic heterocyclyl having 1 to 2 nitrogen atoms, wherein said alkyl or heterocyclyl is optionally substituted with one to two R¹³ groups; wherein R¹⁴ and R¹⁵ are H; n is 0-2; each R¹¹ is independently H, C₁-C₄ alkyl, or 5-6 membered monocyclic heterocyclyl having 1 to 2 nitrogen atoms, wherein said alkyl or heterocyclyl is optionally substituted with one to two R¹³ groups; R¹³ is OH, O(C₁-C₃ alkyl), or C₁-C₃ alkyl; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁵ is 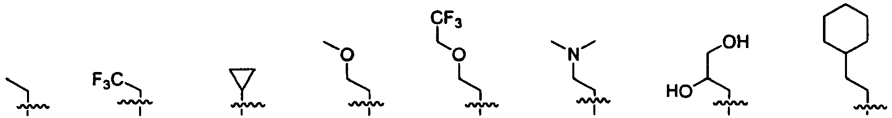 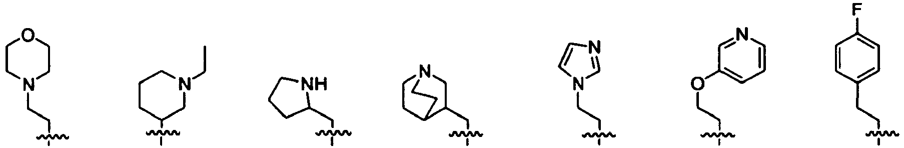 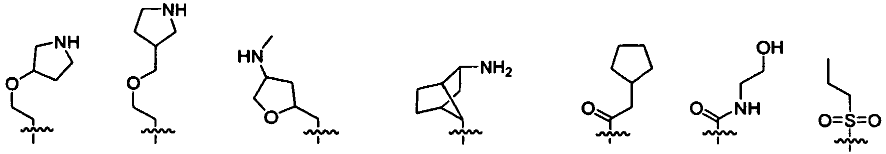 and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN, -CF₃, -OCF₃, halo, -C(=Y')OR", -C(=Y')NR¹¹R¹², -OR¹¹, -OC(-Y')R¹¹, -NR¹¹R¹², -NR¹²C(=Y')R¹¹, -NR¹²C(=Y')NR¹¹R¹², -NR¹²S(O)_{q}R¹¹, -S¹¹, -S(O)R¹¹, -S(O)₂R¹¹, -OC(=Y')NR¹¹R¹², -S(O)₂NR¹¹R¹², -S(O)₂(OR¹¹), -SC(=Y')R¹¹, -SC(=Y')OR", -SC(=Y')NR¹¹R¹², alkyl, alkenyl, alkynyl, cycloalkyl, heterocyclyl, aryl, or heteroaryl wherein said alkyl, alkenyl, alkynyl, cycloalkyl, heterocyclyl, aryl and heteroaryl are optionally substituted by one to four R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN, CF₃, -OCF₃, halo, -C(O)OR¹¹, -C(O)NR¹¹R¹², -OR¹¹, -NR¹¹R¹², -NR¹²C(O)R¹¹, -NR¹²C(=NR¹²)R¹¹, -NR¹²S(O)₂R¹¹, -SR¹¹, -S(O)₂R¹¹, alkyl, cycloalkyl, heterocyclyl, aryl, or heteroaryl wherein said alkyl is substituted with one to four R¹³ groups except H and said heterocyclyl or heteroaryl is optionally substituted by one to four R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN, halo, -C(O)NR¹¹R¹² -OR¹¹, -NR¹¹R¹², -NR¹²C(O)R¹¹, alkyl, cycloalkyl, heterocyclyl, aryl, or heteroaryl, wherein said alkyl is substituted with one to two R¹³ groups except H, and said heteroaryl is optionally substituted by one to two R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN, halo, -C(O)NR¹¹R¹², -OR¹¹, -NR¹¹R¹², -NR¹²C(O)R¹¹, C₁-C₃ alkyl, C₃-C₆ cycloalkyl, 5-6 membered heterocyclyl having 1 to 2 heteroatoms, C₆ aryl, or 5-6 membered heteroaryl having I to 2 heteroatoms; wherein said alkyl is substituted with one to two R¹³ groups except H; and said cycloalkyl, aryl, heterocyclyl or heteroaryl is optionally substituted by one to two R¹³ groups; wherein heteroatoms are selected from N, O and S; wherein each R¹² is H or C₁-C₃ alkyl and each R¹¹ is independently H or C₁-C₃ alkyl optionally substituted by one to two R¹³ groups; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN, halo, -C(O)NR¹¹R¹², -OR¹¹, -NR¹¹R¹², -NR¹²C(O)R¹¹, C₁-C₃ alkyl, or 5-6 membered heteroaryl having 1 to 2 nitrogens, wherein said alkyl is substituted with one to two R¹³ groups (wherein R¹³ is OR¹⁶ where R¹⁶ is H or alkyl), and said heteroaryl is optionally substituted by one to two R¹³ groups (wherein R¹³ is R¹³ is OR¹⁶, NR¹⁶R¹⁷, or C₁-C₂ alkyl optionally substituted with R¹⁸ where each of R¹⁶ and R¹⁷ is independently H or alkyl); wherein each R¹² is H or C₁-C₃ alkyl and each R¹¹ is independently H or C₁-C₃ alkyl optionally substituted by one to two R¹³ groups (wherein R¹³ is OR¹⁶ where R¹⁶ is H or alkyl); and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN, or 5-6 membered heteroaryl having 1 to 2 nitrogens, wherein said alkyl substituted with one to two R¹³ groups, and said heteroaryl is optionally substituted by one to two R¹³ groups wherein R¹³ is C₁-C₂ alkyl; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN, F, Cl, -C(O)OH, -C(O)NH₂, -C(O)NHCH₂CH₂OH, -C(O)N(CH₃)₂, -OCH₃, -CH₂OH, -C(CH₃)₂OH, pyridyl, or pyrazolyl optionally substituted with methyl; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is CN; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments of the present invention, R⁶ is pyridyl, or pyrazolyl optionally substituted with methyl; and all other variables are as defined in Formula (**I**), (**I-a**), (**I-b**), (**I-c**), (**I-d**), (**I-e**), (**I-f**), (**I-g**), or (**I-h**), or as defined in any one of the embodiments above.

In certain embodiments, compounds are of Formula (**II**) or (**III**): 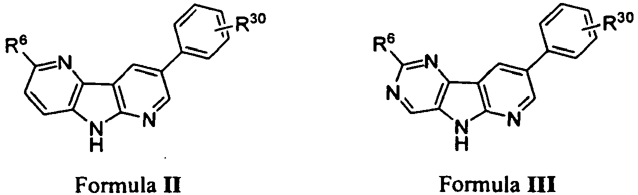 wherein R⁶ is CN, pyridyl, or pyrazolyl optionally substituted by methyl; and R³⁰ is

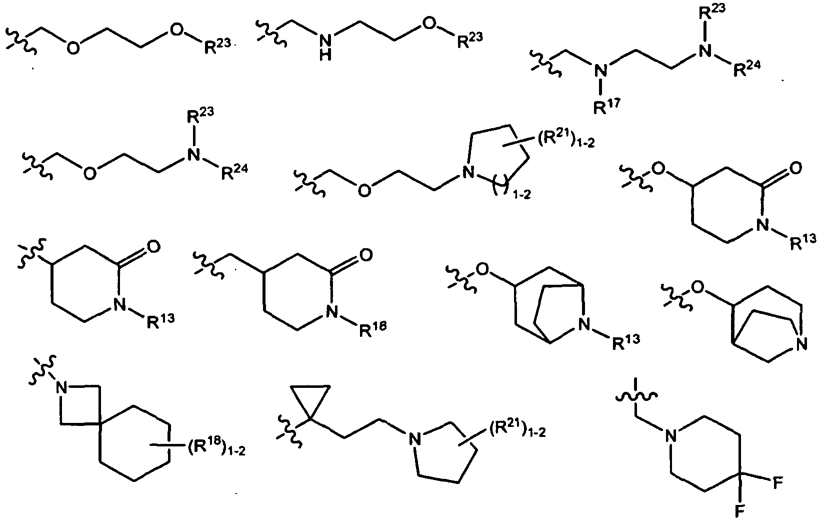

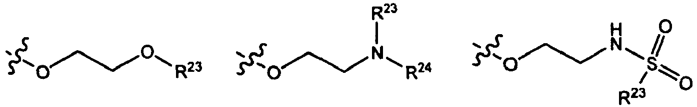

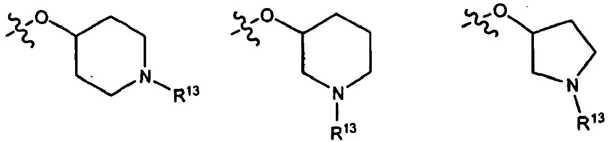

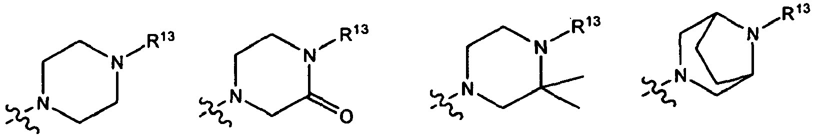

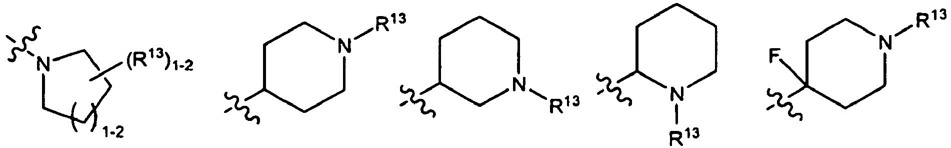

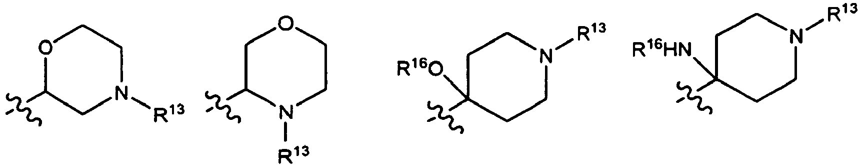

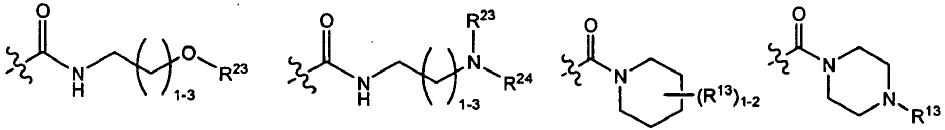

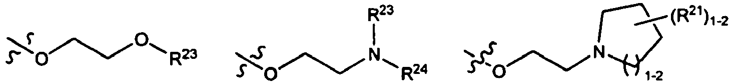

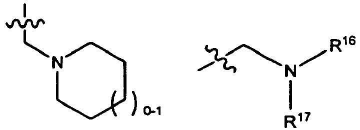

In certain embodiments, compounds are of Formula (**IV**) or (**V**): 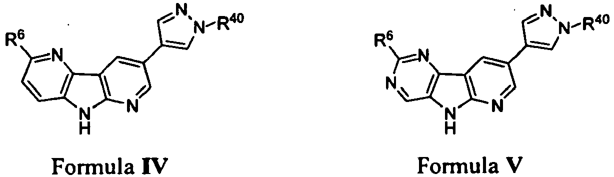 wherein R⁶ is CN, pyridyl, or pyrazolyl optionally substituted by methyl; and R⁴⁰ is H, CH₃

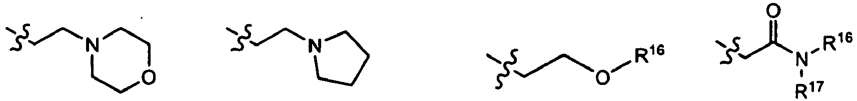

In certain embodiments of the present invention, compounds are of Formula (**VI**): 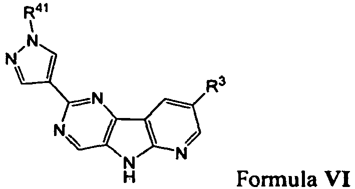 wherein R⁴¹ is H or methyl.

Another embodiment of the present invention includes title compounds described in EXAMPLES 1-17 below.

The present compounds (such as 5H-dipyrido[2,3-b;2',3'-d]pyrroles of formula (**6-5**) and 9H-1,5,7,9-tetraaza-fluorenes of formula (**1-10**) are prepared according to the procedures described below in the schemes and examples, or by methods known in the art. The starting materials and various intermediates may be obtained from commercial sources, prepared from commercially available compounds, or prepared using well known synthetic methods. Accordingly, methods for making the present compounds of Formula (**I**), (**I-a**), (**I-b**), (**I-c**), (**I-d**), (**I-e**), (**I-f**), (**I-g**), or (**I-h**) according to Scheme 1, 2, 3, 4, 5, 6, 7, 8, 9, 10, 11, and/or 12, are within the scope of the present invention.

For example, 9H-1,5,7,9-tetraaza-fluorenes of formula (**1-10**) may be prepared using the synthetic route outlined in Scheme 1.

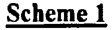

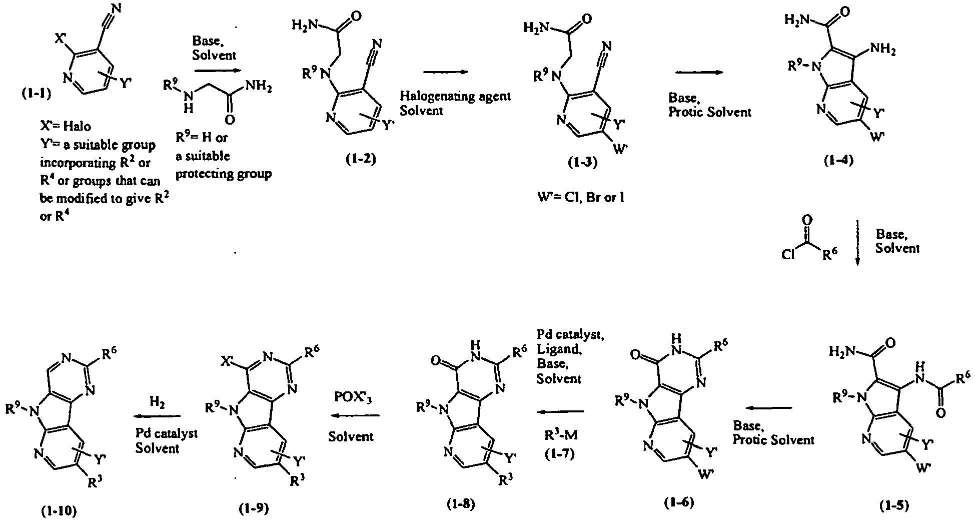

Compounds of formula (**1-1**) may be prepared using published methods described in the literature. They may be reacted with glycinamide or a glycinamide derivative such as *N-*benzyl glycinamide in the presence of an additive, such as potassium fluoride, in a suitable solvent, such as DMSO, at a temperature from ambient temperature to 120 °C, to obtain compounds of formula (**1-2**). Intermediates of formula (**1-2**) may then be halogenated in the presence of a suitable halogenating agent such as *N*-bromosuccinimide, in a suitable solvent such as DMF, at a temperature from 20 °C to 50 °C, to obtain compounds (**1-3**). Intermediates of formula (**1-3**) may then be cyclised in the presence of a base such as sodium hydrogen carbonate in a solvent such as ethanol at a temperature between 20 °C and 120°C to provide compounds of formula (**1-4**).

Compounds of formula (**1-5**) can be obtained by reaction of intermediate (**1-4**) with the appropriate acid chloride in the presence of a base such as pyridine at a temperature between 20 °C and 100 °C. Compounds of formula (**1-6**) may be formed by cyclisation of compound (**1-5**) with a suitable base such as aqueous sodium hydroxide in a co-solvent such as ethanol at temperatures between 50 °C and 170 °C. Compounds of formula (**1-8**) may be obtained from compounds of formula (**1-6**) by reaction with a boronic acid or boronate ester of formula (**1-7**) (incorporating appropriate substituents R³), in the presence of a catalyst such as bis(triphenylphosphine) palladium(II) dichloride, a base such as aqueous sodium carbonate in a suitable solvent such as acetonitrile at a temperature of from room temperature to the reflux temperature of the solvent, or under microwave irradiation at a temperature between 70 °C and 150 °C.

Compounds of formula (**1-8**) may be converted to compounds of formula (**1-9**) by reaction with a halogenating agent such as phosphorus oxychloride, neat or in a suitable solvent such as toluene, at a temperature from 20 °C to 140°C. Compounds of formula (**1-10**) can be formed by catalytic reduction of compounds of formula (**1-9**) using a catalyst such as palladium in a suitable polar solvent such as DMF/EtOH under an atmosphere of hydrogen.

Compounds of general formula (**2-4**) may also be prepared according to the procedure shown in Scheme 2.

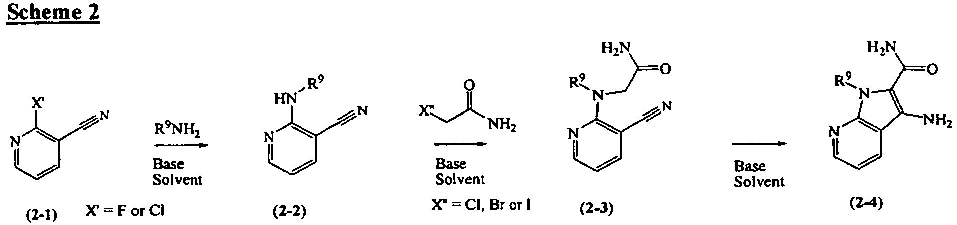

Compounds of general formula (**2-1**) may be prepared using published methods described in the literature. Compounds of formula (**2-1**) may be reacted with amines such as benzylamine, in a solvent such as ethanol, at a temperature from 0 °C to reflux to give intermediates of general formula (**2-2**). Compounds of formula (**2-2**) may be alkylated with alkyl acetates such as bromo *tert*-butyl acetate, in the presence of a base such as sodium hydrogen carbonate, in a solvent such as DMF, to give compounds of general formula (**2-3**). Cyclisation of compounds of general formula (**2-3**) to give compounds of general formula (**2-4**) may be achieved using a base such as sodium tert- butoxide, in a solvent such as THF, at a temperature between -40 °C and the boiling point of the solvent. Alternatively compounds of general formula (**2-4**) may be prepared directly from compounds of general formula (**2-2**) using more than one equivalent of base and prolonged reaction times or higher temperatures.

Alternatively, 9H-1,5,7,9-tetraaza-fluorene compounds of formula (**3-9**) may be prepared using the synthetic routes outlined in Scheme 3.

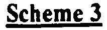

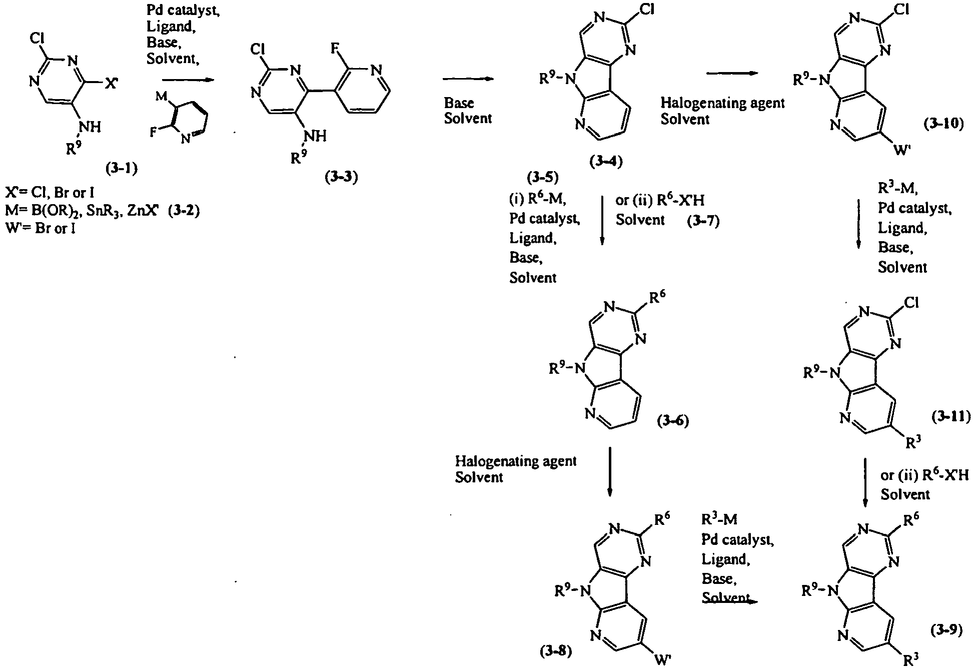

Compounds of general formula (**3-3**) may be obtained from compounds of formula (**3-1**) by reaction with a boronic acid or boronate ester of formula (**3-2**), in the presence of a catalyst such as *bis*(triphenylphosphine)palladium(II)dichloride, a base such as aqueous sodium carbonate in a suitable solvent such as acetonitrile at a temperature between room temperature and the reflux temperature of the solvent, or under microwave irradiation at a temperature between 70 °C and 150 °C. Compounds of general formula (**3-3**) may be cyclised to obtain compounds of formula (**3-4**) with a suitable base such as sodium hexamethyldisilazane in a suitable solvent such as THF at a temperature between 0 °C and 50 °C.

Compounds of general formula (**3-4**) may then be converted to compounds of general formula (**3-6**) by reaction with a boronic acid or boronate ester of formula (**3-5**) (incorporating appropriate substituents R⁶), in the presence of a catalyst such as *bis*(triphenylphosphine)palladium(II)dichloride, a base such as aqueous sodium carbonate in a suitable solvent such as acetonitrile at a temperature of from room temperature to the reflux temperature of the solvent, or under microwave irradiation at a temperature between 70 °C and 150 °C. Alternatively, compounds of formula (**3-4**) may be coupled with an aryl or alkyl tin compound of formula (**3-5**) (incorporating appropriate substituents R⁶), in the presence of a catalyst such as *bis*(triphenylphosphine)palladium(II) dichloride or [1,1'-*bis*(diphenylphosphino) ferrocene] dichloropalladium (II), with or without an aqueous base such as sodium carbonate, in a suitable solvent such as acetonitrile at a temperature from room temperature to the reflux temperature of the solvent, or under microwave irradiation at a temperature from 70 °C to 150 °C.

Compounds of general formula (3-6) may be obtained from compounds of formula (**3-4**) by reaction with compounds of general formula (**3-7**) in the presence of a catalyst such as [1,1'-*bis*(diphenylphosphino)ferrocene] dichloropalladium(II), in the presence of a base such as potassium *tert*-butoxide in a suitable solvent such as DME, or a mixture of two or more appropriate solvents, at a temperature from room temperature to the reflux temperature of the solvent or solvents, or under microwave irradiation at a temperature from 70 °C to 160 °C, which may be similar to conditions described in the literature by Buchwald and Hartwig.

Intermediates of formula (**3-6**) may then be halogenated in the presence of a suitable halogenating agent, such as bromine, in a solvent such as acetic acid, at a temperature from 20 °C to 120 °C, to obtain compounds of formula (**3-8**). Compounds of formula (**3-8**) may then be converted to compounds of formula (**3-9**) using methods described in Scheme 1. Alternatively, compounds of formula (**3-4**) may be halogenated to give compounds of formula (**3-10**), then converted to compounds of formula (**3-11**) by reaction with a boronic acid, boronate ester or stannane then converted to compounds of formula (**3-9**) using similar conditions to those described for the introduction of R³.

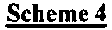

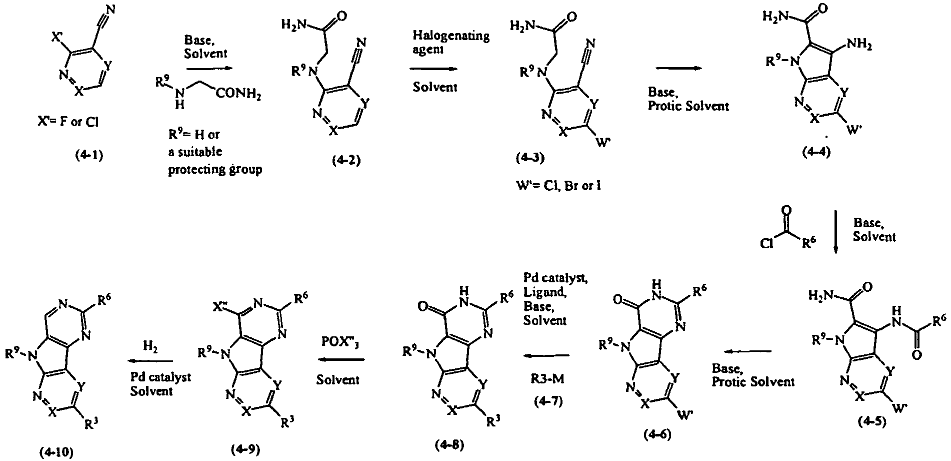

Compounds of formula (**4-1**) may be prepared according to methods described in the literature. Compounds of formula (**4-10**) may be obtained from compounds of formula (**4-1**) using similar methods to the ones described for the preparation of compounds of formula (**1-10**) from compounds of formula (**1-1**), as shown in Scheme 1.

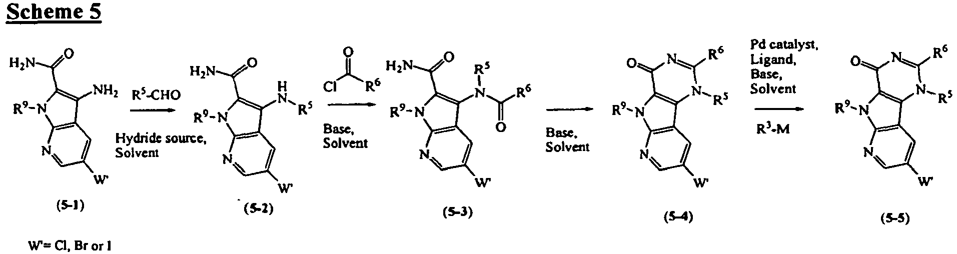

Compounds of general formula (**5-2**) can be prepared by alkylation of compounds of formula (**5-1**) by reductive alkylation employing an aldehyde in the presence of a borohydride, such as sodium cyanoborohydride in a polar solvent such as EtOH at a temperature between 0°C and 50 °C. Compounds of formula (**5-5**) may be synthesised using methods described above for the preparation of compounds of formula (**1-6**) from compounds of formula (**1-4**) in Scheme 1.

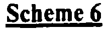

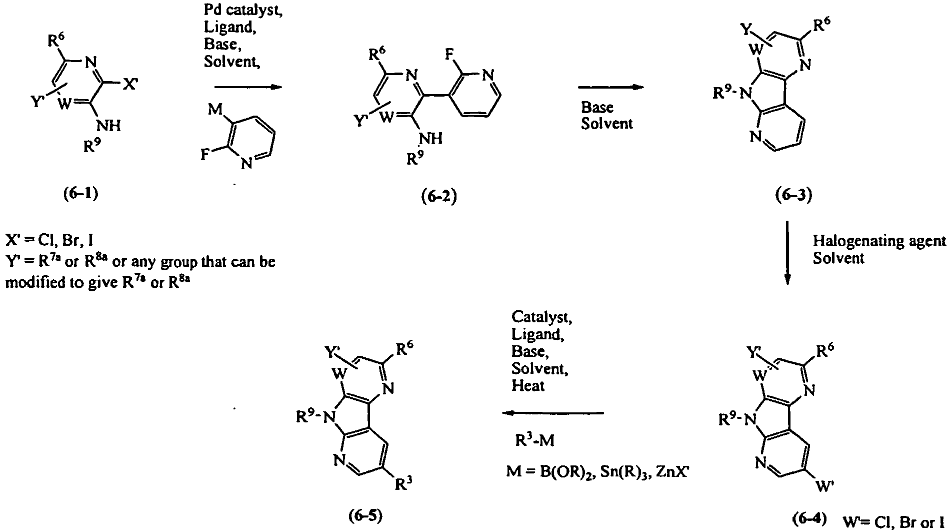

Compounds of general formula (**6-1**) may be obtained from commercial sources or prepared using published methods described in the literature. Compounds of general formula (**6-2**) may be obtained from a compound of formula (**6-1**), a boronic acid or boronate ester in the presence of a transition metal catalyst such as *bis*(triphenylphosphine)palladium(II) dichloride, a base such as aqueous sodium carbonate in a suitable solvent such as acetonitrile at a temperature from room temperature to the reflux temperature of the solvent, or under microwave irradiation at a temperature between 70 °C and 150°C. Cyclisation of compounds with general formula (**6-2**) may be accomplished with a suitable base such as sodium hexamethyldisilazide in a suitable solvent such as THF at a temperature between 0 °C and 50 °C. Compounds of general formula (**6-3**) may then be halogenated with a halogenating agent such as bromine in a solvent such as acetic acid at a temperature between room temperature and the reflux temperature of the solvent to give intermediates of formula (**6-4**).

Compounds of formula (**6-5**) may be obtained from compounds of formula (**6-4**) using similar methods to the ones described for the preparation of compounds of formula (**1-8**) from compounds of formula (**1-6**), as shown in Scheme 1.

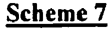

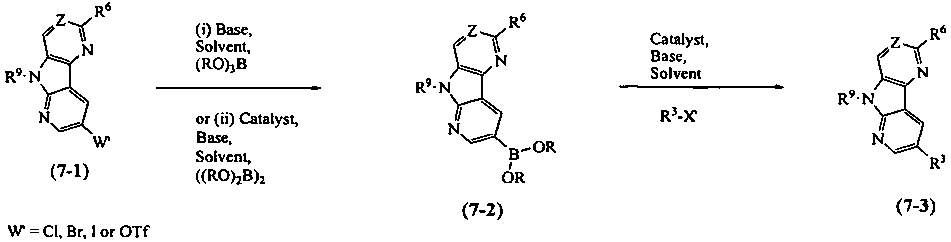

Compounds of formula (**7-3**) may also be prepared according to the procedure shown in Scheme 7. The boronic acid of formula (**7-2**) may be prepared from compounds of formula (**7-1**) by treatment with a base such as butyl lithium in the presence of an alkyl borate such as trimethyl borate.in a suitable solvent such as THF at a temperature between -78 °C and ambient temperature.

Alternatively, the boronate ester of formula (**7-2**) may be prepared from compounds of formula (**7-1**) with the appropriate alkylatodiboron in the presence of a catalyst such as *bis*(diphenylphosphino)ferrocenepalladium(II) dichloride, using a suitable base such as potassium acetate in a solvent such as dioxane at a temperature from room temperature to the reflux temperature of the solvent, or under microwave irradiation at a temperature from 70 °C to 150°C.

Compounds of formula (**7-3**) may be prepared by reaction of compounds of formula (**7-2**) with an appropriate halide (incorporating appropriate substituents R³), in the presence of a catalyst such as bis(triphenylphosphine)palladium(II) dichloride, with a base such as aqueous sodium carbonate in a suitable co-solvent such as acetonitrile at a temperature between room temperature and the reflux temperature of the solvent, or under microwave irradiation at a temperature between 70 °C and 150 °C.

The protecting group (R⁹) of compounds of formula (**7-1**), (**7-2**) and (**7-3**) may be manipulated at any stage of the synthesis. A protecting group such as SEM (trimethylsilyl ethoxymethyl), can be installed using an alkylating agent such as SEM-chloride, in a solvent such as DMF in the presence of a suitable base such as sodium hydride. Compounds of general formula (**7-3**) where R⁹ is a protecting group such as SEM may be de-protected using a reagent such as tetrabutylammonium fluoride in a solvent such as THF at a temperature from -20 °C to 50 °C to provide compounds where R⁹ is H.

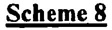

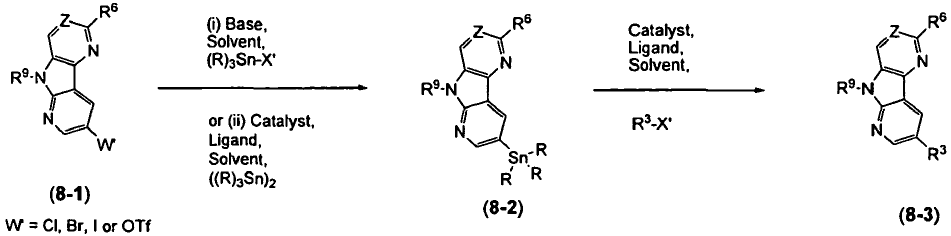

Compounds of general formula (**8-3**) may also be prepared according to the procedure shown in Scheme 8. Stannanes of general formula (**8-2**) may be prepared from compounds of general formula (**8-1**) with a base and the appropriate tin halide in a suitable solvent such as THF.

Alternatively, stannanes of general formula (**8-2**) may be prepared from compounds of general formula (**8-1**) with the appropriate alkylditin in the presence of a catalyst such as *tetrakis*(triphenylphosphine) palladium(0) in a suitable solvent such as toluene at a temperature between room temperature and the reflux temperature of the solvent, or under microwave irradiation at a temperature between 70 °C and 150 °C.

Compounds of general formula (**8-3**) may be prepared from compounds of general formula (**8-2**) with the appropriate halide, in the presence of a catalyst such as *tetrakis*(triphenylphosphine) palladium(0) in a suitable solvent such as dioxane at a temperature from room temperature to the reflux temperature of the solvent, or under microwave irradiation at a temperature between 70 °C and 150°C.

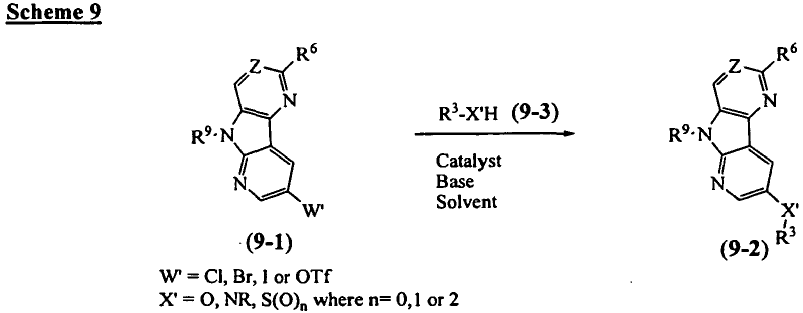

Compounds of general formula (**9-2**) may be obtained from compounds of formula (**9-1**) by reaction with compounds of general formula (HX'-R³) in the presence of reagents such as copper(II)iodide or copper powder in the presence of a base such as cesium carbonate in a suitable solvent such as DMF at a temperature between room temperature and the reflux temperature of the solvent, or under microwave irradiation at a temperature between 70 °C and 240 °C, which may be similar to conditions described in the literature by Ullmann.

Compounds of general formula (**9-2**) may be obtained from compounds of formula (**9-1**) by reaction with compounds of general formula (HX'-R³) in the presence of a catalyst such as [1,1'-*bis*(diphenylphosphino)ferrocene] dichloropalladium(II), in the presence of a base such as potassium *tert*-butoxide in a suitable solvent such as DME, or a mixture of two or more appropriate solvents, at a temperature between room temperature and the reflux temperature of the solvent or solvents, or under microwave irradiation at a temperature between 70°C and 160 °C, which may be similar to conditions described in the literature by Buchwald and Hartwig.

Appropriate boronic acids, boronate esters and stannanes may be prepared using methods described in the literature or the synthetic route outlined in Scheme 10.

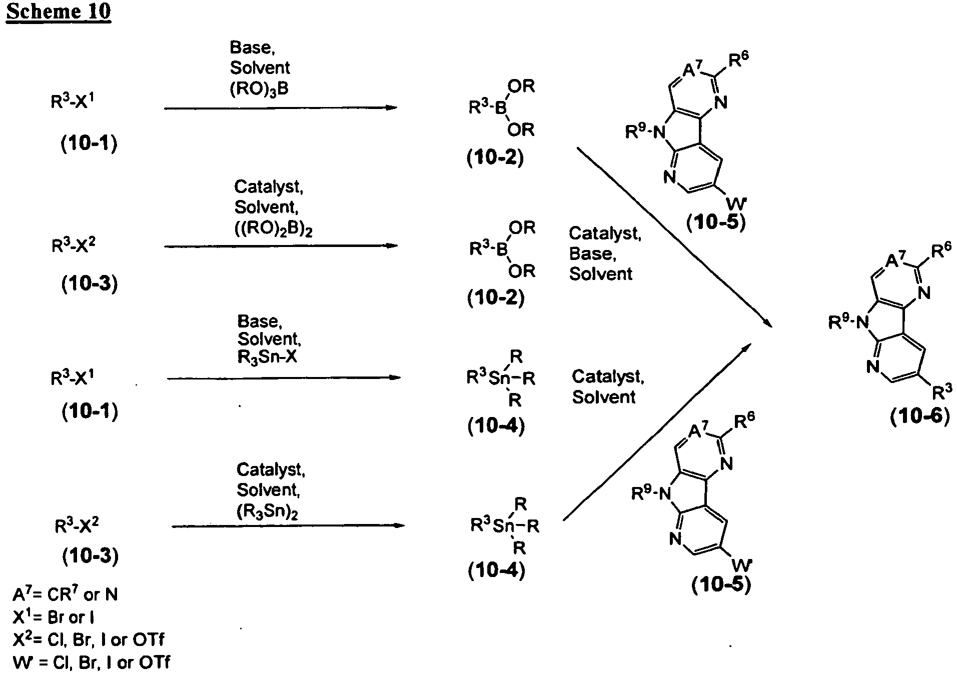

Compounds of general formula (**10-2**) may be obtained from compounds of formula (**10-1**) by reaction with a reagent such as *n*-butyllithium in a polar aprotic solvent such as THF or diethylether at temperatures between -100 °C and 0 °C and quenched with a boronic ester such as trimethyl borate or triisopropyl borate.

Compounds of general formula (**10-2**) may be obtained from compounds of formula (**10-3**) by reaction with a reagent such as *bis*(pinacolato)diborane in the presence of a catalyst such as [1,1'-*bis*(diphenylphosphino)ferrocene] dichloropalladium(II), in the presence of a base such as potassium acetate in a suitable solvent such as dioxane, a mixture of two or more appropriate solvents, at a temperature between room temperature to the reflux temperature of the solvent or solvents, or under microwave irradiation at a temperature between 70 °C and 160 °C.

Compounds of general formula (**10-4**) may be obtained from compounds of formula (**10-1**) by reaction with a reagent such as hexamethyl ditin or triethyl tin chloride in the presence of a catalyst such as *tetrakis*(triphenylphosphine) palladium (0), in the presence of a base such as potassium carbonate in a suitable solvent such as DMF, or a mixture of two or more appropriate solvents, at a temperature of from room temperature to the reflux temperature of the solvent or solvents, or under microwave irradiation at a temperature between 70 °C and 160 °C. Alternatively, these compounds of general formula (**10-4**) may be obtained from compounds of formula (**10-3**) by reaction with a reagent such as *n*-butyllithium in a suitable aprotic solvent such as THF at temperatures between -100 °C and 25 °C and then reacted with a reagent such as hexamethyl ditin or triethyl tin chloride in a suitable aprotic solvent such as THF at temperatures between -100 °e and 50 °C.

Compounds of general formula (**10-6**) may be obtained from compounds of formula (**10-5**) by reaction with a boronic acid or boronate ester of formula (**10-2**) (incorporating appropriate substituents R³), in the presence of a catalyst such as *bis*(triphenylphosphine) palladium(II)dichloride or [1,1'-*bis*(diphenylphosphino)ferrocene] dichloropalladium (II), an aqueous base such as sodium carbonate, in a suitable solvent such as acetonitrile at a temperature from room temperature to the reflux temperature of the solvent, or under microwave irradiation at a temperature between 70 °C and 150 °C. Alternatively, compounds of formula (**10-6**) may be obtained from compounds of formula (**10-5**) by reaction with an aryl or alkyl tin compound of formula (**10-4**) (incorporating appropriate substituents R³), in the presence of a catalyst such as bis(triphenylphosphine) palladium(II)dichloride or [1,1'-*bis*(diphenylphosphine)ferrocene] dichloropalladium (II), with or without an aqueous base such as sodium carbonate, in a suitable solvent such as acetonitrile at a temperature between room temperature and the reflux temperature of the solvent, or under microwave irradiation at a temperature between 70 °C and 150°C.

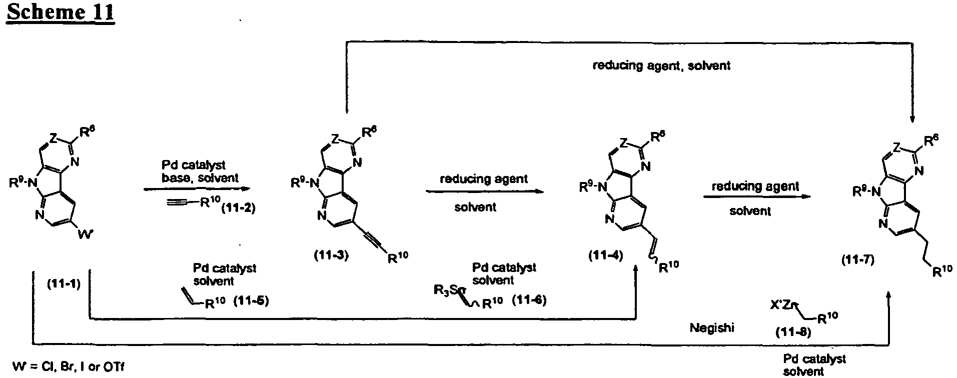

Compounds of general formula (**11-7**) may be prepared using published methods described in the literature. Compounds of formula (**11-7**) may also be prepared using the synthetic routes outlined in Scheme 11. Compounds of general formula (**11-3**) may be obtained from compounds of general formula (**11-1**) and a suitable alkyne (**11-2**) (incorporating a group R¹⁰ that could be either maintained without modification after coupling, or that could later be modified to give other groups R¹⁰) by reaction in the presence of a catalyst system such as tetrakis(triphenylphosphine)palladium(0) and copper (I) iodide in the presence of a base such as triethylamine and a suitable solvent such as DMF at a temperature between room temperature and the boiling point of the solvent. Such a coupling reaction could also be carried out in the presence of palladium on carbon, triphenylphosphine, copper (I) iodide and triethylamine in the presence of a suitable solvent such as acetonitrile at a temperature from room temperature to the reflux temperature of the solvent or solvents, or under microwave irradiation at a temperature between 70 °C and 160°C.

Compounds of general formula (**11-4**) may be obtained from compounds of general formula (**11-3**) and hydrogen in the presence of a suitable catalyst such as Lindlar catalyst or palladium on barium sulfate in the presence of quinoline and a suitable solvent such as methanol or ethanol. Compounds of general formula (**11-4**) may also be obtained by reaction of a compound of general formula (**11-1**) with a suitable alkene (**11-5**) (incorporating a group R¹⁰ that could be either maintained without modification after coupling or that could later be modified to give other groups R¹⁰) in the presence of a base such as triethylamine or potassium carbonate, a phosphine such as triphenyl phosphine, a metal species such as palladium acetate and a solvent such as acetonitrile at a temperature between room temperature and the boiling point of the solvent. Compounds of general formula (**11-4**) may also be obtained by the reaction of a compound of general formula (**11-1**) by reaction with a vinyl stannane (**11-6**) (incorporating a group R¹⁰ that could be either maintained without modification after coupling or that could later be modified to give other groups R¹⁰) in the presence of a metal species such as tetrakis(triphenylphosphine)palladium (0) in a suitable solvent such as toluene.

Compounds of general formula (**11-7**) may be obtained from compounds of general formula (**11-4**) or (**11-3**) by reaction with hydrogen in the presence of a catalyst such as palladium on carbon or platinum (IV) oxide monohydrate in a suitable solvent such as methanol or ethanol.

They may also be obtained by reaction of compounds of general formula (**11-1**) by reaction with a suitable alkyl zinc reagent (**11-8**) in the presence of a catalyst such as allyl palladium (II) chloride dimer or *bis*(tri-*tert*-butylphosphine)palladium (0) and a suitable solvent such as 1,4-dioxane at a temperature between room temperature and the boiling point of the solvent.

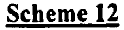

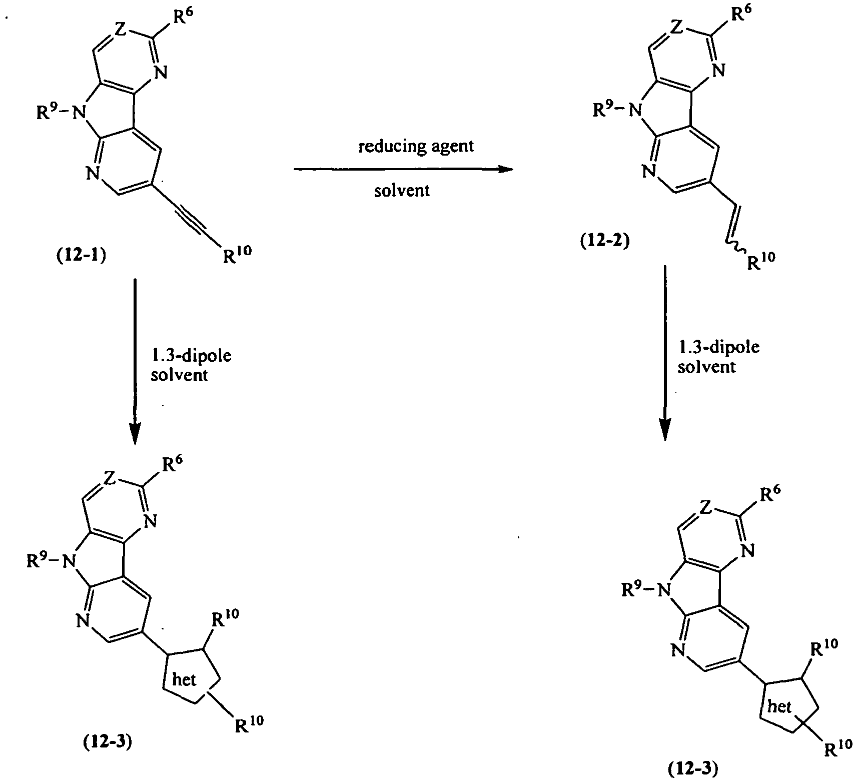

Compounds of general formula (**12-3**) may be prepared from compounds of general formula (**12-1**) by reaction with a suitable 1,3-dipole (incorporating a group R¹⁰ that could be either maintained without modification after coupling or that could later be modified to give other groups R¹⁰), such as trimethylsilylazide in a suitable solvent such as toluene at a temperature between room temperature and the boiling point of the solvent.

Compounds of general formula (**12-2**) may be obtained by reduction of compound of formula (**12-1**) using a suitable reducing agent such as hydrogen in the presence of a suitable catalyst such as Lindlar catalyst or palladium on barium sulfate in the presence of quinoline and a suitable solvent such as methanol or ethanol.

Compounds of general formula (**12-3**) may be obtained by reaction of compounds of general formula (**12-2**) with a suitable 1,3-dipole (or its precursors, incorporating a group R¹⁰ that could be either maintained without modification after coupling or that could later be modified to give other groups R¹⁰) such as *N*-methoxymethyl-*N*-(trimethylsilylmethyl) benzylamine and lithium fluoride in a solvent such as acetonitrile with or without ultrasonic treatment, or nitroethane and phenyl isocyanate in a suitable solvent such as toluene in the presence of a base such as triethylamine at a temperature between 0 °C and the boiling point of the solvent.

It will be appreciated by those skilled in the art that where suitable halogenated intermediates are prepared, compounds of various formulae may participate in coupling reactions as shown in Schemes 7, 8, 9, 10, 11 and 12 to give compounds where the R⁸ substituent is a functional group other than hydrogen which may then be further modified chemically.

The substituent R⁹ may be manipulated at any stage of the synthesis. For example, compounds where R⁹ is H may be appended with protecting groups such as SEM (trimethylsilyl ethoxy), using an alkylating agent such as SEM-chloride, in a solvent such as DMF in the presence of a base such as sodium hydride. Additionally compounds of various formulae where R⁹ is a protecting group such as SEM may be de-protected using a reagent such as tetrabutylammonium fluoride in a solvent such as THF at a temperature of from -20 °C to 50 °C to provide compounds where R⁹ is H.

It will be appreciated that where appropriate functional groups exist, compounds of various formulae or any intermediates used in their preparation may be further derivatised by one or more standard synthetic methods employing substitution, oxidation, reduction, or cleavage reactions. Particular substitution approaches include conventional alkylation, arylation, heteroarylation, acylation, sulfonylation, halogenation, nitration, formylation and coupling procedures.

In a further example, primary amine or secondary amine groups may be converted into amide groups (-NHCOR' or -NRCOR') by acylation. Acylation may be achieved by reaction with an appropriate acid chloride in the presence of a base, such as triethylamine, in a suitable solvent, such as dichloromethane, or by reaction with an appropriate carboxylic acid in the presence of a suitable coupling agent such HATU (O-(7-azabenzotriazol-1-yl)-N,N,N',N'-tetramethyluronium hexafluorophosphate) in a suitable solvent such as dichloromethane. Similarly, amine groups may be converted into sulphonamide groups (-NHSO₂R' or - NR"SO₂R') groups by reaction with an appropriate sulphonyl chloride in the presence of a suitable base, such as triethylamine, in a suitable solvent such as dichloromethane. Primary or secondary amine groups can be converted into urea groups (-NHCONR'R" or -NRCONR'R") by reaction with an appropriate isocyanate in the presence of a suitable base such as triethylamine, in a suitable solvent, such as dichloromethane.

An amine (-NH₂) may be obtained by reduction of a nitro (-NO₂) group, for example by catalytic hydrogenation, using for example hydrogen in the presence of a metal catalyst, for example palladium on a support such as carbon in a solvent such as ethyl acetate or an alcohol e.g. methanol. Alternatively, the transformation may be carried out by chemical reduction using for example a metal, e.g. tin or iron, in the presence of an acid such as hydrochloric acid.

In a further example, amine (-CH₂NH₂) groups may be obtained by reduction of nitriles (-CN), for example by catalytic hydrogenation using for example hydrogen in the presence of a metal catalyst, for example palladium on a support such as carbon, or Raney nickel, in a solvent such as an ether e.g. a cyclic ether such as tetrahydrofuran, at a temperature from -78 °C to the reflux temperature of the solvent.

In a further example, amine (-NH₂) groups may be obtained from carboxylic acid groups (-CO₂H) by conversion to the corresponding acyl azide (-CON₃), Curtius rearrangement and hydrolysis of the resultant isocyanate (-N=C=O).

Aldehyde groups (-CHO) may be converted to amine groups (-CH₂NR'R")) by reductive amination employing an amine and a borohydride, for example sodium triacetoxyborohydride or sodium cyanoborohydride, in a solvent such as a halogenated hydrocarbon, for example dichloromethane, or an alcohol such as ethanol, where necessary in the presence of an acid such as acetic acid at around ambient temperature.

In a further example, aldehyde groups may be converted into alkenyl groups (-CH=CHR') by the use of a Wittig or Wadsworth-Emmons reaction using an appropriate phosphorane or phosphonate under standard conditions known to those skilled in the art.

Aldehyde groups may be obtained by reduction of ester groups (such as -CO₂Et) or nitriles (-CN) using diisobutylaluminium hydride in a suitable solvent such as toluene. Alternatively, aldehyde groups may be obtained by the oxidation of alcohol groups using any suitable oxidising agent known to those skilled in the art.

Ester groups (-CO₂R') may be converted into the corresponding acid group (-CO₂H) by acid- or base-catalused hydrolysis, depending on the nature of R. If R is *t*-butyl, acid-catalysed hydrolysis can be achieved for example by treatment with an organic acid such as trifluoroacetic acid in an aqueous solvent, or by treatment with an inorganic acid such as hydrochloric acid in an aqueous solvent.

Carboxylic acid groups (-CO₂H) may be converted into amides (CONHR' or - CONR'R") by reaction with an appropriate amine in the presence of a suitable coupling agent, such as HATU, in a suitable solvent such as dichloromethane.

In a further example, carboxylic acids may be homologated by one carbon (i.e - CO₂H to -CH₂CO₂H) by conversion to the corresponding acid chloride (-COCI) followed by Arndt-Eistert synthesis.

In a further example, -OH groups may be generated from the corresponding ester (e.g. -CO₂R'), or aldehyde (-CHO) by reduction, using for example a complex metal hydride such as lithium aluminium hydride in diethyl ether or tetrahydrofuran, or sodium borohydride in a solvent such as methanol. Alternatively, an alcohol may be prepared by reduction of the corresponding acid (-CO₂H), using for example lithium aluminium hydride in a solvent such as tetrahydrofuran, or by using borane in a solvent such as tetrahydrofuran.

Alcohol groups may be converted into leaving groups, such as halogen atoms or sulfonyloxy groups such as an alkylsulfonyloxy, e.g. trifluoromethylsulfonyloxy or arylsulfonyloxy, e.g. p-toluenesulfonyloxy group using conditions known to those skilled in the art. For example, an alcohol may be reacted with thioyl chloride in a halogenated hydrocarbon (e.g. dichloromethane) to yield the corresponding chloride. A base (e.g. triethylamine) may also be used in the reaction.

In another example, alcohol, phenol or amide groups may be alkylated by coupling a phenol or amide with an alcohol in a solvent such as tetrahydrofuran in the presence of a phosphine, e.g. triphenylphosphine and an activator such as diethyl-, diisopropyl, or dimethylazodicarboxylate. Alternatively alkylation may be achieved by deprotonation using a suitable base e.g. sodium hydride followed by subsequent addition of an alkylating agent, such as an alkyl halide.

Aromatic halogen substituents in the compounds may be subjected to halogen-metal exchange by treatment with a base, for example a lithium base such as n-butyl or *t*-butyl lithium, optionally at a low temperature, e.g. around -78 °C, in a solvent such as tetrahydrofuran, and then quenched with an electrophile to introduce a desired substituent. Thus, for example, a formyl group may be introduced by using *N,N*-dimethylformamide as the electrophile. Aromatic halogen substituents may alternatively be subjected to metal (e.g. palladium or copper) catalysed reactions, to introduce, for example, acid, ester, cyano, amide, aryl, heteraryl, alkenyl, alkynyl, thio- or amino substituents. Suitable procedures which may be employed include those described by Heck, Suzuki, Stille, Buchwald or Hartwig.

Aromatic halogen substituents may also undergo nucleophilic displacement following reaction with an appropriate nucleophile such as an amine or an alcohol. Advantageously, such a reaction may be carried out at elevated temperature in the presence of microwave irradiation.

The compounds of the present invention are tested for their capacity to inhibit chk1 activity and activation (primary assays) and for their biological effects on growing cells (secondary assays) as described below. The compounds having IC₅₀ of less than 10 µM (more preferably less than 5 µM, even more preferably less than 1 µM, most preferably less than 0.5 µM) in the chk1 activity and activation assay of Example i, and EC₅₀ of less than 10 µM (more preferably less than 5 µM, most preferably less than 1 µM) in the cellular assay of Example ii, are useful as chk1 inhibitors.

The present invention includes a composition (e.g., a pharmaceutical composition) comprising a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) and a carrier (a pharmaceutically acceptable carrier). The present invention also includes a composition (e.g., a pharmaceutical composition) comprising a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) and a carrier (a pharmaceutically acceptable carrier), further comprising a second chemotherapeutic agent such as those described herein. The present invention also includes a composition (e.g., a pharmaceutical composition) comprising a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) and a carrier (a pharmaceutically acceptable carrier), further comprising a second chemotherapeutic agent such as a DNA damaging agent including those described herein. The present compositions are useful for inhibiting abnormal cell growth or treating a hyperproliferative disorder such as cancer in a mammal (e.g., human). For example, the present compounds and compositions are useful for treating breast cancer, colorectal cancer, ovarian cancer, non-small cell lung cancer, malignant brain tumors, sarcomas, melanoma, lymphoma, myelomas and/or leukemia in a mammal (e.g., human).

The present invention includes a method of inhibiting abnormal cell growth or treating a hyperproliferative disorder such as cancer in a mammal (e.g., human) comprising administering to said mammal a therapeutically effective amount of a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) or a composition thereof. For example, the present invention includes a method of treating breast cancer, colorectal cancer, ovarian cancer, non-small cell lung cancer, malignant brain tumors, sarcomas, melanoma, lymphoma, myelomas and/or leukemia in a mammal (e.g., human), comprising administering to said mammal a therapeutically effective amount of a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) or a composition thereof.

The present invention includes a method of inhibiting abnormal cell growth or treating a hyperproliferative disorder such as cancer in a mammal (e.g., human) comprising administering to said mammal a therapeutically effective amount of a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) or a composition thereof, in combination with a second chemotherapeutic agent such as those described herein. The present invention also includes a method of inhibiting abnormal cell growth or treating a hyperproliferative disorder such as cancer in a mammal (e.g., human) comprising administering to said mammal a therapeutically effective amount of a compound of Formula (**I**), (**I-a**), (**I-b**), (**I-c**), (**I-d**), (**I-e**), (**I-f**), (**I-g**) and/or (**I-h**) (and/or solvates, hydrates and/or salts thereof) or a composition thereof, in combination with a second chemotherapeutic agent such as a DNA damaging agent including those described herein. For example, the present invention includes a method of treating breast cancer, colorectal cancer, ovarian cancer, non-small cell lung cancer, malignant brain tumors, sarcomas, melanoma, lymphoma, myelomas and/or leukemia in a mammal (e.g., human), comprising administering to said mammal a therapeutically effective amount of a compound of Formula (**I**), (**I-a**), (**I-b**), (**I-c**), **(I-d),** (**I-e**), (**If**), (**I-g**) and/or (**I-h**) (and/or solvates, hydrates and/or salts thereof) or a composition thereof, in combination with a second chemotherapeutic agent such as those described herein, the present invention includes a method of treating breast cancer, colorectal cancer, ovarian cancer, non-small cell lung cancer, malignant brain tumors, sarcomas, melanoma, lymphoma, myelomas and/or leukemia in a mammal (e.g., human), comprising administering to said mammal a therapeutically effective amount of a compound of Formula (**I**), (**I-a**), (**I-b**), (**I-c**), (**I-d**), (**I-e**), (**If**), (**I-g**) and/or (**I-h**) (and/or solvates, hydrates and/or salts thereof) or a composition thereof, in combination with a second chemotherapeutic agent such as a DNA damaging agent including those described herein.

The present invention includes a method of using the present compounds for *in vitro*, *in situ*, and *in vivo* diagnosis or treatment of mammalian cells, organisms, or associated pathological conditions.

Administration of the compounds of the present invention (hereinafter the "active compound(s)") can be effected by any method that enables delivery of the compounds to the site of action. These methods include oral routes, intraduodenal routes, parenteral injection (including intravenous, subcutaneous, intramuscular, intravascular or infusion), topical, inhalation and rectal administration.

The amount of the active compound administered will be dependent on the subject being treated, the severity of the disorder or condition, the rate of administration, the disposition of the compound and the discretion of the prescribing physician. However, an effective dosage is in the range of about 0.001 to about 100 mg per kg body weight per day, preferably about 1 to about 35 mg/kg/day, in single or divided doses. For a 70 kg human, this would amount to about 0.05 to 7 g/day, preferably about 0.05 to about 2.5 g/day. In some instances, dosage levels below the lower limit of the aforesaid range may be more than adequate, while in other cases still larger doses may be employed without causing any harmful side effect, provided that such larger doses are first divided into several small doses for administration throughout the day.

The active compound may be applied as a sole therapy or in combination with one or more chemotherapeutic agents, for example those described herein. Such conjoint treatment may be achieved by way of the simultaneous, sequential or separate dosing of the individual components of treatment.

The pharmaceutical composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition will include a conventional pharmaceutical carrier or excipient and a compound according to the invention as an active ingredient. In addition, it may include other medicinal or pharmaceutical agents, carriers, adjuvants, etc.

Exemplary parenteral administration forms include solutions or suspensions of active compounds in sterile aqueous solutions, for example, aqueous propylene glycol or dextrose solutions. Such dosage forms can be suitably buffered, if desired.

Suitable pharmaceutical carriers include inert diluents or fillers, water and various organic solvents. The pharmaceutical compositions may, if desired, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefore, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

Methods of preparing various pharmaceutical compositions with a specific amount of active compound are known, or will be apparent, to those skilled in this art. For examples, see Remington's Pharmaceutical Sciences, Mack Publishing Company, Ester, Pa., 15.sup.th Edition (1975).

### EXAMPLES

### Abbreviations

- DCM: Dichloromethane
- DIPEA: Diisopropylethylamine
- DMSO: Dimethylsulfoxide
- DMF: Dimethylformamide
- EtOH: Ethanol
- HCl: Hydrochloric acid
- HM-N: Isolute® HM-N is a modified form of diatomaceous earth that can efficiently absorb aqueous samples
- IMS: industrial methylated spirits
- MeOH: Methanol
- POCl₃: Phosphorus oxychloride
- NaHCO₃: Sodium bicarbonate
- NaOH: Sodium hydroxide
- Pd(PPh₃)₄: Tetrakis(triphenylphosphine)palladium(0)
- NEt₃: Triethylamine
- Pd₂dba₃: Tris-(dibenzylideneacetone)dipalladium(0)
- Si-SPE: Pre-packed Isolute® silica flash chromatography cartridge
- Si-ISCO: Pre-packed ISCO® silica flash chromatography cartridge
- THF: Tetrahydrofuran

### General Experimental Conditions

¹H NMR spectra were recorded at ambient temperature using a Varian Unity Inova (400MHz) spectrometer with a triple resonance 5mm probe. Chemical shifts are expressed in ppm relative to tetramethylsilane. The following abbreviations have been used: br = broad signal, s = singlet, d = doublet, dd = double doublet, t = triplet, q = quartet, m = multiplet.

High Pressure Liquid Chromatography - Mass Spectrometry (LCMS) experiments to determine retention times (R_{T}) and associated mass ions were performed using one of the following methods.

Method A: Experiments performed on a Waters Micromass ZQ quadrupole mass spectrometer linked to a Hewlett Packard HP1100 LC system with diode array detector. This system uses a Higgins Clipeus 5micron C18 100 x 3.0mm column and a 1 ml / minute flow rate. The initial solvent system was 95% water containing 0.1% formic acid (solvent A) and 5% acetonitrile containing 0.1% formic acid (solvent B) for the first minute followed by a gradient up to 5% solvent A and 95% solvent B over the next 14 minutes. The final solvent system was held constant for a further 5 minutes.

Method B: Experiments performed on a Waters Platform LC quadrupole mass spectrometer linked to a Hewlett Packard HP1100 LC system with diode array detector and 100 position autosampler using a Phenomenex Luna C18(2) 30 x 4.6mm column and a 2 ml / minute flow rate. The solvent system was 95% water containing 0.1% formic acid (solvent A) and 5% acetonitrile containing 0.1% formic acid (solvent B) for the first 0.50 minutes followed by a gradient up to 5% solvent A and 95% solvent B over the next 4 minutes. The final solvent system was held constant for a further 0.50 minutes.

Microwave experiments were carried out using a Biotage Initiator 60™ which uses a single-mode resonator and dynamic field tuning. Temperature from 40-250°C can be achieved, and pressures of up to 30 bar can be reached.

**EXAMPLE i** Chk1 and chk2 Assays (chk primary assays)

Full length human mutant recombinant protein, histidine tagged and expressed in insect cells is used as source of enzymatic activity (Invitrogen, chk1 from product PV3982 and chk2 from product PV3983).

The chk1 AlphaScreen assay is carried out for 30 minutes in the presence of 10µM ATP using biotinylated Akt substrate-1 peptide (Cell Signalling Technology, product #1065) as a substrate. Phosphorylation of the substrate is detected and quantified using AlphaScreen technology. This consists of an anti-phospho-Akt substrate-1 antibody (Cell Signalling technology Product #9611) and two AlphaScreen beads (Perkin Elmer), one product coated with Protein A which binds the antibody Ig chain (Product 6760137), and one coated with Streptavidin which binds the biotin on the biotinylated Akt substrate peptide-1 (Product 6760002). Chk1 activity results in the production of phosphorylated Akt substrate peptide-1 an event which causes the two bead species to be brought into close proximity in the presence of antibody leading to the generation of luminescence which is detected on a Perkin Elmer reader (Fusion).

The ATP Radiometric ChK1 assay is carried out by incubation for 30 minutes in the presence of 10µM ATP containing 0.3 µCi ³³P-ATP per sample and using ChKTide (peptide sequence KKKVSRSGLYRSPSMPENLNRPR) as a substrate. Following acidification with 1% phosphoric acid and washing to remove unincorporated ATP, phosphorylation of the substrate is detected and quantified by measurement of radioactivity incorporated using a Perkin Elmer Topcount.

The chk2 assay is carried out for 30 minutes in the presence of 30µM ATP using biotinylated tyrosine hydroxylase (ser 40) peptide (Cell Signalling Technology, product # 1132) as a substrate. Phosphorylation of the substrate is detected and quantified using AlphaScreen technology. This consists of an anti-phospho-tyrosine hydroxylase (ser 40) peptide antibody (Cell Signalling technology Product #2791) and two AlphaScreen beads (Perkin Elmer), one product coated with Protein A which binds the antibody Ig chain (Product 6760137), and one coated with Streptavidin which binds the biotin on the biotinylated tyrosine hydroxylase (ser 40) peptide (Product 6760002). Chk2 activity results in the production of phosphorylated tyrosine hydroxylase peptide an event which causes the two bead species to be brought into close proximity in the presence of antibody leading to the generation of luminescence which is detected on a Perkin Elmer reader (Fusion).

The ATP radiometric ChK2 assay is carried out by incubation for 30 minutes in the presence of 30µM ATP containing 0.3µCi ³³P-ATP per sample and using ChKTide (peptide sequence KKKVSRSGLYRSPSMPENLNRPR) as a substrate. Following acidification with 1% phosphoric acid and washing to remove unincorporated ATP, phosphorylation of the substrate is detected and quantified by measurement of radioactivity incorporated using a Perkin Elmer Topcount.

Test compounds are diluted in DMSO prior to addition to assay buffer, the final DMSO concentration in the assay is 1%.

The IC₅₀ is defined as the concentration at which a given test compound achieved 50% inhibition of the control. IC₅₀ values are calculated using the XLfit software package (version 2.0.5).

Tested title compounds of EXAMPLES 1-25 exhibited an IC₅₀ of less than 5 µM in the assays described in EXAMPLE i against chk 1.

**EXAMPLE ii** Cellular Assay (Checkpoint Abrogation)

Compounds are tested in a cellular assay using the human colorectal adenocarcinoma derived cell line HT-29 (ATCC HTB-38).

The cell line is maintained in DMEM/F12 (1:1) media (Invitrogen Gibco, # 31331) supplemented with 10% FCS at 37 °C in a 5% CO₂ humidified incubator.

Cells are seeded in 96-well plates at 30,000 cells/well and after 24 h they are exposed to 20nM SN-38 in 0.4% DMSO. One column of 8 wells on each plate was used to generate a maximum signal control. These cells are treated with 0.4% DMSO without SN-38. Cells are grown for a further 16h, then the media containing DMSO plus or minus SN-38 is removed and replaced with media containing 300nM Nocodazole alone (to determine baseline) or in combination with ten concentrations of chk1 inhibitor (final DMSO concentration is 0.4%). Cells are grown for a further 24 h. The media is removed and replaced with 50 µl lysis buffer containing protease inhibitors and phosphatase inhibitors. This buffer contains detergent to bring about cellular disruption. Following complete cellular disruption, 25 µl lysate is transferred to a MesoScale 96 well 4-spot plate coated with an antibody to Histone H3 (MesoScale Discovery (MSD) Product K110WA-3) which have been previously blocked with 3% bovine serum albumin in Tris buffered saline. Following the transfer of lysate to the MSD plate, Histone H3 in the lysate is captured on the coated antibody by incubation at room temperature for 2 h. Following the capture step the plate is washed and then incubated with an antibody to phosphorylated Histone H3 which is conjugated with a Sulfo-Tag. This tag gives a signal when in proximity to the electrode on the base of the MSD plate. Binding the tagged antibody to the captured protein allow detection on a MSD reader.

The EC₅₀ is defined as the concentration at which a given compound achieves 50% decrease of the measured levels of phospho-Histone H3 within the range of a normal sigmoidal dose response curve compared to the signal generated by 300nM Nocodazole alone. EC₅₀ values are calculated using the XLfit software package (version 2.0.5) or Graphpad Prism, (version 3.03) fitting a sigmoidal curve with a variable slope.

Tested title compounds of EXAMPLES 1-25 exhibited an EC₅₀ of less than 10 µM in the assay described in EXAMPLE ii.

3-Amino-5-bromo-1H-pyrrolo[2,3-b]pyridine-2-carboxylic acid amide

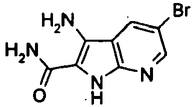

Step 1: 2-(3-Cyano-pyridin-2-ylamino)-acetamide

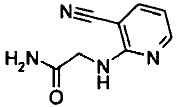

Following the procedure of Kaspersen, Frans M. et al, Journal of Labelled Compounds and Radiopharmaceuticals (1989), 27(9), 1055-68, glycinamide (8.8 g, 79.4 mmol) and sodium carbonate (4.6 g, 43.3 mmol) were suspended in DMSO (200 ml) and stirred at ambient temperature for 16 h. The solid was removed by filtration through celite, the filtrate treated with 2-chloronicotinonitrile (10.0 g, 72.2 mmol) and potassium fluoride (10.0 g, 173.3 mmol) and heated at 120 °C for 4 h. The mixture was allowed to cool to ambient temperature then diluted with water (800 ml). The precipitated solid was collected by filtration, washed with dichloromethane (50 ml) and water (50 ml), then triturated with diethyl ether (100 ml), filtered and left to air dry which afforded the title compound as an off white solid (6.7 g, 53%). ¹H NMR (DMSO-D₆, 400MHz) 8.26 (dd, J = 4.9Hz, 1.8Hz, 1H), 7.93 (dd, J = 7.6Hz, 1.8Hz, 1H), 7.40 (s, 1H), 7.12 (t, J = 4.9Hz, 1H), 7.00 (s, 1H), 6.69 (dd, J = 7.6Hz, 4.9Hz, 1H), 3.34 (s, 2H).

Step 2: 2-(5-Bromo-3-cyano-pyridin-2-ylamino)-acetamide

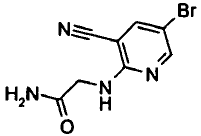

A solution of *N*-bromosuccinamide (7.1 g, 38.2 mmol) in *N,N-*dimethylformamide (20 ml) was added dropwise over 25 minutes to a suspension of 2-(3-cyano-pyridin-2-ylamino)-acetamide in *N,N*-dimethylformamide (30 ml). On complete addition the mixture was allowed to stir at ambient temperature for 16 h then poured onto water (400 ml). The precipitated solid was collected by filtration, washed with water (50 ml) and left to air dry which gave the title compound as a white solid (8.35 g, 96%). ¹H NMR (DMSO-D₆, 300MHz) 8.35 (d, J = 2.5Hz, 1H), 8.24 (d, J = 2.5Hz, 1H), 7.36-7.44 (m, 2H), 7.01 (s, 1H), 3.85 (d, J = 5.6Hz, 1H). LCMS (method B): R_{T} = 2.23 min, M+H⁺ = 255/257.

Step 3: 3-Amino-5-bromo-1H-pyrrolo[2,3-b]pyridine-2-carboxylic acid amide

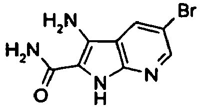

A suspension of 2-(5-bromo-3-cyano-pyridin-2-ylamino)-acetamide (8.35 g, 32.7 mmol) and sodium hydrogen carbonate (5.5 g, 65.5 mmol) in ethanol (150 ml) was heated under reflux for 66 h. The mixture was allowed to cool to ambient temperature then cooled further in an ice/water bath. The precipitated solid was collected by filtration, washed with ethanol (15 ml), water (2 x 20 ml), ethanol (20 ml) and diethyl ether (20 ml) and left to air dry to afford the title compound as a yellow solid (5.9 g, 71%). ¹H NMR (DMSO-D₆, 300MHz) 8.38 (d, J = 2.3Hz, 1H), 8.32 (d, J = 2.3Hz, 1H), 7.19 (s, 2H), 5.82 (s, 2H). LCMS (method B): R_{T} = 2.38 min, M+H⁺ = 255/257.

### General Acid Chloride Preparation

Acid chloride intermediates unless otherwise stated were either commercially available, prepared using literature methods or could be readily prepared by those skilled in the art.

1-(4-Methoxy-benzyl)-1H-pyrazole-4-carbonyl chloride

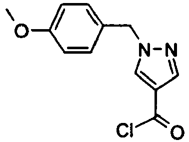

Step 1: 1-(4-Methoxy-benzyl)-1H-pyrazole-4-carboxylic acid ethyl ester

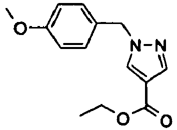

A solution of 1H-pyrazole-4-carboxylic acid ethyl ester (0.55 g, 3.95 mmol), 4-methoxybenzyl bromide (0.74 g, 4.74 mmol) and potassium carbonate (1.64 g, 11.9 mmol) in acetone (20 ml) was left to stir at ambient temperature for 16 h then heated at 50 °C for 1 h. The reaction mixture was allowed to cool to ambient temperature, concentrated under reduced pressure then diluted with water and extracted with dichloromethane (4 x 20 ml). The combined organic phase was washed with saturated ammonium chloride solution (20 ml) and brine (20 ml), dried over anhydrous sodium sulfate, filtered and evaporated. The resultant residue was purified by flash column chromatography on silica (ISCO, 40g) eluting with pentane on a gradient of ethyl acetate (0-30%). Collecting appropriate fractions afforded the title compound as a colourless oil (1.0 g, 97%). ¹H NMR (CDCl₃, 300 MHz) 7.92 (s, 1H), 7.81 (s, 1H), 7.33-7.15 (m, 2H), 6.92-6.86 (m, 2H), 5.23 (s, 2H), 4.26 (q, J = 7.1Hz, 2H), 3.81 (s, 3H), 1.29 (t, J = 7.1Hz, 3H). LCMS (method B): R_{T} = 3.27 min, M+H⁺ = 261.

Step 2: 1-(4-Methoxy-benzyl)-1H-pyrazole-4-carboxylic acid

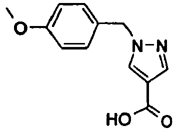

A mixture of 1-(4-methoxy-benzyl)-1H-pyrazole-4-carboxylic acid ethyl ester (0.50 g, 1.91 mmol) and 1M sodium hydroxide (5.7 ml, 5.7 mmol) in IMS (6 ml) was stirred at ambient temperature for 16 h. The reaction mixture was then concentrated to approximately half the original volume. The pH of the concentrate was adjusted to 4 by the addition of 1M hydrochloric acid and the mixture extracted with ethyl acetate (4 x 10 ml). The combined organic phase washed with brine (20 ml), dried over anhydrous sodium sulfate, filtered and evaporated to give the title compound as a white solid (0.44 g, 99%). ¹H NMR (CD₃OD, 300 MHz) 8.11 (s, 1H); 7.87 (s, 1H); 7.30-7.17 (m, 2H); 6.93-6.85 (m, 2H); 5.26 (s, 2H); 3.77 (s, 3H). LCMS (method B): R_{T} = 2.52 min, M+H⁺ = 233.

Step 3: 1-(4-Methoxy-benzyl)-1H-pyrazole-4-carbonyl chloride

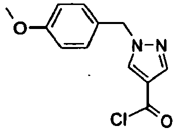

A mixture of 1-(4-methoxy-benzyl)-1H-pyrazole-4-carboxylic acid (0.45 g, 1.94 mmol), oxalyl chloride (1.2 ml) and DMF (1 drop) was heated at 60°C for 1 h. The mixture was allowed to cool to ambient temperature and evaporated to afford the title compound as a colourless oil (0.48 g, 99%). The material was used in the next step without further purification.

### General Method 1

A suspension of 3-amino-5-bromo-1H-pyrrolo[2,3-b]pyridine-2-carboxylic acid amide (1.0 eq.) and the appropriate acid chloride (1.2 eq.) in pyridine (15 ml/mmol) were heated at 80 °C for 18 h. The mixture was allowed to cool to ambient temperature then poured into water. The resultant precipitated solid was collected by filtration, washed with water and diethyl ether and dried at 60 °C under high vacuum to afford the title compound.

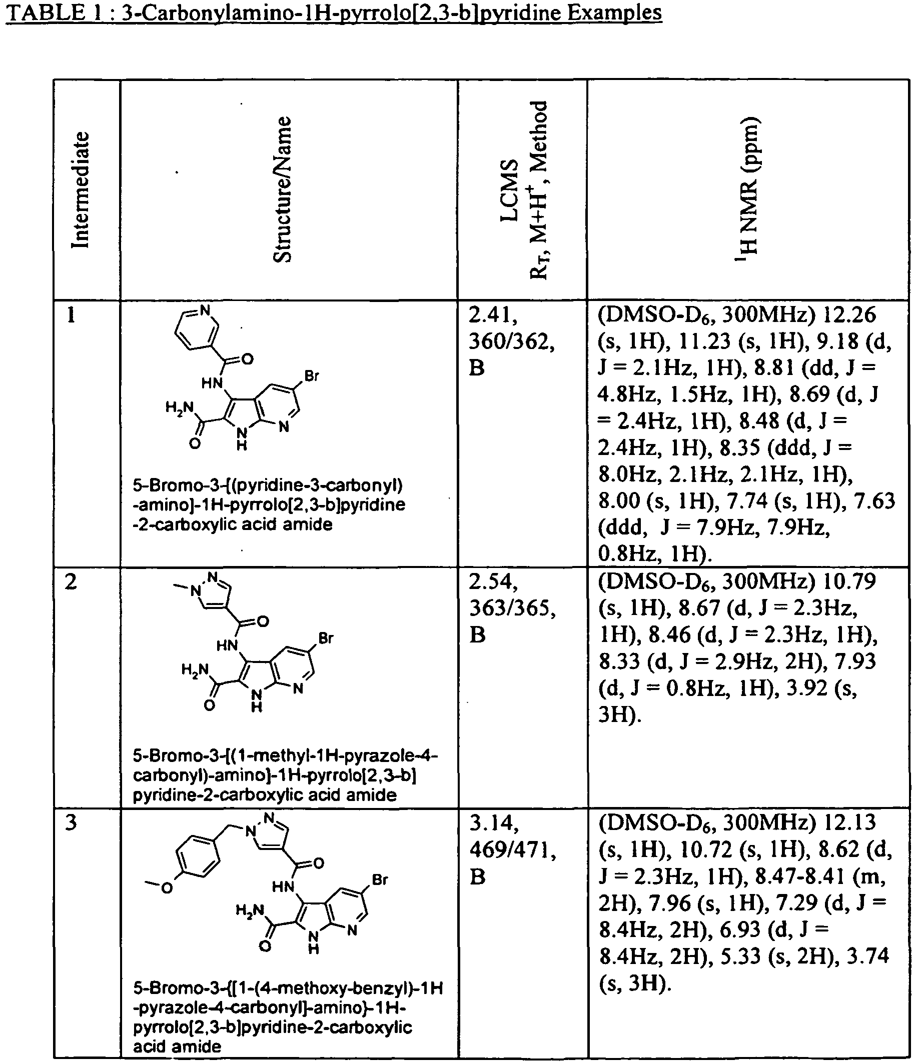

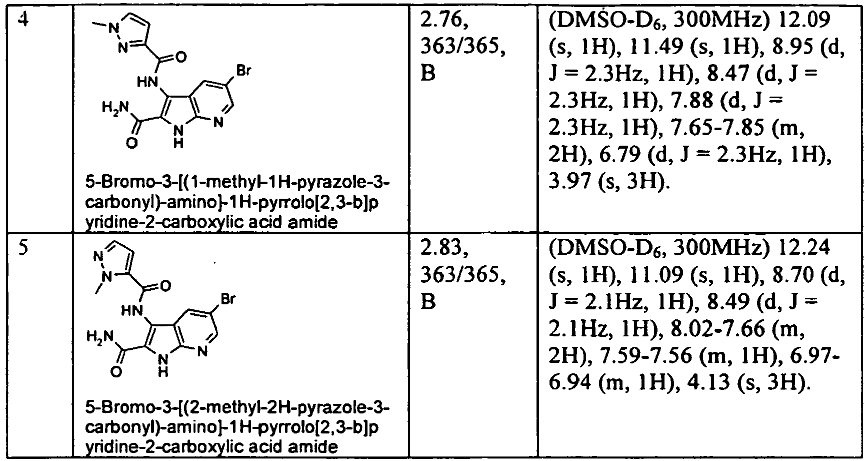

### General Method 2

A suspension of the appropriate 5-bromo-3-carbonylamino-1H-pyrrolo[2,3-b]pyridine-2-carboxylic acid amide (1.0eq.) in 10% w/w aqueous potassium hydroxide (3 ml/mmol) and ethanol (1.5 ml/mmol) was heated under microwave irradiation at 170°C for 1 h. The mixture was allowed to cool to ambient temperature, diluted with water and the resultant precipitated solid was collected by filtration. The solid was washed with water, methanol: diethyl ether and diethyl ether and left to air dry.

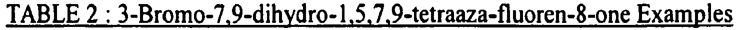

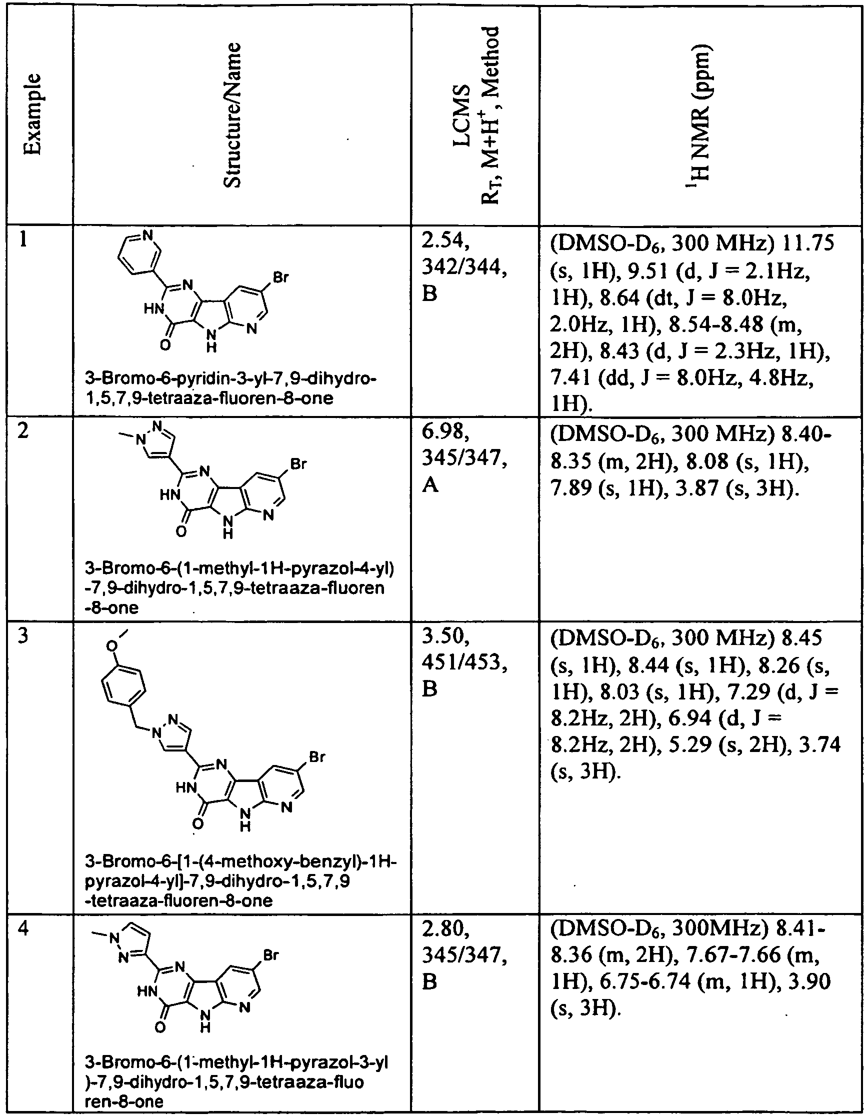

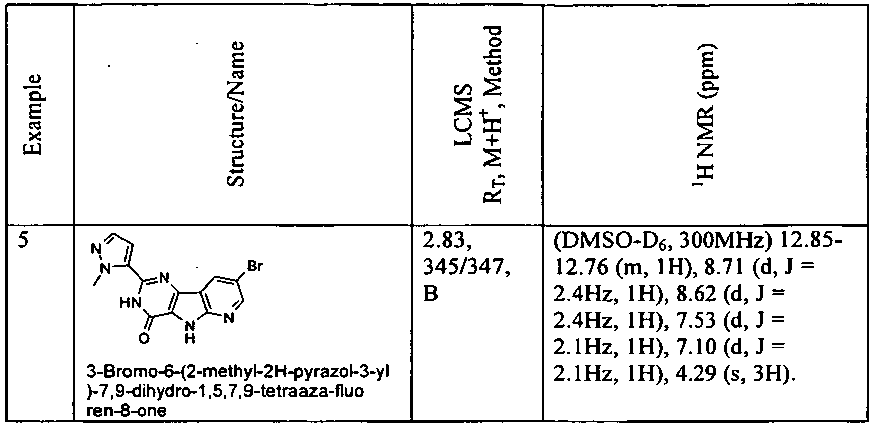

### General Boronic Acid / Boronate Ester Preparation Method

Boronic acids and boronate esters were prepared from the appropriate aryl halide intermediate by using the coupling general method described below. All aryl halide intermediates were either commercially available, prepared using literature methods or could be readily prepared by those skilled in the art. In some cases the intermediate was not isolated, and the coupling reaction performed on the crude boronic acid/ boronate ester. Suzuki reactions were performed using either commercially available boronic acids/ boronate esters or from compounds prepared using the procedures detailed above. If necessary, any protecting groups were then removed using one of the deprotection conditions described below. Stille reactions were performed using either commercially available stannanes or from compounds prepared using the procedures detailed above. If necessary, any protecting groups were then removed using one of the deprotection conditions described below.

Method A: The appropriate aryl halide (1-3 eq) was suspended in a mixture of THF under an inert atmosphere then *n*butyl lithium (1-3 eq) was added at -78°C. After 5-30 minutes at this temperature, trialkylborate (1-3 eq) was added then the reaction mixture was warmed to ambient temperature and quenched by the addition of ammonium chloride. The resultant mixture was purified by one of the general purification methods described below or used crude in the next step.

Method B: The appropriate aryl halide (1-3 eq) was suspended in a mixture of dioxane and DMSO before *bis*(pinacolato)diboron (1-2 eq), aqueous potassium acetate solution and 1,1'-*bis*(diphenylphosphino)-ferrocene]dichloropalladium(II) (5-10 mol%) were added and the reaction mixture was then heated with microwave irradiation (100-160°C) for between 1 and 20 minutes. The resultant mixture was purified by one of the general purification methods described below or used crude in the next step.

Method C: The appropriate electrophile (1-2 eq) and potassium carbonate (3-5 eq) were added to 4,4,5,5-tetramethyl-2(1*H*-pyrazol-4-yl)-1,3,2-dioxaborolane in acetonitrile and the mixture was stirred under reflux for between 1 to 7 days. The mixture was purified by one of the general purification methods described below.

Method D: The appropriate (bromomethyl)phenyl boronic acid (1 eq) was stirred with sodium iodide (0.05 eq), potassium carbonate (3.0 eq) in acetonitrile and the appropriate amine (1.2 eq) added. The mixture was heated to 50 °C for 2 h and then cooled to ambient temperature then the volatile components were removed *in vacuo* and the residue re-suspended in MeOH. The remaining solid was removed by filtration then the methanolic solution was collected and concentrated to dryness under reduced pressure. The resulting boronic acid was used with no further purification.

### General Method 3

A degassed suspension of the appropriate boronic acid / boronate ester (1-3 eq) and the appropriate 6-substituted-3-bromo-7,9-dihydro-1,5,7,9-tetraaza-fluoren-8-one (1.0 eq) in anhydrous acetonitrile (2 ml/mmol) and 1M aqueous sodium carbonate solution (2.5 ml/mmol)was treated with *bis*(triphenylphosphine)palladium(II) dichloride (5-10 mol%) and the reaction mixture was heated under microwave irradiation (100-160 °C) for between 1 and 30 minutes. The resultant residue was purified by one of the general purification methods described below.

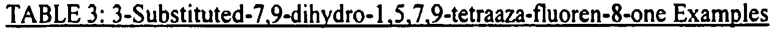

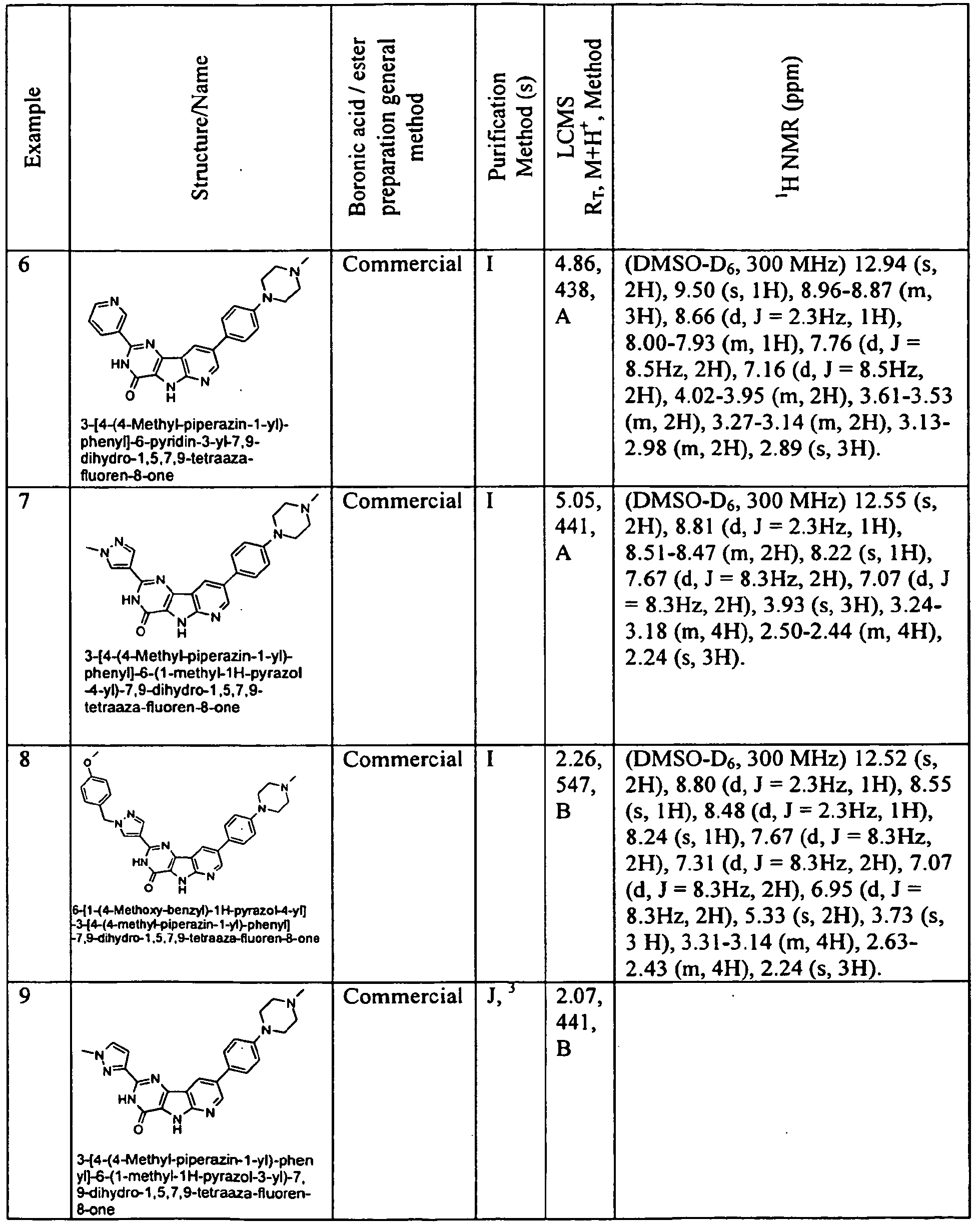

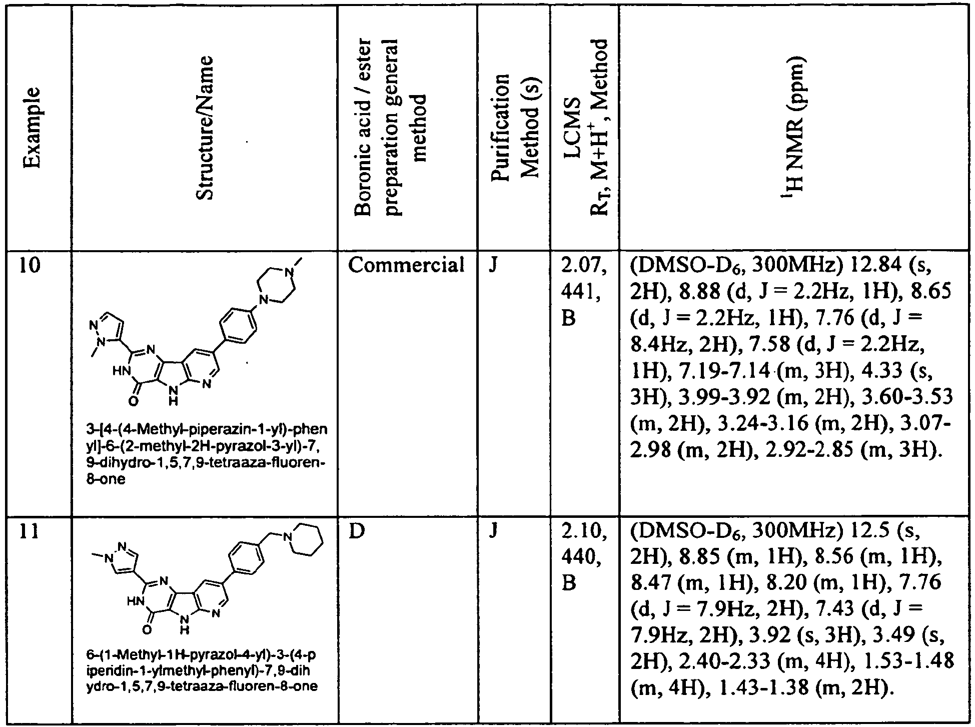

### General Method 4

The appropriate 7,9-dihydro-1,5,7,9-tetraaza-fluoren-8-one (1 eq) was suspended in neat POCl₃ under an inert atmosphere then heated under reflux for between 30 minutes and 18 h or under microwave irradiation at between 120 °C and 180 °C for 20 to 60 minutes. The reaction mixture was allowed to cool to ambient temperature and evaporated. The resultant residue was treated with ice and the pH of the aqueous phase was adjusted to between 7 and 9 by the addition of saturated sodium hydrogen carbonate solution. The resultant solid was collected by filtration, washed with water and diethyl ether and used crude in the next step.

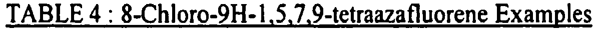

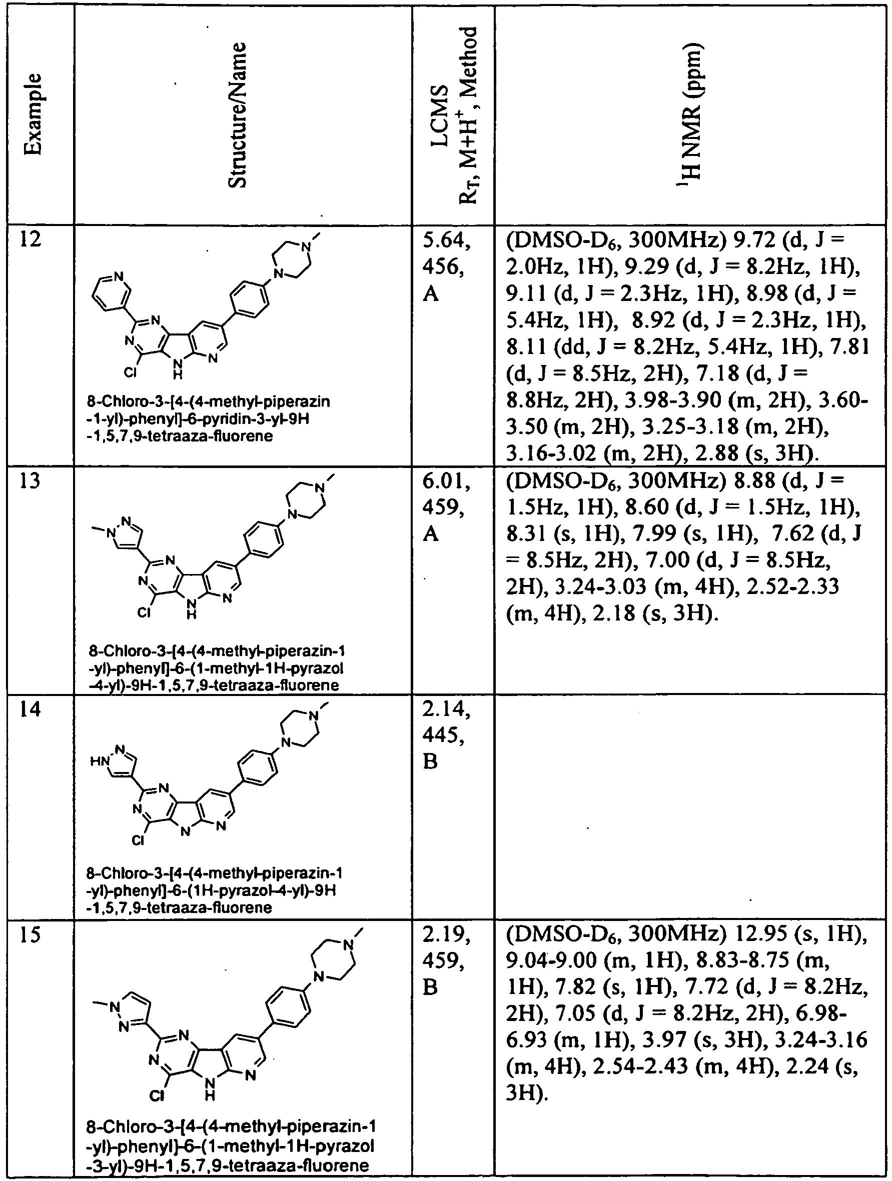

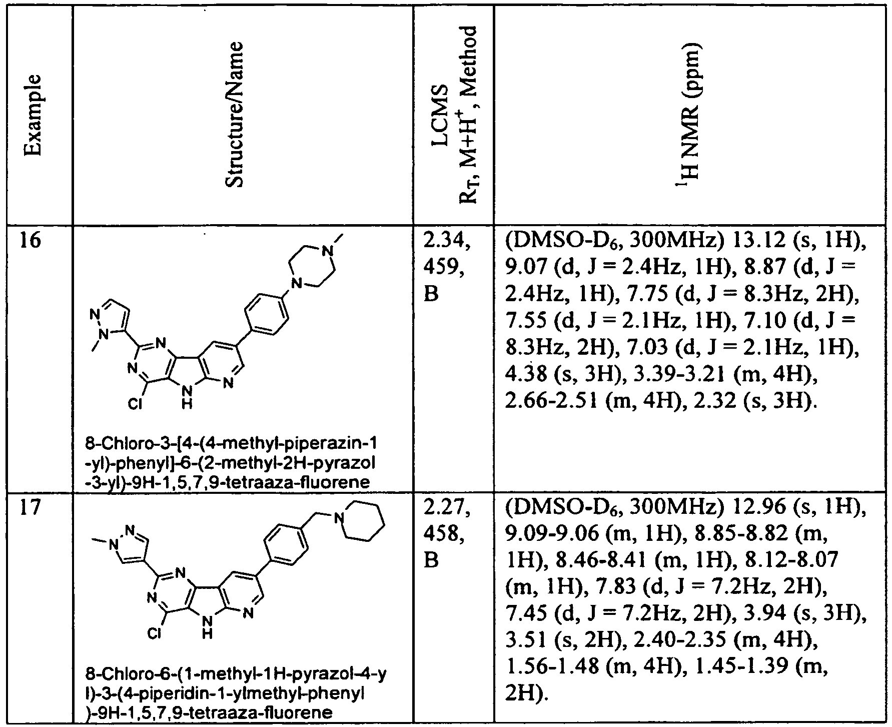

### General Method 5 : Reduction of Chloro Intermediates

Method A : The appropriate 8-chloro-9H-1,5,7,9-tetraaza-fluorene (1.0 eq) and Pd/C (10% w/w, 0.2 eq) were suspended in DMF/EtOH/NEt₃ (20-40 ml/mmol) and hydrogenated under an atmosphere of hydrogen for between 1 h and 18 h. The catalyst was removed by filtration, and the filtrate evaporated to give a residue. The resultant residue was purified by one of the general purification methods described below.

Method B : Sodium borohydride (3.0 eq) was added to the appropriate 8-chloro-9H-1,5,7,9-tetraaza-fluorene (1.0 eq) in methanol (300-400 ml/mmol) and the resultany mixture was stirred at ambient temperature, with further addition of sodium borohydride if required, until analysis (TLC/LCMS) showed the reaction to have gone to completion. Water was added to the reaction mixture and then the mixture wasconcentrated to dryness under reduced pressure. The resultant residue was purified by one of the general purification methods described below.

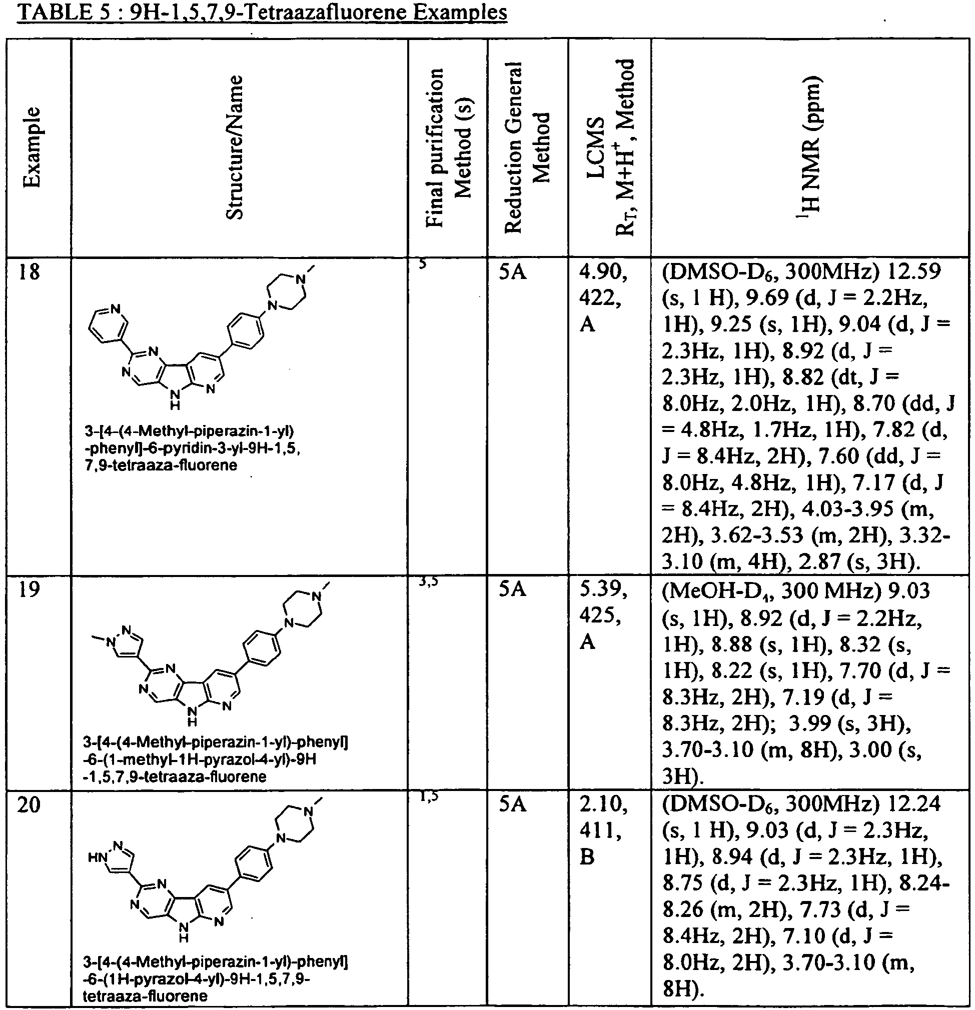

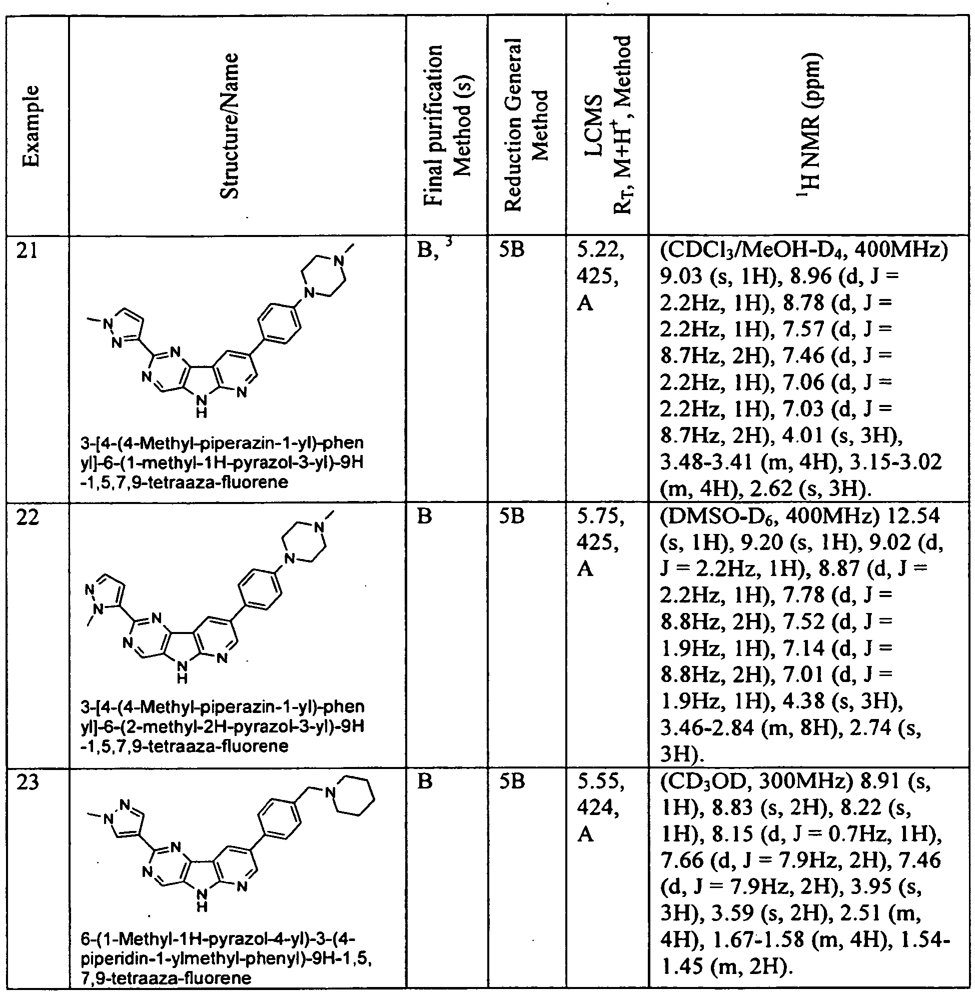

### Purification general methods

Method A: Si-SPE or Si-ISCO, ethyl acetate/DCM gradient
Method B: Si-SPE or Si-ISCO, methanol/DCM gradient
Method C : A solution of the substrate in methanol was loaded onto an Isolute® SCX-2 cartridge. The cartridge was then washed with methanol before the desired product was eluted using 2M ammonia in MeOH.
Method D: reversed phase HPLC Phenomenex Gemini C18, 20 mM triethylamine in water on a gradient of acetonitrile
Method E: Si-SPE or Si-ISCO, 2M NH3 in methanol/DCM gradient
Method F: ethyl acetate / methanol recrystallisation
Method G: Solid isolated from reaction mixture and washed thoroughly with water.
Method H: Si-SPE or Si-ISCO, 2M ammonia in methanol/DCM gradient Method I: Reaction mixture was diluted with water, filtered and the resulting solid washed with THF
Method J: Reaction mixture was diluted with water, filtered and the resulting solid washed with water and diethyl ether.

### Deviations from general methods:

¹ Triturated in methanol; ² triturated in ethyl acetate, ³ triturated in acetonitrile; ⁴ recrystallised from DMSO-water; ⁵ triturated in diethyl ether.

Example 24 : 8-Methyl-3-[4-(4-methyl-piperazin-1-yl)-phenyl]-6-(1-methyl-1H-pyrazol-4-yl)-9H-1,5,7,9-tetraaza-fluorene

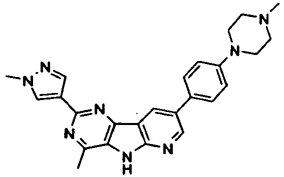

Tetramethyltin (0.023 ml, 0.168 mmol) was added to a degassed suspension of 8-chloro-3-[4-(4-methyl-piperazin-1yl)-phenyl]-6-(1-methyl-1H-pyrazol-4-yl)-9H-1,5,7,9-tetraaza-fluorene [13] (70 mg, 0.153 mmol), lithium chloride (19 mg, 0.458 mmol) and bis(triphenylphosphine)palladium(II) dichloride (11 mg, 0.015 mmol) in DMF (2 ml) and was heated under microwave irradiation at 140 °C for 20 minutes. The reaction mixture was diluted with water (10 ml), the precipitated solid was removed by filtration, then washed with water and diethyl ether. The filtrate was concentrated under reduced pressure and the resultant residue triturated with diethyl ether then methanol to give the title compound as a cream solid (29 mg, 43%). ¹H NMR (DMSO-D₆, 400MHz) 12.35 (s, 1H), 8.93 (d, J = 2.2Hz, 1H), 8.68 (d, J = 2.2Hz, 1H), 8.35 (s, 1H), 8.06 (s, 1H), 7.70 (d, J = 8.8Hz, 2H), 7.07 (d, J = 8.8Hz, 2H), 3.91 (s, 3H), 3.21 (t, J = 4.9Hz, 4H), 2.79 (s, 3H), 2.47-2.45 (m, 4H), 2.23 (s, 3H). LCMS (method A): R_{T} = 5.55 min, M+H⁺= 439.

Example 25 : 8-[4-(4-Methyl-piperazin-1-yl)-phenyl]-5H-dipyrido[2,3-b;2',3'-d]pyrrole-2-carbonitrile

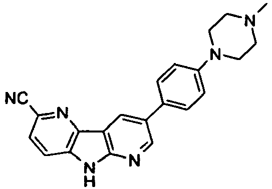

Step 1: 3-Amino-2'-fluoro-[2,3']bipyridinyl-6-carbonitrile

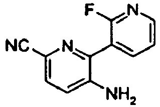

A degassed suspension of 5-amino-6-bromo-pyridine-2-carbonitrile (293 mg, 1.48 mmol), 2-fluoropyridine boronic acid (250 mg, 1.77 mmol) and *bis*-(triphenylphosphine) palladium(II) dichloride (104 mg, 0.15 mmol) in 1N aqueous sodium carbonate (2.5 ml) and acetonitrile (2.5 ml) was heated under microwave irradiation at 140 °C for 25 minutes. The mixture was allowed to cool to ambient temperature and partitioned between ethyl acetate (75 ml) and water (30 ml). The organic layer was separated, dried over anhydrous sodium sulfate, filtered and evaporated. The resultant residue was purified by flash column chromatography on silica (ISCO, 40g) eluting with cyclohexane on a gradient of ethyl acetate (20-100%). Collecting appropriate fractions afforded the title compound as a white solid (190 mg, 60%). ¹H NMR (DMSO-D₆, 300MHz) 8.35 (ddd, J = 4.9Hz, 2.0Hz, 1.1 Hz, 1H), 8.03 (ddd, J = 9.4Hz, 7.4Hz, 2.0Hz, 1H), 7.54 (d, J = 8.4Hz, 1H), 7.41 (ddd, J = 7.4Hz, 4.9Hz, 2.1 Hz, 1H), 7.12 (d, J = 8.4Hz, 1H). LCMS (method B): R_{T} = 2.27 min, M+H⁺ = 215.

Step 2: 5H-Dipyrido[2,3-b;2',3'-d]pyrrole-2-carbonitrile

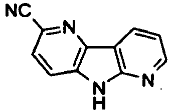

Sodium hexamethyldisilazane (1M in THF, 1.33 ml, 1.33 mmol) was added dropwise over 5 minutes to a solution of 3-amino-2'-fluoro-[2,3']bipyridinyl-6-carbonitrile (190 mg, 0.89 mmol) in THF (10 ml). On complete addition, the mixture was heated at 50°C for 18 h. The mixture was allowed to cool to ambient temperature then water (50 ml) and ethyl acetate (50 ml) were added. The precipitated solid was collected by filtration, washed with water (10 ml) and diethyl ether (20 ml) and left to air dry which afforded the title compound as a brown solid (95 mg, 55%). ¹H NMR (DMSO-D₆, 400MHz) 8.70-8.66 (m, 2H), 8.11-8.01 (m, 2H), 7.42 (dd, J = 7.7Hz, 4.9Hz, 1H). LCMS (method B): R_{T} = 2.43 min, M+H⁺ = 195.

Step 3: 8-Bromo-5H-dipyrido[2,3-b;2',3'-d]pyrrole-2-carbonitrile

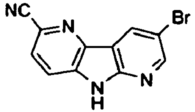

Bromine (0.06 ml, 1.16 mmol) was added dropwise to a suspension of 5H-dipyrido[2,3-b;2',3'-d]pyrrole-2-carbonitrile (75 mg, 0.39 mmol) and sodium acetate (98 mg, 1.19 mmol) in acetic acid (10 ml). On complete addition the mixture was heated at 80 °C for 30 minutes then allowed to cool to ambient temperature. The reaction mixture was evaporated, the resultant residue treated with saturated aqueous sodium thiosulfate solution (2 ml) followed by water (5 ml) and the pH of the aqueous phase was then adjusted to 9 by the addition of saturated aqueous sodium hydrogen carbonate solution. The solid was collected by filtration, washed with water (5 ml) and diethyl ether (10 ml) then dried at 50 °C under high vacuum to afford the title compound as an off white solid (95 mg, 89%). ¹H NMR (DMSO-D₆, 300MHz) 8.91 (d, J = 2.3Hz, 1H), 8.75 (d, J = 2.3Hz, 1H), 8.14-8.06 (m, 2H). LCMS (method B): R_{T} = 3.08 min, M+H⁺ = 274/276.

Step 4 : 8-[4-(4-Methyl-piperazin-1-yl)-phenyl-5H-dipyrido[2,3-b;2',3'-d]pyrrole-2-carbonitrile

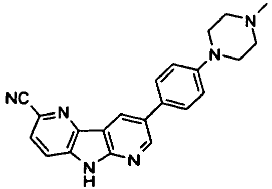

A degassed suspension of 8-bromo-5H-dipyrido[2,3-b;2',3'-d]pyrrole-2-carbonitrile (92 mg, 0.34 mmol), 4-(4-methylpiperazin-1-yl)phenylboronic acid, pinacol ester (122 mg, 0.40 mmol) and *bis*(triphenylphosphine) palladium (II) dichloride (12 mg, 0.02 mmol) in 1N aqueous sodium carbonate (2 ml) and acetonitrile (2 ml) was heated under microwave irradiation at 140 °C for 20 minutes then allowed to cool to ambient temperature. The precipitated solid was collected by filtration, washed with acetonitrile (2 ml), water (2 ml) and diethyl ether (5 ml) and left to air dry. The solid was preadsorbed onto HM-N and purified by flash column chromatography on silica (ISCO, 12g) eluting with dichloromethane on a gradient of methanol (0-20%). Collecting appropriate fractions followed by trituration with hot methanol afforded the title compound as a yellow solid (65 mg, 52%). ¹H NMR (DMSO-D₆, 300MHz) 8.93 (d, J = 2.3Hz, 1H), 8.82 (d, J = 2.3Hz, 1H), 8.09-8.02 (m, 2H), 7.73 (d, J = 8.5Hz, 2H), 7.08 (d, J = 8.5Hz, 2H), 3.26-3.16 (m, 4H), 2.53-2.45 (m, 4H), 2.24 (s, 3H). LCMS (method A): R_{T} = 5.74 min, M+H⁺ = 369.

## Claims

1. A compound of formula (**I**), (**I-a**), (**I-b**), (**I-c**), (**I-d**), (**I-e**), (**I-f**), (**I-g**), or (**I-h**), or a solvate, hydrate, or salt thereof: 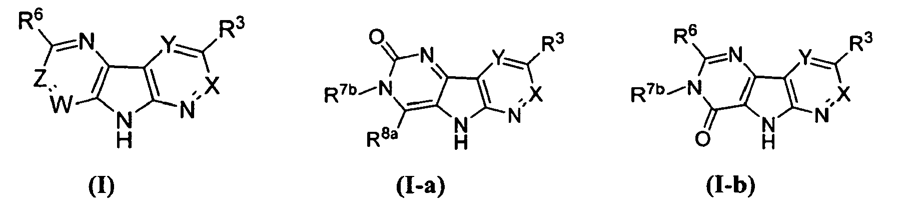 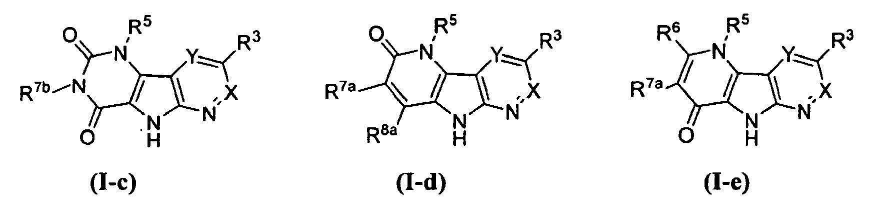 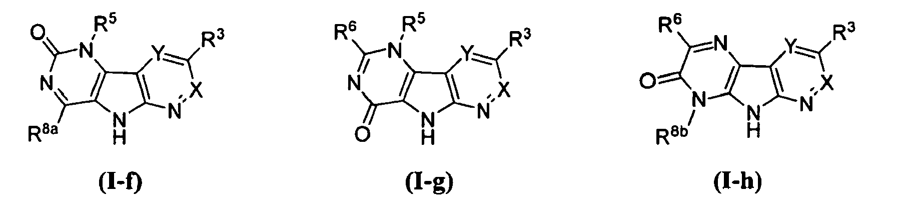
X is CR² or N;
Y is CR⁴ or N;
Z is CR^{7a} or N;
W is CR^{8a} or N; provided that (i) Z and W are not both N at the same time and (ii) X and Y are not both N at the same time;
R² is H, halo, CN, CF₃, -OCF₃, OH, -NO₂, C₁-C₅ alkyl, -O(C₁-C₅ alkyl), -S(C₁-C₅ alkyl), or N(R²²)₂;
R³ is H, halo, -O-R⁹, -N(R²²)-R⁹, -S(O)ₚ-R⁹, or R⁹;
p is 0, 1 or 2;
R⁴ is H, halo, CN, CF₃, -OCF₃, OH, -NO₂, -(CR¹⁴R¹⁵)ₙC(=Y')OR¹¹, -(CR¹⁴R¹⁵)ₙC(=Y')NR¹¹R¹², -(CR¹⁴R¹⁵)ₙNR¹¹R¹², -(CR¹⁴R¹⁵)ₙOR¹¹, -(CR¹⁴R¹⁵)ₙS(O)ₚR¹¹, -(CR¹⁴R¹⁵)ₙNR¹²C(=Y')R¹¹, -(CR¹⁴R¹⁵)ₙNR¹²C(=Y')OR¹¹, -(CR¹⁴R¹⁵)ₙNR¹²C(=Y')NR¹¹R¹², -(CR¹⁴R¹⁵)ₙNR¹²SO₂R¹¹, -(CR¹⁴R¹⁵)ₙOC(=Y')R¹¹, -(CR¹⁴R¹⁵)ₙOC(=Y')NR¹¹R¹², -(CR¹⁴R¹⁵)ₙS(O)₂NR¹¹R¹², C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, or 5- or 6-membered heteroaryl wherein said alkyl, alkenyl, alkynyl, cycloalkyl, heterocyclyl, aryl and heteroaryl are optionally substituted with one to four R¹³ groups;
each n is independently 0-5;
R⁵ is H, CN, CF₃, OH, -(CR¹⁴R¹⁵)ₙC(=Y')OR¹¹, -(CR¹⁴R¹⁵)ₙC(=Y')NR¹¹R¹², -(CR¹⁴R¹⁵)ₙNR¹²C(=Y')R¹¹, -(CR¹⁴R¹⁵)ₙNR¹¹R¹², -(CR¹⁴R¹⁵)ₙOR¹¹, -(CR¹⁴R¹⁵)ₙS(O)ₚR¹¹, -(CR¹⁴R¹⁵)ₙNR¹²C(=Y')OR¹¹, -(CR¹⁴R¹⁵)ₙNR¹²C(=Y')NR¹¹R¹², -(CR¹⁴R¹⁵)ₙNR¹²SO₂R¹¹, -(CR¹⁴R¹⁵)ₙOC(=Y')R¹¹, -(CR¹⁴R¹⁵)ₙOC(=Y')NR¹¹R¹², -(CR¹⁴R¹⁵)ₙS(O)₂NR¹¹R₁₂, C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, 5- or 6-membered heteroaryl wherein the said alkyl, alkenyl, alkynyl, cycloalkyl, heterocyclyl, aryl and heteroaryl are optionally substituted with one to four R¹³ groups;
R⁶ is H, CN, -CF₃, -OCF₃, halo, -C(=Y')OR¹¹, -C(=Y')NR¹¹R¹², -OR¹¹, -OC(=Y')R¹¹, -NR¹¹R¹², -NR¹²C(=Y')R¹¹, -NR¹²C(=Y')NR¹¹R¹², -NR¹²S(O)_{q}R¹¹, -SR¹¹, -S(O)R¹¹, -S(O)₂R¹¹, -OC(=Y')NR¹¹R¹², -S(O)₂NR¹¹R¹², -S(O)₂(OR¹¹), -SC(=Y')R¹¹, -SC(=Y')OR¹¹, -SC(=Y')NR¹¹R¹², C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, or 5- or 6-membered heteroaryl wherein said alkyl, alkenyl, alkynyl, cycloalkyl, heterocyclyl, aryl and heteroaryl are optionally substituted by one to four R¹³ groups;
R^{7a} is H, halo, -CN, -OH, -NO₂, -O(C₁-C₃ alkyl), or C₁-C₄ alkyl, wherein each said alkyl is optionally substituted with one to three groups selected from halo, N(R²²)₂ or OR²².
R^{7b} is H, -OH, -CN, -O(C₁-C₃ alkyl), or C₁-C₄ alkyl, wherein each said alkyl is optionally substituted with one to three groups selected from halo, N(R²²)₂ or OR²²;
R^{8a} is H, halo, -CN, -NO₂, -N(R²²)₂, -OH, -O(C₁-C₃ alkyl), or C₁-C₃ alkyl, wherein each said alkyl is optionally substituted with one to three halo groups;
R^{8b} is H, -OH, -CN, -O(C₁-C₃ alkyl), or C₁-C₃ alkyl, wherein each said alkyl is optionally substituted with one to three halo groups;
each R⁹ is independently C₁-C₁₂-alkyl, C₂-C₁₂-alkenyl, C₂-C₁₂-alkynyl, C₃-C₁₂-cycloalkyl, heterocyclyl monocycle having 3 to 7 ring members or a bicycle having 6 to 10 ring members, aryl, 5- or 6-membered heteroaryl, wherein each member of R⁹ is independently substituted with one to three R¹⁰ groups;
each R¹⁰ is independently H, CN, -CF₃, -OCF₃, -NO₂, halo, R¹¹, -OR¹¹, -NR¹²C(=Y')R¹¹, -NR¹²C(=NR¹²)R¹¹, -NR¹²S(O)_{q}R¹¹, -SR¹¹, -NR¹¹R¹², oxo, -C(=Y')OR¹¹, -C(=Y')NR¹¹R¹², -S(O)_{q}R¹¹, -NR¹²C(=Y')OR¹¹, -NR¹²C(=Y')NR¹¹R¹², -OC(=Y')R¹¹, -OC(=Y')NR¹¹R¹², or -S(O)₂NR¹¹R¹²;
each q independently is 1 or 2;
R¹¹ and R¹² are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R¹³ groups, wherein two geminal R¹³ groups are optionally taken together with the atom to which they are attached to form a 3-6 membered ring having additional 0-2 heteroatoms selected from O, S, and N, said ring being optionally substituted with one to four R¹⁸ groups;
R¹¹ and R¹² are optionally taken together with the attached N atom to form a 4-7 membered ring having additional 0-2 heteroatoms selected from O, S, and N, said ring being optionally substituted with one to four R¹³ groups;
each R¹³ is independently halo, CN, CF₃, -OCF₃, -NO₂, oxo, -(CR¹⁴R¹⁵)ₙC(=Y')R¹⁶, -(CR¹⁴R¹⁵)ₙC(=Y')OR¹⁶, -(CR¹⁴R¹⁵)ₙC(=Y')NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙSR¹⁶, -(CR¹⁴R¹⁵)ₙNR¹⁶C(Y')R¹⁷, -(CR¹⁴R¹⁵)ₙNR¹⁶C(=Y')OR¹⁷, -(CR¹⁴R¹⁵)ₙNR¹⁷C(=Y')NR¹⁶R¹⁷, -(CR¹⁴R¹⁵)ₙNR¹⁷SO₂R¹⁶, -(CR¹⁴R¹⁵)ₙOC(Y')R¹⁶, -(CR¹⁴R¹⁵)ₙOC(=Y')NR¹⁶R¹⁷, -(CR¹⁴R¹⁵)ₙ(O)R¹⁶, -(CR¹⁴R¹⁵)ₙS(O)₂R¹⁶, -(CR¹⁴R¹⁵)ₙS(O)₂NR¹⁶R¹⁷, or R¹⁶;
R¹⁴ and R¹⁵ are independently selected from H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R¹⁸ groups;
R¹⁶ and R¹⁷ are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R¹⁸ groups;
R¹⁶ and R¹⁷ are optionally taken together with the attached N atom to form a 5-6 membered ring having additional 0-2 heteroatoms selected from O, S, and N, said ring being optionally substituted with one to four R¹⁸ groups;
each R¹⁸ is independently H, C₁-C₁₂-alkyl, C₃-C₁₂-cycloalkyl, heterocyclyl monocycle having 3 to 7 ring members or a bicycle having 6 to 10 ring members, aryl, 5- or 6-membered heteroaryl, halo, CN, CF₃, -OCF₃, -NO₂, oxo, -(CR¹⁹R²⁰)ₙ C(=Y')R²³, -(CR¹⁹R²⁰)ₙC(=Y')OR²³, -(CR¹⁹R²⁰)ₙ C(=Y')NR²³R²⁴, -(CR¹⁹R²⁰)ₙNR²³R²⁴, -(CR¹⁹R²⁰)ₙOR²³, -(CR¹⁹R²⁰)ₙ-SR²³, -(CR¹⁹R²⁰)ₙ NR²⁴C(=Y')R²³, -(CR¹⁹R²⁰)ₙ NR²⁴C(=Y')OR²³, -(CR¹⁹R²⁰)ₙNR²²C(-Y')NR²³R²⁴, -(CR¹⁹R²⁰)ₙNR²⁴SO₂R²³, -(CR¹⁹R²⁰)ₙOC(=Y')R²³, -(CR¹⁹R²⁰)ₙ OC(=Y')NR²³R²⁴, -(CR¹⁹R²⁰)ₙS(O)R²³, -(CR¹⁹R²⁰)ₙS(O)₂R²³, or -(CR¹⁹R²⁰)ₙS(O)₂NR²³R²⁴, wherein said alkyl, cycloalkyl, heterocyclyl, aryl, and heteroaryl are optionally substituted with one to four R²¹ groups;
R¹⁹ and R²⁰ are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R²⁵ groups;
R²³ and R²⁴ are independently H, C₁-C₁₂-alkyl, C₃-C₁₂-cycloalkyl, heterocyclyl monocycle having 3 to 7 ring members or a bicycle having 6 to 10 ring members, aryl or 5- or 6-membered heteroaryl, wherein said alkyl, cycloalkyl, heterocyclyl, aryl and heteroaryl are optionally substituted with one to four R²¹ groups;
R²³ and R²⁴ are optionally taken together with the attached N atom to form a 5-6 membered ring having additional 0-2 heteroatoms selected from O, S, and N, said ring being optionally substituted with one to four R²¹ groups;
each R²¹ is independently H, C₁-C₁₂-alkyl, C₃-C₁₂-cycloalkyl, heterocyclyl monocycle having 3 to 7 ring members or a bicycle having 6 to 10 ring members, aryl, 5- or 6-membered heteroaryl, halo, CN, CF₃, -OCF₃, -NO₂, oxo, -C(=Y')R²⁵, -C(=Y')OR²⁵, -C(=Y')NR²⁵R²⁶, -NR²⁵R²⁶, -OR²⁵, -SR²⁵, -NR²⁶C(=Y')R²⁵, -NR²⁶C(=Y')OR²⁵, -NR²²C(=Y')NR²⁵R²⁶, -NR²⁶SO₂R²⁵, -OC(=Y')R²⁵, -OC(=Y')NR²⁵R²⁶, -S(O)R²⁵, -S(O)₂R²⁵, or -S(O)₂NR²⁵R²⁶, wherein said alkyl, cycloalkyl, heterocyclyl, aryl, and 5- or 6-membered heteroaryl are optionally substituted with one to four R²⁵ groups;
each R²⁵ and R²⁶ is independently H, C₁-C₁₂-alkyl, C₃-C₁₂-cycloalkyl, heterocyclyl monocycle having 3 to 7 ring members or a bicycle having 6 to 10 ring members, aryl, or 5- or 6-membered heteroaryl, wherein said alkyl, cycloalkyl, heterocyclyl, aryl, or heteroaryl is optionally substituted with one to four groups selected from halo, -CN, -OCF₃, -CF₃, -NO₂, -C₁-C₆ alkyl, -OH, oxo, -SH, -O(C₁-C₆ alkyl), -S(C₁-C₆ alkyl), -NH₂, -NH(C₁-C₆ alkyl), -N(C₁-C₆ alkyl)₂, -SO₂(C₁-C₆ alkyl), -CO₂H, -CO₂(C₁-C₆ alkyl), -C(O)NH₂, -C(O)NH(C₁-C₆ alkyl), -C(O)N(C₁-C₆ alkyl)₂, -N(C₁-C₆ alkyl)C(O)(C₁-C₆ alkyl), -NHC(O)(C₁-C₆ alkyl), -NHSO₂(C₁-C₆ alkyl), -N(C₁-C₆ alkyl)SO₂(C₁-C₆ alkyl), -SO₂NH₂, -SO₂NH(C₁-C₆ alkyl), -SO₂N(C₁-C₆ alkyl)₂, -OC(O)NH₂, -OC(O)NH(C₁-C₆ alkyl), -OC(O)N(C₁-C₆ alkyl)₂, -NHC(O)NH(C₁-C₆ alkyl), -NHC(O)N(C₁-C₆ alkyl)₂, -N(C₁-C₆ alkyl)C(O)NH(C₁-C₆ alkyl), -N(C₁-C₆ alkyl)C(O)N(C₁-C₆ alkyl)₂, -NHC(O)NH(C₁-C₆ alkyl), -NHC(O)N(C₁-C₆ alkyl)₂, -NHC(O)O(C₁-C₆ alkyl), and -N(C₁-C₆ alkyl)C(O)O(C₁-C₆ alkyl);
R²⁵ and R²⁶ are optionally taken together with the attached N atom to form a 5-6 membered ring having additional 0-2 heteroatoms selected from O, S, and N, said ring being optionally substituted with one to four groups selected from halo, -CN, -OCF₃, -CF₃, -NO₂, -C₁-C₆ alkyl, -OH, oxo, -SH, -O(C₁-C₆ alkyl), -S(C₁-C₆ alkyl), -NH₂, -NH(C₁-C₆ alkyl), -N(C₁-C₆ alkyl, -SO₂(C₁-C₆ alkyl), -CO₂H, -CO₂(C₁-C₆ alkyl), -C(O)NH₂, -C(O)NH(C₁-C₆ alkyl), -C(O)N(C₁-C₆ alkyl)₂, -N(C₁-C₆ alkyl)C(O)(C₁-C₆ alkyl), -NHC(O)(C₁-C₆ alkyl), -NHSO₂(C₁-C₆ alkyl), -N(C₁-C₆ alkyl)SO₂(C₁-C₆ alkyl), -SO₂NH₂, -SO₂NH(C₁-C₆ alkyl), -SO₂N(C₁-C₆ alkyl)₂, -OC(O)NH₂, -OC(O)NH(C₁-C₆ alkyl), -OC(O)N(C₁-C₆ alkyl)₂, -NHC(O)NH(C₁-C₆ alkyl), -NHC(O)N(C₁-C₆ alkyl)₂, -N(C₁-C₆ alkyl)C(O)NH(C₁-C₆ alkyl), -N(C₁-C₆ alkyl)C(O)N(C₁-C₆ alkyl)₂, -NHC(O)NH(C₁-C₆ alkyl), -NHC(O)N(C₁-C₆ alkyl)₂, -NHC(O)O(C₁-C₆ alkyl), and -N(C₁-C₆ alkyl)C(O)O(C₁-C₆ alkyl);
Y' is independently O, NR²², or S; and
each R²² is independently H or C₁-C₅ alkyl.

2. The compound of claim 1 wherein X is CR².

3. The compound of claim 2 wherein R² is H.

4. The compound of claim 2 or 3 wherein Y is CR⁴.

5. The compound of claim 4 wherein R⁴ is H.

6. The compound of claim 4 or 5 wherein Z is CR^{7a}.

7. The compound of claim 6 wherein R^{7a} is H.

8. The compound of claim 4 or 5 wherein Z is N.

9. The compound of any one of claims 6-8 wherein W is CR^{8a}.

10. The compound of claim 9 wherein R^{8a} is H.

11. The compound of claim 9 or 10 wherein R³ is Br.

12. The compound of claim 9 or 10 wherein R³ is R⁹.

13. The compound of claim 12 wherein R⁹ is C₂-C₃ alkynyl, C₆ aryl, or 5-6 membered monocyclic or 8-10 membered bicyclic heteroaryl having 1 to 2 ring atoms selected from N, O and S; and wherein each member of R⁹ is independently substituted with one to two R¹⁰ groups.

14. The compound of claim 13 wherein R⁹ is propynyl, phenyl, pyrazolyl, pyridyl, pyrimidinyl, thienyl, furanyl, imidazolyl, or benzothienyl, wherein each member of R⁹ is independently substituted with one to two R¹⁰ groups.

15. The compound of claim 14 wherein R⁹ is phenyl substituted with one to two R¹⁰ groups.

16. The compound of claim 14 or 15 wherein R¹⁰ is halo, R¹¹, -OR¹¹, CN, -CF₃, -OCF₃, -NR¹²C(=O)R¹¹, -NR¹²S(O)_{q}R¹¹, -SR¹¹, -NR¹¹R¹², -C(=O)NR¹¹R¹², -S(O)_{q}R¹¹, or -S(O)₂NR¹¹R¹², wherein R¹¹ and R¹² are optionally taken together with the attached N atom to form a 4-7 membered ring having additional 0-2 heteroatoms selected from O, S, and N, said ring being optionally substituted with one to four R¹³ groups.

17. The compound of claim 16 wherein R¹⁰ is R¹¹.

18. The compound of claim 17 wherein R¹¹ is C₁-C₆ alkyl, or 5-6 membered monocyclic or 8-10 membered bicyclic heterocyclyl having 1 to 2 heteroatoms selected from N and O, wherein said alkyl and heterocyclyl are optionally substituted with one to four R¹³ groups, wherein two geminal R¹³ groups are optionally taken together with the atom to which they are attached to form a six-membered ring having 0-2 heteroatom selected from O, S, and N, said ring being optionally substituted with one to four R¹⁸ groups.

19. The compound of claim 18 wherein R¹¹ is C₁-C₆ alkyl, wherein alkyl is optionally substituted with one to two R¹³ groups and wherein each R¹³ is independently halo, CN, CF₃, -OCF₃, oxo, -(CR¹⁴R¹⁵)ₙC(O)OR¹⁶, -(CR¹⁴R¹⁵)ₙC(O)NR¹⁶R¹⁷, -( CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷,or R¹⁶.

20. The compound of claim 18 wherein R¹¹ is 5-6 membered monocyclic or 8-10 membered bicyclic heterocyclyl having 1 to 2 heteroatoms selected from N and O, wherein said alkyl and heterocyclyl are optionally substituted with one to two R¹³ groups and wherein each R¹³ is independently halo, CN, CF₃, -OCF₃, oxo, -(CR¹⁴R¹⁵)ₙC(O)OR¹⁶, -(CR¹⁴R¹⁵)ₙC(O)NR¹⁶R¹⁷, -(CR¹⁴R¹⁷)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷, or R¹⁶.

21. The compound of claim 16 wherein R¹⁰ is -OR¹¹.

22. The compound of claim 21 wherein R¹¹ is H, C₁-C₄ alkyl, or 5-6 membered monocyclic or 8-10 membered bicyclic heterocyclyl having 1 to 2 nitrogen atoms, wherein said alkyl or heterocyclyl is optionally substituted with one to two R¹³ groups, wherein each R¹³ is independently halo, CN, CF₃, -OCF₃, oxo, -(CR¹⁴R¹⁵)ₙC(O)OR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷, or R¹⁶.

23. The compound of any one of claims 11-22 wherein R⁵ is H.

24. The compound of any one of claims 11-22 wherein R⁵ is

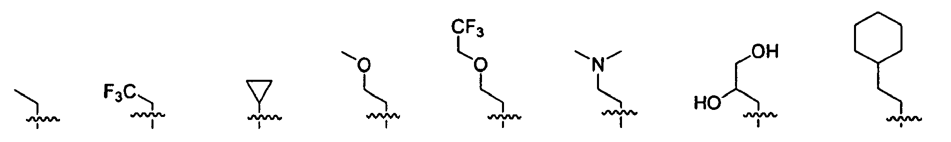

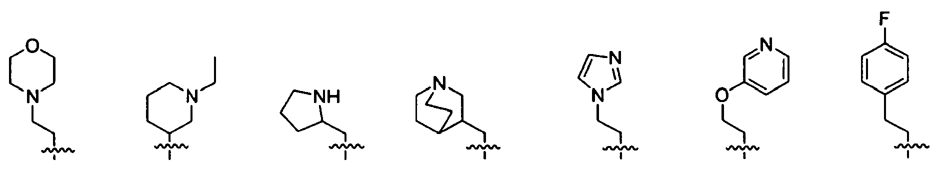

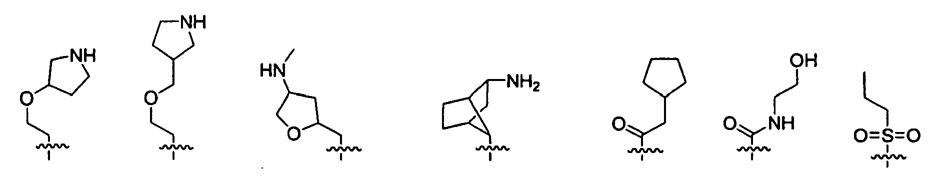

25. The compound of claim 23 wherein R⁶ is CN, halo, -C(O)NR¹¹R¹², -OR¹¹, -NR¹¹R¹², -NR¹²C(O)R¹¹, C₁-C₃ alkyl, C₃-C₆ cycloalkyl, 5-6 membered heterocyclyl having 1 to 2 heteroatoms, C₆ aryl, or 5-6 membered heteroaryl having 1 to 2 heteroatoms; wherein said alkyl is substituted with one to two R¹³ groups except H; and said cycloalkyl, aryl, heterocyclyl or heteroaryl is optionally substituted by one to two R¹³ groups; wherein heteroatoms are selected from N, O and S; wherein each R¹² is H or C₁-C₃ alkyl and each R¹¹ is independently H or C₁-C₃ alkyl optionally substituted by one to two R¹³ groups.

26. The compound of claim 25 wherein R⁶ is CN.

27. The compound of claim 25 wherein R⁶ is pyridyl, or pyrazolyl optionally substituted with methyl.

28. The compounds of claim 1 that are selected from the group consisting of:
3-Bromo-6-pyridin-3-yl-7,9-dihydro-1,5,7,9-tetraaza-fluoren-8-one,
3-Bromo-6-(1-methyl-1H-pyrazol-4-yl)-7,9-dihydro-1,5,7,9-tetraaza-fluoren-8-one,
3-Bromo-6-[1-(4-methoxy-benzyl)-1H-pyrazol-4-yl]-7,9-dihydro-1,5,7,9-tetraaza-fluoren-8-one,
3-Bromo-6-(1-methyl-1H-pyrazol-3-yl)-7,9-dihydro-1,5,7,9-tetraaza-fluoren-8-one,
3-Bromo-6-(2-methyl-2H-pyrazol-3-yl)-7,9-dihydro-1,5,7,9-tetraaza-fluoren-8-one,
3-[4-(4-Methyl-piperazin-1-yl)-phenyl]-6-pyridin-3-yl-7,9-dihydro-1,5,7,9-tetraaza-fluoren-8-one,
3-[4-(4-Methyl-piperazin-1-yl)-phenyl]-6-(1-methyl-1H-pyrazol-4-yl)-7,9-dihydro-1,5,7,9-tetraaza-fluoren-8-one,
6-[1-(4-Methoxy-benzyl)-1H-pyrazol-4-yl]-3-[4-(4-methyl-piperazin-1-yl)-phenyl] -7,9-dihydro-1,5,7,9-tetraaza-fluoren-8-one,
3-[4-(4-Methyl-piperazin-1-yl)-phenyl]-6-(1-methyl-1H-pyrazol-3-yl)-7, 9-dihydro-1,5,7,9-tetraaza-fluoren-8-one,
3-[4-(4-Methyl-piperazin-1-yl)-phenyl]-6-(2-methyl-2H-pyrazol-3-yl)-7,9-dihydro-1,5,7,9-tetraaza-fluoren-8-one,
6-(1-Methyl-1H-pyrazol-4-yl)-3-(4-piperidin-1-ylmethyl-phenyl)-7,9-dihydro-1,5,7,9-tetraaza-fluoren-8-one,
8-Chloro-3-[4-(4-methyl-piperazin-1-yl)-phenyl]-6-pyridin-3-yl-9H-1,5,7,9-tetraaza-fluorene,
8-Chloro-3-[4-(4-methyl-piperazin-1-yl)-phenyl]-6-(1-methyl-1H-pyrazol-4-yl)-9H-1,5,7,9-tetraaza-fluorene,
8-Chloro-3-[4-(4-methyl-piperazin-1-yl)-phenyl]-6-(1H-pyrazol-4-yl)-9H-1,5,7,9-tetraaza-fluorene,
8-Chloro-3-[4-(4-methyl-piperazin-1-yl)-phenyl]-6-(2-methyl-2H-pyrazol-3-yl)-9H-1,5,7,9-tetraaza-fluorene,
8-Chloro-3-[4-(4-methyl-piperazin-1-yl)-phenyl]-6-(2-methyl-2H-pyrazol-3-yl)-9H-1,5,7,9-tetraaza-fluorene,
8-Chloro-6-(1-methyl-1H-pyrazol-4-yl)-3-(4-piperidin-1-ylmethyl-phenyl)-9H-1,5,7,9-tetraaza-fluorene,
3-[4-(4-Methyl-piperazin-1-yl)-phenyl]-6-pyridin-3-yl-9H-1,5,7,9-tetraaza-fluorene
3-[4-(4-Methyl-piperazin-1-yl)-phenyl]-6-(1-methyl-1H-pyrazol-4-yl)-9H -1,5,7,9-tetraaza-fluorene,
3-[4-(4-Methyl-piperazin-1-yl)-phenyl]-6-(1H-pyrazol-4-yl)-9H-1,5,7,9-tetraaza-fluorene,
3-[4-(4-Methyl-piperazin-1-yl)-phenyl]-6-(1-methyl-1H-pyrazol-3-yl)-9H-1,5,7,9-tetraaza-fluorene,
3-[4-(4-Methyl-piperazin-1-yl)-phenyl]-6-(2-methyl-2H-pyrazol-3-yl)-9H-1,5,7,9-tetraaza-fluorene,
6-(1-Methyl-1H-pyrazol-4-yl)-3-(4-piperidin-1-ylmethyl-phenyl)-9H-1,5,7,9-tetraaza-fluorene,
8-Methyl-3-[4-(4-methyl-piperazin-1-yl)-phenyl]-6-(1-methyl-1H-pyrazol-4-yl)-9H-1,5,7,9-tetraaza-fluorene, and
8-[4-(4-Methyl-piperazin-1-yl)-phenyl]-5H-dipyrido[2,3-b;2',3'-d]pyrrole-2-carbonitrile.

29. A pharmaceutical composition comprising a compound of any one of claims 1-28 and a pharmaceutically acceptable carrier.

30. The pharmaceutical composition of claim 29, further comprising a second chemotherapeutic agent.

31. The pharmaceutical composition of claim 30, wherein said second chemotherapeutic agent is a DNA damaging agent.

32. A compound of any one of claims 1 to 28 or the pharmaceutical composition of any one of claims 29 to 30 for use in a method of inhibiting abnormal cell growth or treating a hyperproliferative disorder in a mammal.

33. The compound of any one of claims 1 to 28 or the pharmaceutical composition of any one of claims 29 to 30 for use in a method of treating cancer in a mammal.

34. The compound of claim 33, wherein cancer is selected from breast cancer, colorectal cancer, ovarian cancer, non-small cell lung cancer, malignant brain tumors, sarcomas, melanoma, lymphoma, myelomas and leukemia.

35. The compound of claims 31, wherein said second chemotherapeutic agent is administered to said mammal sequentially or concurrently to said compound(s).

36. Use of a compound of any one of claims 1-28 in the preparation of a medicament for the treatment of cancer.

37. The use of claim 36, wherein the cancer is selected from breast cancer, colorectal cancer, ovarian cancer, non-small cell lung cancer, malignant brain tumors, sarcomas, melanoma, lymphoma, myelomas and leukemia.

## Patentansprüche

1. Verbindung der Formel (I), (I-a), (I-b), (I-c), (I-d), (I-e), (I-f), (I-g) oder (I-h) oder ein Solvat, Hydrat oder Salz davon: 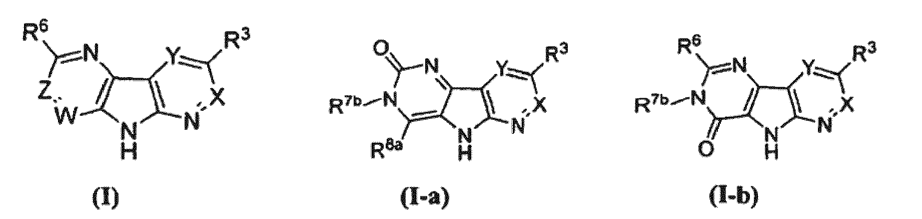 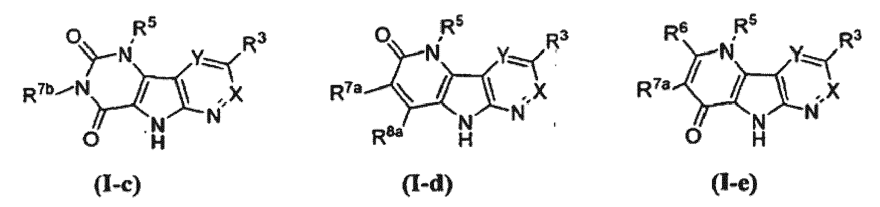 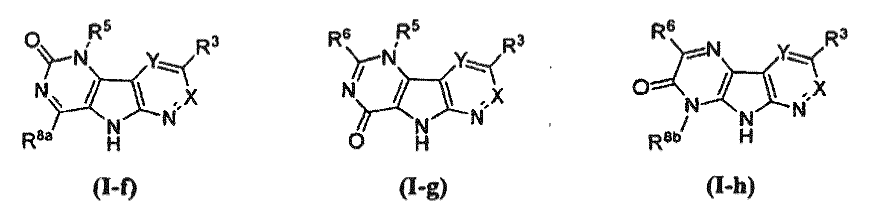 worin
X = CR² oder N ist;
Y = CR⁴ oder N ist;
Z = CR^{7a} oder N ist;
W = CR^{8a} oder N ist, mit der Maßgabe, dass (i) Z und W nicht beide gleichzeitig N sind und (ii) X und Y nicht beide gleichzeitig N sind;
R² = H, Halogen, CN, CF₃, -OCF₃, OH, -NO₂, C₁₋₅-Alkyl, -O(C₁₋₅-Alkyl), -S(C₁₋₅-Alkyl) oder N(R²²)₂ ist,
R³ = H, Halogen, -O-R⁹, -N(R²²)-R⁹, -S(O)ₚ-R⁹ oder R⁹ ist,
p = 0, 1 oder 2 ist,
R⁴ = H, Halogen, CN, CF₃, -OCF₃, OH, -NO₂, -(CR¹⁴R¹⁵)ₙC(=Y')OR¹¹, **-(CR¹⁴R¹⁵)ₙC(=Y')NR¹¹R¹² -(CR¹⁴R¹⁵)ₙNR¹¹R¹²,-(CR¹⁴R¹⁵)ₙOR¹¹, -(CR¹⁴R¹⁵)ₙS(O)ₚR¹¹, -(CR¹⁴R¹⁵)ₙNR¹²C(=Y')R¹¹, -(CR¹⁴R¹⁴)ₙNR¹²C(=Y')OR¹¹, -(CR¹⁴R¹⁵)ₙNR¹²C(=Y')NR¹¹R¹², -(CR¹⁴R¹⁵)ₙNR¹²SO₂R¹¹, -(CR¹⁴R¹⁵)ₙOC(=Y')R¹¹,** -(CR¹⁴R¹⁵)ₙOC(=Y')NR¹¹ R¹², -(CR¹⁴R¹⁵)ₙS(O)₂NR¹¹R¹², C₁₋₁₂-Alkyl, C₂₋₁₂-Alkenyl, -(CR¹⁴R¹⁵)ₙOC(=Y')NR¹¹R¹², -(CR¹⁴R¹⁵)ₙS(O)₂NR¹¹R¹² C₁₋₁₂-Alkyl, C₂₋₁₂-Alkenyl, C₂₋₁₂-Alkinyl, C₃₋₁₂-Cycloalkyl, monozylisches Heterocyclyl mit 3 bis 7 Ringelementen oder ein Bizyklus mit 6 bis 10 Ringelementen, Aryl oder 5- oder 6-gliedriges Heteroaryl ist, worin das Alkyl, das Alkenyl, das Alkinyl, das Cycloalkyl, das Heterocyclyl, das Aryl und das Heteroaryl jeweils gegebenenfalls mit 1 bis 4 R¹³-Gruppen substituiert sind;
die n jeweils unabhängig 0 bis 5 sind;
R⁵ = H, CN, CF₃, OH, -(CR¹⁴R¹⁵)ₙC(=Y')OR¹¹, -(CR¹⁴R¹⁵)ₙC(=Y')NR¹¹R¹², -(CR¹⁴R¹⁵)ₙNR¹²C(=Y')R¹¹, -(CR¹⁴R¹⁵)ₙNR¹¹R¹², -(CR¹⁴R¹⁵)ₙOR¹¹, -(CR¹⁴R¹⁵)ₙS(O)ₚR¹¹, -(CR¹⁴R¹⁵)ₙNR¹²C(=Y')OR¹¹, -(CR¹⁴R¹⁵)ₙNR¹²C(=Y')NR¹¹R¹², -(CR¹⁴R¹⁵)ₙNR**¹²**SO₂R¹¹, -(CR¹⁴R¹⁵)ₙOC(=Y')R¹¹, -(CR¹⁴R¹⁵)ₙOC(=Y')NR¹¹R¹², -(CR¹⁴R¹⁵)ₙS(O)₂NR¹¹R¹², C₁₋₁₂-Alkyl, C₂₋₁₂-Alkenyl, C₂₋₁₂-Alkinyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl ist, worin das Alkyl, das Alkenyl, das Alkinyl, das Cycloalkyl, das Heterocyclyl, das Aryl und das Heteroaryl jeweils gegebenenfalls mit 1 bis 4 R¹³-Gruppen substituiert sind;
R⁶ = H, CN, -CF₃, OCF₃, Halogen, -C(=Y')OR¹¹ -C(=Y')NR¹¹R¹², -OR¹¹, -OC(=Y')R¹¹, -NR¹¹R¹², -NR¹²C(=Y')R¹¹, -NR¹²C(=Y')NR¹¹R¹²,-NR¹²S(O)_{q}R¹¹, -SR¹¹, **-S(O)R¹¹, -S(O)₂R¹¹, -OC(=Y')NR¹¹R¹², -S(O)₂NR¹¹R¹², -S(O)₂(OR¹¹), -**SC(=Y')R¹¹, **-S(O)R¹¹, -S(O)₂R¹¹, -OC(-Y')NR¹¹R¹², -S(O)₂NR¹¹R¹², -S(O)₂(OR¹¹)**, -SC(=Y')R¹¹, -SC(=Y')OR¹¹, -SC(=Y')NR¹¹R¹², C₁₋₁₂-Alkyl, C₂₋₁₂-Alkenyl, C₂₋₁₂-Alkinyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl ist, worin das Alkyl, das Alkenyl, das Alkinyl, das Cycloalkyl, das Heterocyclyl, das Aryl und das Heteroaryl jeweils gegebenenfalls mit 1 bis 4 R¹³-Gruppen substituiert sind;
R^{7a} = H, Halogen, -CN, -OH, -NO₂, -O(C₁₋₃-Alkyl) oder C₁₋₄-Alkyl ist, worin jedes solche Alkyl gegebenenfalls mit 1 bis 3 aus Halogen, N(R²²)₂ und OR²² ausgewählten Gruppen substituiert ist;
R^{7b} = H, -OH, -CN, -O(C₁₋₃-Alkyl) oder C₁₋₄-Alkyl ist, worin jedes solche Alkyl gegebenenfalls mit 1 bis 3 aus Halogen, N(R²²)₂ und OR²² ausgewählten Gruppen substituiert ist;
R^{8a} = H, Halogen, -CN, -NO₂, -N(R²²)₂, -OH, -O(C₁₋₃-Alkyl) oder C₁₋₃-Alkyl ist, worin jedes solche Alkyl gegebenenfalls mit 1 bis 3 Halogengruppen substituiert ist;
R^{8b} = H, -OH, -CN, -O(C₁₋₃-Alkyl) oder C₁₋₃-Alkyl ist, worin jedes solche Alkyl gegebenenfalls mit 1 bis 3 Halogengruppen substituiert ist;
die Reste R⁹ jeweils unabhängig C₁₋₁₂-Alkyl, C₂₋₁₂-Alkenyl, C₂₋₁₂-Alkinyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl sind, worin das Alkyl, das Alkenyl, das Alkinyl, das Cycloalkyl, das Heterocyclyl, das Aryl und das Heteroaryl jeweils gegebenenfalls mit 1 bis 3 R¹⁰-Gruppen substituiert sind;
die Reste R¹⁰ jeweils unabhängig H, CN, -CF₃, -OCF₃, -NO₂, Halogen, R¹¹, -OR¹¹, **-NR¹²C(=Y')R¹¹,-NR¹²C(=NR¹²)R¹¹,-NR¹² S(O)_{q}R¹¹, -SR¹¹, -NR¹¹R¹²**, Oxo, **-C(=Y')OR¹¹, -C(=Y')NR¹¹R¹², -S(O)_{q}R¹¹,-NR¹²C(=Y')OR¹¹**, -NR¹²C(=Y')NR¹¹R¹², -OC(=Y')R¹¹, -OC(=Y')NR¹¹R¹² oder -S(O)₂NR¹¹R¹² sind;
die q jeweils unabhängig 1 oder 2 sind;
R¹¹ und R¹² jeweils unabhängig voneinander H, C₁₋₁₂-Alkyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl sind, worin das Alkyl, das Alkenyl, das Alkinyl, das Cycloalkyl, das Heterocyclyl, das Aryl und das Heteroaryl jeweils gegebenenfalls mit 1 bis 4 R¹³-Gruppen substituiert sind, worin zwei geminale R¹³-Gruppen gegebenenfalls zusammen mit dem Atom, an das sie gebunden sind, einen 3- bis 6-gliedrigen Ring mit 0 bis 2 aus O, S und N ausgewählten zusätzlichen Heteroatomen bilden, wobei der Ring gegebenenfalls mit 1 bis 4 R¹⁸-Gruppen substituiert ist;
die Reste R¹³ jeweils Halogen, CN, CF₃, -OCF₃, -NO₂, Oxo, -(CR¹⁴R¹⁵)ₙ(C)(=Y')R¹⁶, **-(CR¹⁴R¹⁵)ₙC(=Y')OR¹⁶,-(CR¹⁴R¹⁵)ₙC(=Y')NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙSR¹⁶, -(CR¹⁴R¹⁵)ₙNR¹⁶C(=Y')R¹⁷, -(CR¹⁴R¹⁵)ₙNR¹⁶C(=Y')OR¹⁷,-(CR¹⁴R¹⁵)ₙNR¹⁷C(-Y')NR¹⁶R¹⁷, -(CR¹⁴R¹⁵)ₙNR¹⁷SO₂R¹⁶, -(CR¹⁴R¹⁵)ₙOC(=Y')R¹⁶,-(CR¹⁴R¹⁵)ₙOC(=Y')NR¹⁶R¹⁷,** -(CR¹⁴R¹⁵)ₙS(O)R¹⁶, -(CR¹⁴R¹⁵)ₙS(O)₂R¹⁶, -(CR¹⁴R¹⁵)ₙS(O)₂R¹⁶R¹⁷ oder R¹⁶ sind;
R¹⁴ und R¹⁵ jeweils unabhängig voneinander aus H, C₁₋₁₂-Alkyl, C₃₋₁₂-Cycloalkyl, monozyklischem Heterocyclyl mit 3 bis 7 Ringelementen und Bizyklen mit 6 bis 10 Ringelementen, Aryl oder 5- oder 6-gliedrigem Heteroaryl ausgewählt sind, worin das Alkyl, das Cycloalkyl, das Heterocyclyl, das Aryl und das Heteroaryl jeweils gegebenenfalls mit 1 bis 4 R¹⁸-Gruppen substituiert sind;
R¹⁶ und R¹⁷ jeweils unabhängig voneinander H, C₁₋₁₂-Alkyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl sind, worin das Alkyl, das Cycloalkyl, das Heterocyclyl, das Aryl und das Heteroaryl jeweils gegebenenfalls mit 1 bis 4 R¹⁸-Gruppen substituiert sind;
Rⁱ⁶ und R¹⁷ gegebenenfalls zusammen mit dem daran gebundenen N-Atom einen 5- bis 6-gliedrigen Ring mit 0 bis 2 aus O, S und N ausgewählten zusätzlichen Heteroatomen bilden, wobei der Ring gegebenenfalls mit 1 bis 4 R¹⁸-Gruppen substituiert ist;
die Reste R¹⁸ jeweils H, C₁₋₁₂-Alkyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl, Halogen, CN, CF₃, -OCF₃, -NO₂, Oxo, -(CR¹⁹R²⁰), C(=Y')R²³, **-(CR¹⁹R²⁰)ₙC(=Y')OR²³, -(CR¹⁹R²⁰)ₙ C(=Y')NR²³R²⁴, -(CR¹⁹R²⁰)ₙNR²³R²⁴, -(CR¹⁹R²⁰)ₙOR²³, -(CR¹⁹R²⁰)ₙ-SR²³, -(CR¹⁹R²⁰)ₙ NR²⁴C(=Y')R²³, -(CR¹⁹R²⁰)ₙ NR²⁴C(=Y")OR²³, -(CR¹⁹R²⁰)ₙNR²²C(=Y')NR²³R²⁴, -(CR¹⁹R²⁰)ₙRNR²⁴SO₂R²³, -(CR¹⁹R²⁰)ₙOC(=Y')R²³, -(CR¹⁹R²⁰)ₙ OC(=Y')NR²³R²⁴, -(CR¹⁹R²⁰)ₙS(O)R²³,** -(CR¹⁹R²⁰)ₙS(O)₂R²³ oder -(CR¹⁹R²⁰)ₙS(O)NR²³R²⁴ sind, worin das Alkyl, das Cycloalkyl, das Heterocyclyl, das Aryl und das Heteroaryl jeweils gegebenenfalls mit 1 bis 4 R²¹-Gruppen substituiert sind;
R¹⁹ und R²⁰ jeweils unabhängig voneinander H, C₁₋₁₂-Alkyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl sind, worin das Alkyl, das Cycloalkyl, das Heterocyclyl, das Aryl und das Heteroaryl jeweils gegebenenfalls mit 1 bis 4 R²⁵-Gruppen substituiert sind;
R²³ und R²⁴ jeweils unabhängig voneinander H, C₁₋₁₂-Alkyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl sind, worin das Alkyl, das Cycloalkyl, das Heterocyclyl, das Aryl und das Heteroaryl jeweils gegebenenfalls mit 1 bis 4 R²¹ -Gruppen substituiert sind;
R²³ und R²⁴ gegebenenfalls zusammen mit dem daran gebundenen N-Atom einen 5- bis 6-gliedrigen Ring mit 0 bis 2 aus O, S und N ausgewählten zusätzlichen Heteroatomen bilden, wobei der Ring gegebenenfalls mit 1 bis 4 R²¹-Gruppen substituiert ist;
die Reste R²¹ H, C₁₋₁₂-Alkyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl, Halogen, CN, CF₃, -OCF₃, -NO₂, Oxo, -C(=Y')R²⁵, -C(=Y')OR²⁵, **-C(=Y')NR²⁵R²⁶, -NR²⁵R²⁶, -OR²⁵, -SR²⁵, -NR²⁶C(=Y')R²⁵, ,-NR²⁶C(=Y')OR²⁵ , -NR²²C(=Y')NR²⁵R²⁶, -NR²⁶SO₂R²⁵, -OC(=Y')R^{25,} -OC(=Y')NR²⁵R²⁶, -S(O)R²⁵,** S(O)₂R²⁵ oder S(O)₂NR²⁵R²⁶ sind, worin das Alkyl, das Cycloalkyl, das Heterocyclyl, das Aryl und das 5- bis 6-gliedrige Heteroaryl jeweils gegebenenfalls mit 1 bis 4 R²⁵-Gruppen substituiert sind;
die Reste R²⁵ und die Reste R²⁶ jeweils unabhängig voneinander H, C₁₋₁₂-Alkyl, C₃₋₁₂-Cycloalkyl, monozyklisches Heterocyclyl mit 3 bis 7 Ringelementen oder ein Bizyklus mit 6 bis 10 Ringelementen, Aryl oder 5- oder 6-gliedriges Heteroaryl sind, worin das Alkyl, das Cycloalkyl, das Heterocyclyl, das Aryl und das Heteroaryl jeweils gegebenenfalls mit 1 bis 4 aus Halogen, -CN, -OCF₃, -CF₃, -NO₂, C₁₋₆-Alkyl, -OH, Oxo, -SH, -O(C₁₋₆-Alkyl), -S(C₁₋₆-Alkyl), -NH₂, -NH(C₁₋₆-Alkyl), -N(C₁₋₆-Alkyl)₂, -SO₂(C₁₋₆-Alkyl), -CO₂H, -CO₂(C₁₋₆-Alkyl), -C(O)NH₂, -C(O)NH(C₁₋₆-Alkyl), -C(O)N(C₁₋₆-Alkyl)₂, -N(C₁₋₆-Alkyl)C(O)(C₁₋₆-Alkyl), -NHC(O)(C₁₋₆-Alkyl), -NHSO₂(C_{1- 6}-Alkyl), -N(C₁₋₆-Alkyl)SO₂(C₁₋₆-Alkyl), -SO₂NH₂, -SO₂NH(C₁₋₆-Alkyl), -SO₂N(C₁₋₆-Alkyl)₂, -OC(O)NH₂, -OC(O)NH(C₁₋₆-Alkyl), -OC(O)N(C₁₋₆-Alkyl)₂, -NHC(O)NH(C₁₋₆-Alkyl), -NHC(O)N(C₁₋₆-Alkyl)₂, -N(C₁₋₆-Alkyl)C(O)NH(C₁₋₆-Alkyl), -N(C₁₋₆-Alkyl)C(O)N-(C₁₋₆-Alkyl)₂, -NHC(O)NH(C₁₋₆-Alkyl), -NHC(O)N(C₁₋6-Alkyl)₂, -NHC(O)(C₁₋₆-Alkyl) und -N(C₁₋₆-Alkyl)C(O)(C₁₋₆-Alkyl) ausgewählten Gruppen substituiert sind;
R²⁵ und R²⁶ gegebenenfalls zusammen mit dem daran gebundenen N-Atom einen 5- bis 6-gliedrigen Ring mit 0 bis 2 aus O, S und N ausgewählten zusätzlichen Heteroatomen bilden, wobei der Ring gegebenenfalls mit 1 bis 4 aus Halogen, -CN, -OCF₃, -CF₃, -NO₂, C₁₋₆-Alkyl, -OH, Oxo, -SH, -O(C₁₋₆-Alkyl), -S(C₁₋₆-Alkyl), -NH₂, -NH(C₁₋₆-Alkyl), -N(C₁₋₆-Alkyl)₂, -SO₂(C₁₋₆-Alkyl), -CO₂H, -CO₂(C₁₋₆-Alkyl), -C(O)NH₂, -C(O)NH(C₁₋₆-Alkyl), -C(O)N(C₁₋₆-Alkyl)₂, -N(C₁₋₆-Alkyl)C(O)(C₁₋₆-Alkyl), -NHC(O)(C₁₋₆-Alkyl), -NHSO₂(C₁₋₆-Alkyl), -N(C₁₋₆-Alkyl)SO₂(C₁₋₆-Alkyl), -SO₂NH₂, -SO₂NH(C₁₋₆-Alkyl), -SO₂N(C₁₋₆-Alkyl)₂, -OC(O)NH₂, -OC(O)NH(C₁₋₆-Alkyl), -OC(O)N(C₁₋₆-Alkyl)₂, -NHC(O)NH(C₁₋₆-Alkyl), -NHC(O)N(C₁₋₆-Alkyl)₂, -N(C₁₋₆-Alkyl)-C(O)NH(C₁₋₆-Alkyl), -N(C₁₋₆-Alkyl)C(O)N(C₁₋₆-Alkyl)₂, -NHC(O)NH(C₁₋₆-Alkyl), -NHC(O)N(C₁₋₆-Alkyl)₂, -NHC(O) (C₁₋₆-Alkyl) und -N(C₁₋₆-Alkyl)C(O)(C₁-₆-Alkyl) ausgewählten Gruppen substituiert ist;
Y' jeweils unabhängig O, NR²² oder S ist; und
die Reste R²² jeweils H oder C₁₋₅-Alkyl sind.

2. Verbindung nach Anspruch 1, worin X = CR² ist.

3. Verbindung nach Anspruch 2, worin R² = H ist.

4. Verbindung nach Anspruch 2 oder 3, worin Y = CR⁴ ist.

5. Verbindung nach Anspruch 4, worin R⁴ = H ist.

6. Verbindung nach Anspruch 4 oder 5, worin Z = CR^{7a} ist.

7. Verbindung nach Anspruch 6, worin R^{7a} = H ist.

8. Verbindung nach Anspruch 4 oder 5, worin Z = N ist.

9. Verbindung nach einem der Ansprüche 6 bis 8, worin = CR^{8a} ist.

10. Verbindung nach Anspruch 9, worin R^{8a} = H ist.

11. Verbindung nach Anspruch 9 oder 10, worin R³ = Br ist.

12. Verbindung nach Anspruch 9 oder 10, worin R³ = R⁹ ist.

13. Verbindung nach Anspruch 12, worin R⁹ C₂₋₃-Alkinyl, C₆-Aryl oder 5- bis 6- gliedriges monozyklisches oder 8- bis 10-gliedriges bizyklisches Heteroaryl mit 1 bis 2 aus N, O und S ausgewählten Ringatomen ist und worin jedes Element von R⁹ jeweils unabhängig mit einer oder zwei R¹⁰-Gruppen substituiert ist.

14. Verbindung nach Anspruch 13, worin R⁹ Propinyl, Phenyl, Pyrazolyl, Pyridyl, Pyrimidinyl, Thienyl, Furanyl, Imidazolyl oder Benzthienyl ist, worin jedes Element von R⁹ jeweils mit einer oder zwei R¹⁰-Gruppen substituiert ist.

15. Verbindung nach Anspruch 14, worin R⁹ mit einer oder zwei R¹⁰-Gruppen substituiertes Phenyl ist.

16. Verbindung nach Anspruch 14 oder 15, worin R¹⁰ Halogen, R¹¹, -OR¹¹, CN, -CF₃, -OCF₃, -NR¹²C(=O)R¹¹, -NR¹²S(O)_{q}R¹¹, -SR¹¹, -NR¹¹R¹², -C(=O)NR¹¹R¹², -S(O)_{q}R¹¹ oder S(O)₂NR¹¹R¹² ist, worin R¹¹ und R¹² gegebenenfalls zusammen mit dem daran gebundenen N-Atom einen 5- bis 6-gliedrigen Ring mit 0 bis 2 aus O, S und N ausgewählten zusätzlichen Heteroatomen bilden, wobei der Ring gegebenenfalls mit 1 bis 4 R¹³ -Gruppen substituiert ist.

17. Verbindung nach Anspruch 16, worin R¹⁰ = R¹¹ ist.

18. Verbindung nach Anspruch 17, worin R¹¹ C₁₋₆-Alkyl oder 5- bis 6-gliedriges monozyklisches oder 8- bis 10-gliedriges bizyklisches Heteroaryl mit 1 bis 2 aus N und O ausgewählten Ringatomen ist, worin jedes Alkyl und Heterocyclyl jeweils gegebenenfalls mit 1 bis 4 R¹³ -Gruppen substituiert ist. worin zwei geminale R¹³-Gruppen gegebenenfalls zusammen mit dem Atom, an das sie gebunden sind, einen 6-gliedrigen Ring mit 0 bis 2 aus O, S und N ausgewählten zusätzlichen Heteroatomen bilden, wobei der Ring gegebenenfalls mit 1 bis 4 R¹⁸-Gruppen substituiert ist.

19. Verbindung nach Anspruch 18, worin R¹¹ C₁₋₆-Alkyl ist, worin Alkyl gegebenenfalls mit einer oder zwei R¹³-Gruppen substituiert ist und worin jeder Rest R¹³ jeweils unabhängig Halogen, CN, CF₃, -OCF₃, Oxo, -(CR¹⁴R¹⁵)ₙOR¹⁶, -(CR¹⁴R¹⁵)ₙC(O)N-R¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷ oder R¹⁶ ist.

20. Verbindung nach Anspruch 18, worin R¹¹ 5- bis 6-gliedriges monozyklisches oder 8- bis 10-gliedriges bizyklisches Heteroaryl mit 1 bis 2 aus N und O ausgewählten Ringatomen ist, worin jedes Alkyl und Heterocyclyl jeweils gegebenenfalls mit einer oder zwei R¹³-Gruppen substituiert ist und worin jeder Rest R¹³ jeweils unabhängig Halogen, CN, CF₃, -OCF₃, Oxo, -(CR¹⁴R¹⁵)ₙOR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂N-R¹⁶R¹⁷ oder R¹⁶ ist.

21. Verbindung nach Anspruch 16, worin R¹⁰ = -OR¹¹ ist.

22. Verbindung nach Anspruch 22, worin R¹¹ = H, C₁₋₄-Alkyl oder 5- bis 6-gliedriges monozyklisches oder 8- bis 10-gliedriges bizyklisches Heteroaryl mit 1 bis 2 Stickstoffatomen ist, worin das Alkyl oder das Heterocyclyl gegebenenfalls mit einer oder zwei R¹³-Gruppen substituiert ist, worin jeder Rest R¹³ jeweils unabhängig Halogen, CN, CF₃, -OCF₃, Oxo, -(CR¹⁴R¹⁵)ₙOR¹⁶, -(CR¹⁴R¹⁵)ₙC(O)NR¹⁶R¹⁷, -(CR ¹⁴R¹⁵)ₙN-R¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷ oder R¹⁶ ist.

23. Verbindung nach einem der Ansprüche 11 bis 22, worin R⁵ = H ist.

24. Verbindung nach einem der Ansprüche 11 bis 22, worin R⁵ Folgendes ist:

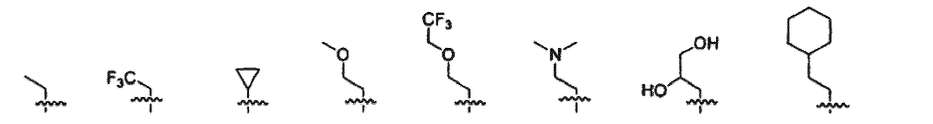

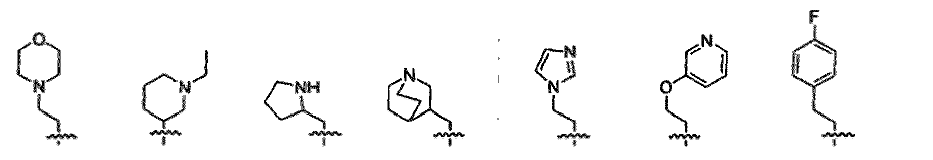

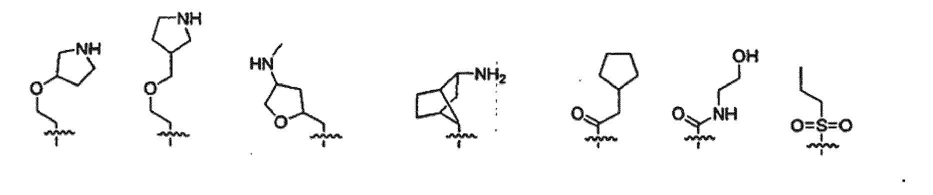

25. Verbindung nach Anspruch 23, worin R⁶ = CN, Halogen, -C(O)NR¹¹R¹², -OR¹¹, -NR¹¹R¹², -NR¹²C(O)R¹¹, C₁₋₃-Alkyl, C₃₋₆-Cycloalkyl, 5- bis 6-gliedriges monozyklisches oder 8- bis 10-gliedriges bizyklisches Heteroaryl mit 1 bis 2 Heteroatomen ist, worin das Alkyl mit einer oder zwei R¹³-Gruppen mit Ausnahme von H substituiert ist und das Cycloalkyl, Aryl, Heterocyclyl oder Heteroaryl gegebenenfalls mit einer oder zwei R¹³-Gruppen substituiert ist, worin Heteroatome aus N, O und S ausgewählt sind, worin jeder Rest R¹² = H oder C₁₋₃-Alkyl ist und jeder Rest R¹¹ jeweils unabhängig H oder C₁₋₃-Alkyl, gegebenenfalls substituiert mit einer oder zwei R¹³-Gruppen, ist.

26. Verbindung nach Anspruch 25, worin R⁶ = CN ist.

27. Verbindung nach Anspruch 25, worin R⁶ Pyridyl oder Pyrazolyl, gegebenenfalls substituiert mit Methyl, ist.

28. Verbindungen nach Anspruch 1, die aus der aus
3-Brom-6-pyridin-3-yl-7,9-dihydro-1,5,7,9-tetraazafluoren-8-on,
3-Brom-6-(1-methyl-1H-pyrazol-4-yl)-7,9-dihydro-7,9-dihydro-1,5,7,9-tetraaza-fluoren-8-on,
3-Brom-6-[1-(4-methoxybenzyl)-1H-pyrazol-4-yl]-7,9-dihydro-1,5,7,9-tetraaza-fluoren-8-on,
3-Brom-6-(1-methyl-1H-pyrazol-3-yl)-7,9-dihydro-1,5,7,9-tetraazafluoren-8-on, 3-Brom-6-(2-methyl-2H-pyrazol-3-yl)-7,9-dihydro-1,5,7,9-tetraazafluoren-8-on, 3-[4-(4-Methylpiperazin-1-yl)phenyl]-6-pyridin-3-yl-7,9-dihydro-1,5,7,9-tetra-azafluoren-8-on,
3-[4-(4-Methylpiperazin-1-yl)phenyl]-6-(1-methyl-1H-pyrazol-4-yl)-7,9-dihydro-1,5,7,9-tetraazafluoren-8-on,
6-[1-(4-Methoxybenzyl)-1H-pyrazol-4-yl]-3-(4-(4-methylpiperazin-1-yl)phenyl]-7,9-dihydro-1,5,7,9-tetraazafluoren-8-on,
3-[4-(4-Methylpiperazin-1-yl)phenyl]-6-(1-methyl-1H-pyrazol-3-yl)-7,9-dihydro-1,5,7,9-tetraazafluaren-8-on,
3-[4-(4-Methylpiperazin-1-yl)phenyl]-6-(2-methyl-2H-pyrazol-3-yl)-7,9-dihydro-1,5,7,9-tetraazafluaren-8-on,
6-(1-Methyl-1H-pyrazol-4-yl)-3-(4-piperidin-1-ylmethylphenyl)-7,9-dihydro-1,5,7,9-tetraazafluoren-8-on,
8-Chlor-3-[4-(4-methylpiperazin-1-yl)phenyl]-6-pyridin-3-yl-9H-1,5,7,9-tetraaza-fluoren,
8-Chlor-3-[4-(4-methylpiperazin-1-yl)phenyl]-6-(1-methy)-1H-pyrazol-4-yl)-9H-1,5,7,9-tetraazafluoren,
8-Chlor-3-[4-(4-methylpiperazin-1-yl)phenyl]-6-(1H-pyrazol-4-yl)-9H-1,5,7,9-tetraazafluoren,
8-Chlor-3-[4-(4-methylpiperazin-1-yl)phenyl]-6-(2-methyl-2H-pyrazol-3-yl)-9H-1,5,7,9-tetraazafluoren,
8-Chlor-3-[4-(4-methylpiperazin-1-yl)phenyl]-6-(2-methyl-2H-pyrazol-3-yl)-9H-1,5,7,9-tetraazafluaren,
8-Chlor-6-(1-methyl-1H-pyrazol-4-yl)-3-(4-piperidin-1-ylmethylphenyl)-9H-1,5,7,9-tetraazafluoren,
3-[4-(4-Methylpiperazin-1-yl)phenyl]-6-piperidin-3-yl-9H-1,5,7,9-tetraaza-fluoren,
3-[4-(4-Methylpiperazin-1-yl)phenyl]-6-(1-methyl-1H-pyrazol-4-yl)-9H-1,5,7,9-tetraazafluoren,
3-[4-(4-Methylpiperazin-1-yl)phenyl]-6-(1H-pyrazol-4-yl)-9H-1,5,7,9-tetraaza-fluoren,
3-[4-(4-Methylpiperazin-1-yl)phenyl]-6-(1-methyl-1H-pyrazol-3-yl)-9H-1,5,7,9-tetraazafluoren,
3-[4-(4-Methylpiperazin-1-yl)phenyl]-6-(2-methyl-2H-pyrazol-3-yl)-9H-1,5,7,9-tetraazafluoren,
6-(1-Methyl-1H-pyrazol-4-yl)-3-(4-piperidin-1-ylmethylphenyl)-9H-1,5,7,9-tetra-azafluoren,
8-Methyl-3-[4-(4-methylpiperazin-1-yl)phenyl]-6-(1-methyl-1H-pyrazol-4-yl)-9H-1,5,7,9-tetraazafluoren und
8-[4-(4-methylpiperazin-1-yl)phenyl]-5H-dipyrido[2,3-b;2',3'-d]pyrrol-2-carbonitril
bestehenden Gruppe ausgewählt sind.

29. Pharmazeutische Zusammensetzung, die eine Verbindung nach einem der Ansprüche 1 bis 28 und einen pharmazeutisch annehmbaren Träger umfasst.

30. Pharmazeutische Zusammensetzung nach Anspruch 29, die weiters ein zweites chemotherapeutisches Mittel umfasst.

31. Pharmazeutische Zusammensetzung nach Anspruch 30, worin das zweite chemotherapeutische Mittel ein DNA zerstörendes Mittel ist.

32. Verbindung nach einem der Ansprüche 1 bis 28 oder pharmazeutische Zusammensetzung nach einem der Ansprüche 29 und 30 zur Verwendung in einem Verfahren zur Hemmung von anomaler Zellenvermehrung oder zur Behandlung einer hyperproliferativen Erkrankung bei einem Säugetier.

33. Verbindung nach einem der Ansprüche 1 bis 28 oder pharmazeutische Zusammensetzung nach einem der Ansprüche 29 und 30 zur Verwendung in einem Verfahren zur Behandlung von Krebs bei einem Säugetier.

34. Verbindung nach Anspruch 33, worin der Krebs aus Brustkrebs, kolorektalem Krebs, Eierstockkrebs, nicht-kleinzelligem Lungenkrebs, malignen Hirntumoren, Sarkomen, Melanom, Lymphom, Myelomen und Leukämie ausgewählt ist.

35. Verbindung nach Anspruch 31, worin das zweite chemotherapeutische Mittel dem Säugetier mit der Verbindung (den Verbindungen) gleichzeitig oder sequenziell verabreicht wird.

36. Verwendung einer Verbindung nach einem der Ansprüche 1 bis 28 bei der Herstellung eines Medikaments zur Behandlung von Krebs.

37. Verwendung nach Anspruch 36, worin der Krebs aus Brustkrebs, kolorektalem Krebs, Eierstockkrebs, nicht-kleinzelligem Lungenkrebs, malignen Hirntumoren, Sarkomen, Melanom, Lymphom, Myelomen und Leukämie ausgewählt ist.

## Revendications

1. Composé de formule (I), (I-a), (I-b), (I-c), (I-d), (I-e), (I-f), (I-g), ou (I-h), ou un de ses produits de solvatation, hydrates ou sels : 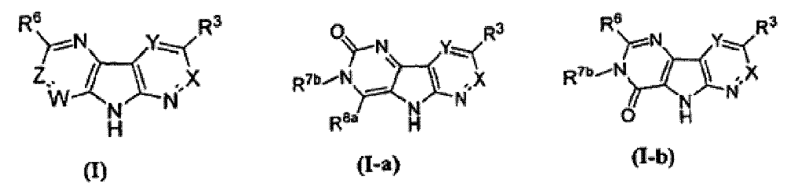 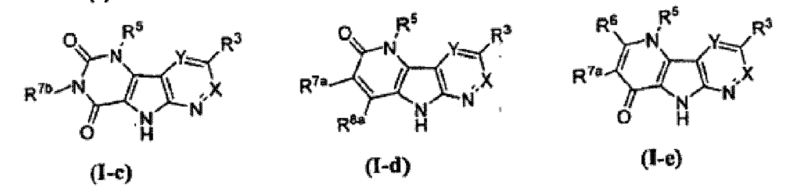 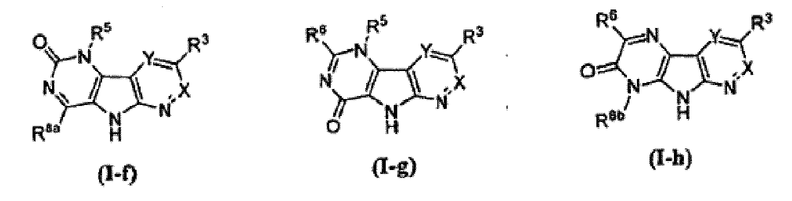 X représente un groupe CR² ou N ;
Y représente un groupe CR⁴ ou N ;
Z représente un groupe CR^{7a} ou N ;
W représente un groupe CR^{8a} ou N ; sous réserve que (i) Z et W ne représentent pas l'un et l'autre N en même temps et (ii) X et Y ne représentent pas l'un et l'autre N en même temps ;
R² représente H, un groupe halogéno, CN, CF₃, -OCF₃, OH, -NO₂, alkyle en C₁ à C₅, -O(alkyle en C₁ à C₅), - S(alkyle en C₁ à C₅) ou N(R²²)₂;
R³ représente H, un groupe halogéno, -O-R⁹, -N(R²²)-R⁹, -S(O)ₚ-R⁹, ou R⁹ ;
p est égal à 0, 1 ou 2 ;
R⁴ représente H, un groupe halogéno, CN, CF₃, -OCF₃, OH, -NO₂, -(CR¹⁴R¹⁵)ₙC(=Y')OR¹¹, -(CR¹⁴R¹⁵)ₙC(=Y')NR¹¹R¹², -(CR¹⁴R¹⁵)ₙNR¹¹R¹², -(CR¹⁴R¹⁵)ₙOR¹¹, -(CR¹⁴R¹⁵)ₙS(O)ₚR¹¹, -(CR¹⁴R¹⁵) ₙNR¹²C(=Y')R¹¹, -(CR¹⁴R¹⁵)ₙNR¹²C(=Y')OR¹¹, -(CR¹⁴R¹⁵)ₙNR¹²/C(=Y')NR¹¹R¹², (CR¹⁴R¹⁵)ₙNR¹²SO2R¹¹, -(CR¹⁴R¹⁵)ₙOC(=Y')R¹¹, -(CR¹⁴R¹⁵ )ₙOC(=Y') NR¹¹R¹², -(CR¹⁴R¹⁵)ₙS(O)₂NR¹¹R¹² alkyle en C₁ à C₁₂, alcényle en C₂ à C₁₂, alcynyle en C₂ à C₁₂, cycloalkyle en C₃ à C₁₂, hétérocyclyle monocyclique ayant 3 à 7 membres dans le noyau ou bicyclique ayant 6 à 10 membres dans le noyau, aryle, ou hétéroaryle penta ou hexagonal, où lesdits groupes alkyle, alcényle, alcynyle, cycloalkyle, hétérocyclyle, aryle et hétéroaryle sont facultativement substitués avec un à quatre groupes R¹³ ;
chaque indice n a indépendamment une valeur de 0 à 5 ; R⁵ représente H, un groupe CN, CF₃, -OCF₃, OH, -(CR¹⁴R¹⁵)ₙC(=Y')OR¹¹, -(CR¹⁴R¹⁵)ₙC(=Y')NR¹¹R¹², (CR¹⁴R¹⁵)ₙNR¹²C(=Y')R¹¹, R¹¹, -(CR¹⁴R¹⁵)ₙNR¹¹R¹², -(CR¹⁴R¹⁵)ₙOR¹¹, -(CR¹⁴R¹⁵)ₙS(O)ₚR¹¹, -(CR¹⁴R¹⁵)ₙNR¹²C(=Y')OR¹¹, (CR¹⁴R¹⁵)ₙNR¹²C(=Y')NR¹¹R¹², - (-CR¹⁴R¹⁵)NR¹²SO₂R¹¹,SO₂R¹¹, - (CR¹⁴R¹⁵)ₙOC(=Y')R¹¹, -(CR¹⁴R¹⁵)ₙOC(=Y')NR¹¹R¹² , - (CR¹⁴R¹⁵)ₙS(O)₂NR¹¹R¹², alkyle en C₁ à C₁₂, alcényle en C₂ à C₁₂, alcynyle en C₂ à C₁₂, cycloalkyle en C₃ à C₁₂, hétérocyclyle monocyclique ayant 3 à 7 membres dans le noyau ou bicyclique ayant 6 à 10 membres dans le noyau, aryle, hétéroaryle penta ou hexagonal, où lesdits groupes alkyle, alcényle, alcynyle, cycloalkyle, hétérocyclyle, aryle et hétéroaryle sont facultativement substitués avec un à quatre groupes R¹³ ;
R⁶ représente H, un groupe CN, -CF₃, -OCF₃, halogéno, -C (=Y')OR¹¹, -C(=Y)NR¹¹R¹² -OR¹¹, -OC(=Y')R¹¹, -NR¹¹R¹², -NR¹²C(=Y')R¹¹, -NR¹²C(=Y')NR¹¹R¹², -NR¹²S(O)_{q}R¹¹, -SR¹¹, -S(O)R¹¹, -S(O)₂R¹¹, -OC(=Y')NR¹¹R¹², -S(O)₂NR¹¹R¹², -S(O)₂(O) R¹¹, -SC(=Y')R¹¹, -SC(=Y')OR¹¹, -SC(=Y')NR¹¹R¹², alkyle en C₁ à C₁₂, alcényle en C₂ à C₁₂, alcynyle en C₂ à C₁₂, cycloalkyle en C₃ à C₁₂, hétérocyclyle monocyclique ayant 3 à 7 membres dans le noyau, ou bicyclique ayant 6 à 10 membres dans le noyau, aryle, hétéroaryle penta- ou hexagonal, où lesdits groupes alkyle, alcényle, alcynyle, cycloalkyle, hétérocyclyle, aryle et hétéroaryle sont facultativement substitués avec un à quatre groupes R¹³ ;
R^{7a} représente H, un groupe halogéno, -CN, -OH, -NO₂, -0(alkyle en C₁ à C₃), ou alkyle en C₁ à C₄, où chacun desdits groupes alkyle est facultativement substitué avec un à trois groupes choisis entre des groupes halogéno, N(R²²)₂ et OR²² ;
R^{7b} représente H, un groupe -OH, -CN, -O(alkyle en C₁ à C₃) , ou alkyle en C₁ à C₄, où chacun desdits groupes alkyle est facultativement substitué avec un à trois groupes choisis entre des groupes halogéno, N(R¹²)₂ ou OR²² ;
R^{8a} représente H, un groupe halogéno, -CN, -NO₂, -N(R²²)₂, -OH, -0 (alkyle en C₁ à C₃) ou alkyle en C₁ à C₃, où chacun desdits groups alkyle est facultativement substitué avec un à trois groupes halogéno ;
R^{8b} représente H, un groupe -OH, -CN, -O(alkyle en C₁ à C₃) ou alkyle en C₁ à C₃, où chacun desdits groupes alkyle est facultativement substitué avec un à trois groupes halogéno ;
chaque groupe R⁹ représente indépendamment un groupe alkyle en C₁ à C₁₂, alcényle en C₂ à C₁₂, alcynyle en C₂ à C₁₂, cycloalkyle en C₃ à C₁₂, hétérocyclyle monocyclique ayant 3 à 7 membres dans le noyau ou bicyclique ayant 6 à 10 membres dans le noyau, aryle, hétéroaryle penta- ou hexagonal, où chaque membre de R⁹ est substitué indépendamment avec un à trois groupes R¹⁰ ;
chaque groupe R¹⁰ représente indépendamment H, un groupe CN, -CF₃, -OCF₃, -NO₂, halogéno, R¹¹, -OR¹¹, -NR¹² C (=Y')R¹¹, -NR¹²C(=NR¹²)R¹¹, -NR¹²S(O)_{q}R¹¹, -SR¹¹, -NR¹¹R¹², oxo, -C(=Y')OR¹¹, -C(=Y')NR¹¹R¹², -S(O)_{q}R¹¹, -NR¹²C(=Y')OR¹¹, -NR¹²C(=Y')NR¹¹R¹², -OC(=Y')R¹¹, -OC(=Y')NR¹¹R¹² ou -S(O)₂NR¹¹R¹² ;
chaque indice q indépendamment égal à 1 ou 2 ;
R¹¹ et R¹² représentent indépendamment H, un groupe alkyle en C₁ à C₁₂, cycloalkyle en C₃ à C₁₂, hétérocyclyle monocyclique ayant 3 à 7 membres dans le noyau ou bicyclique ayant 6 à 10 membres dans le noyau, aryle, ou hétéroaryle penta- ou hexagonal, où lesdits groupes alkyle, cycloalkyle, hétérocyclyle, aryle et hétéroaryle sont facultativement substitués avec un à quatre groupes R¹³, où deux groupes R¹³ géminés sont facultativement pris conjointement avec l'atome auquel ils sont fixés pour former un noyau tri- à hexagonal comprenant 0 à 2 hétéroatomes supplémentaires choisis entre 0, S et N, ledit noyau étant facultativement substitué avec un à quatre groupes R¹⁸ ;
R¹¹ et R¹² sont facultativement pris conjointement avec l'atome de N fixé pour former un noyau tétra- à heptagonal comprenant 0 à 2 hétéroatomes supplémentaires choisis entre 0, S et N, ledit noyau étant facultativement substitué avec un à quatre groupe R¹³ ;
chaque groupe R¹³ représente indépendamment un groupe halogéno, CN, CF₃, -OCF₃, -NO₂, oxo, -(CR¹⁴R¹⁵)ₙC(=Y)R¹⁶; -(CR¹⁴R¹⁵)ₙC(=Y')OR¹⁶, -(CR¹⁴R¹⁵)ₙC(=Y')NR¹⁶R¹⁷, (CR¹⁴R¹⁵)ₙNR¹⁶R¹⁷, -(CR¹⁴R¹⁵)ₙOR¹⁶, (CR¹⁴R¹⁵)ₙSR¹⁶, -(CR¹⁴R¹⁵)ₙNR¹⁶C(=Y')R¹⁷, - (CR¹⁴R¹⁵) ₙNR¹⁶C(=Y') OR¹⁷, - (CR¹⁴R⁵⁵)ₙNR¹⁷C(=Y')NR¹⁶R¹⁷, - (CR¹⁴R¹⁵) ₙNR¹⁷SO₂R¹⁶, -(CR¹⁴R¹⁵)ₙ¹¹OC(=Y')R¹⁶, - (CR¹⁴R¹⁵)ₙ¹¹OC(=Y')NR¹⁶R¹⁷, - (CR¹⁴R¹⁵)ₙS(O)R¹⁶, -(CR¹⁴R¹⁵)ₙS(O)₂R¹⁶, -(CR¹⁴R¹⁵)ₙS(O)₂NR¹⁶R¹⁷, ou R¹⁶ ;
R¹⁴ et R¹⁵ sont choisis indépendamment entre H, des groupes alkyle en C₁ à C₁₂, cycloalkyle en C₃ à C₁₂, hétérocyclyle monocyclique ayant 3 à 7 membres dans le noyau, ou bicyclique ayant 6 à 10 membres dans le noyau, aryle ou hétéroaryle penta- ou hexagonal, où lesdits groupes alkyle, cycloalkyle, hétérocyclyle, aryle, hétéroaryle sont facultativement substitués avec un à quatre groupes R¹⁸ ;
R¹⁶ et R¹⁷ représentent indépendamment H, un groupe alkyle en C₁ à C₁₂, cycloalkyle en C₃ à C₁₂, hétérocyclyle monocyclique ayant 3 à 7 membres dans le noyau, ou bicyclique ayant 6 à 10 membres dans le noyau, aryle et hétéroaryle penta- ou hexagonal, où lesdits groupes alkyle, cycloalkyle, hétérocyclyle, aryle et hétéroaryle sont facultativement substitués avec un à quatre groupes R¹⁸ ;
R¹⁶ et R¹⁷ sont facultativement pris conjointement avec l'atome de N fixé pour former un noyau penta- ou hexagonal comprenant 0 à 2 hétéroatomes supplémentaires choisis entre 0, S et N, ledit noyau étant facultativement substitué avec un à quatre groupes R¹⁸ ;
chaque groupe R¹⁸ représente indépendamment H, un groupe alkyle en C₁ à C₁₂, cycloalkyle en C₃ à C₁₂, hétérocyclyle monocyclique ayant 3 à 7 membres dans le noyau ou bicyclique ayant 6 à 10 membres dans le noyau, aryle, hétéroaryle penta ou hexagonal, halogéno, CN, CF₃, -OCF₃, -NO₂, oxo, -(CR¹⁹R²⁰)ₙC(=Y')R²³, - (CR¹⁹R²⁰)ₙC(=Y')OR²³, -(CR¹⁹R²⁰)ₙC(=Y')NR²³R²⁴, -(CR¹⁹R²⁰)ₙNR²³R²⁴, -(CR¹⁹R²⁰)ₙOR²³, - (CR¹⁹R₂₀)ₙ-SR²³, -(CR¹⁹R²⁰)ₙNR²⁴C(=Y')R²³, -(CR¹⁹R²⁰)ₙNR²⁴C(=Y')OR²³, (CR¹⁹R²⁰)ₙNR²²C(=Y')NR²³R²⁴, - (CR¹⁹R²⁰)ₙNR²⁴SO₂R²³, -(CR¹⁹R²⁰)ₙOC(=Y')R²³, - (CR¹⁹R²⁰)ₙOC(=Y')NR²³R²⁴, - (CR¹⁹R²⁰)ₙNR²⁴S(O)R²³, -(CR¹⁹R²⁰)ₙS(O)₂R²³, ou -(CR¹⁹R²⁰)ₙS(O)₂NR²³R²⁴, où lesdits groupes alkyle, cycloalkyle, hétérocyclyle, aryle et hétéroaryle sont facultativement substitués avec un à quatre groupes R²¹ ;
R¹⁹ et R²⁰ représentent indépendamment H, un groupe alkyle en C₁ à C₁₂, cycloalkyle en C₃ à C₁₂, hétérocyclyle monocyclique ayant 3 à 7 membres dans le noyau ou bicyclique ayant 6 à 10 membres dans le noyau, aryle ou hétéroaryle penta- ou hexagonal, où lesdits groupes alkyle, cycloalkyle, hétérocyclyle, aryle et hétéroaryle sont facultativement substitués avec un à quatre groupes R²⁵;
R²³ et R²⁴ représentent indépendamment H, un groupe alkyle en C₁ à C₁₂, cycloalkyle en C₃ à C₁₂, hétérocyclyle monocyclique ayant 3 à 7 membres dans le noyau ou bicyclique ayant 6 à 10 membres dans le noyau, aryle ou hétéroaryle penta- ou hexagonal, où lesdits groupes alkyle, cycloalkyle, hétérocyclyle, aryle et hétéroaryle sont facultativement substitués avec un à quatre groupes R²¹ ;
R²³ et R²⁴ sont facultativement pris conjointement avec l'atome de N fixé pour former un noyau penta- ou hexagonal comprenant 0 à 2 hétéroatomes supplémentaires choisis entre 0, S et N, ledit noyau étant facultativement substitué avec un à quatre groupes R²¹
chaque groupe R²¹ représente indépendamment H, un groupe alkyle en C₁ à C₁₂, cycloalkyle en C₃ à C₁₂, hétérocyclyle monocyclique ayant 3 à 7 membres dans le noyau ou bicyclique ayant 6 à 10 membres dans le noyau, aryle, hétéroaryle penta- ou hexagonal, halogéno, CN, CF₃, -OCF₃, -NO₂, oxo, -C(=Y')R²⁵, -C(Y')OR²⁵, -C(=Y')NR²⁵R²⁶, -NR²⁵R²⁶, -OR²⁵, -SR²⁵, -NR²⁶C(=Y') R²⁵, -NR²⁶C(=Y')OR²⁵, -NR²²C(=Y')NR²⁵R²⁶, -NR²⁶SO₂R²⁵, -OC(=Y')R²⁵, -OC(Y')NR²⁵R²⁶, -S(O)R²⁵, -S(O)₂R²⁵, ou -S(O)₂NR²⁵R²⁶, où lesdits groupes alkyle, cycloalkyle, hétérocyclyle, aryle et hétéroaryle penta- ou hexagonal sont facultativement substitués avec un à quatre groupes R²⁵ ;
chacun des groupes R²⁵ et R²⁶ représente indépendamment H, un groupe alkyle en C₁ à C₁₂, cycloalkyle en C₃ à C₁₂, hétérocyclyle monocyclique ayant 3 à 7 membres dans le noyau ou bicyclique ayant 6 à 10 membres dans le noyau, aryle, ou hétéroaryle penta- ou hexagonal, où lesdits groupes alkyle, cycloalkyle, hétérocyclyle, aryle ou hétéroaryle sont facultativement substitués avec un à quatre groupes choisis entre des groupes halogéno, -CN, -OCF₃, -CF₃, -NO₂, -alkyle en C₁ à C₆, -OH, oxo, -SH, -O(alkyle en C₁ à C₆), -S(alkyle en C₁ à C₆), -NH₂, -NH(alkyle en C₁ à C₆), -N(alkyle en C₁ à C₆)₂, -SO₂(alkyle en C₁ à C₆), -CO₂H, -CO₂(alkyle en C₁ à C₆) , -C(O)NH₂, -C(O)NH(alkyle en C₁ à C₆), -C(O)N(alkyle en C₁ à C₆)₂, -N(alkyle en C₁ à C₆)C-(O)(alkyle en C₁ à C₆), -NHC(O)(alkyle en C₁ à C₆), -NHSO₂(alkyle en C₁ à C₆), -N(alkyle en C₁ à C₆)SO₂(alkyle en C₁ à C₆), -SO₂NH₂, -SO₂NH(alkyle en C₁ à C₆), -SO₂N(alkyle en C₁ à C₆)₂, -OC(O)NH₂, -OC(O)NH(alkyle en C₁ à C₆), -OC(O)N-(alkyle en C₁ à C₆)₂, -NHC(O)NH(alkyle en C₁ à C₆), -NHC(O)-N(alkyle en C₁ à C₆)₂, -N(alkyle en C₁ à C₆)C(O)NH(alkyle en C₁ à C₆), -N(alkyle en C₁ à C₆)C(O)N(alkyle en C₁ à C₆)₂, -NHC(O)NH(O)O(alkyle en C₁ à C₆) , -NHC(O)N(alkyle en C₁ à C₆)₂, -NHC(O)O(alkyle en C₁ à C₆), et -N(alkyle en C₁ à C₆)C (0) 0 (alkyle en C₁ à C₆) ;
R²⁵ et R²⁶ sont facultativement pris conjointement avec l'atome de N fixé pour former un noyau penta- ou hexagonal ayant 0 à 2 hétéroatomes supplémentaires choisis entre 0, S et N, ledit noyau étant facultativement substitué avec un à quatre groupes choisis entre des groupes halogéno, -CN, -OCF₃, -CF₃, -NO₂, alkyle en C₁ à C₆, -OH, oxo, -SH, -O(alkyle en C₁ à C₆) , -S(alkyle en C₁ à C₆) , -NH₂, -NH(alkyle en C₁ à C₆), -N(alkyle en C₁ à C₆), -SO₂(alkyle en C₁ à C₆), -CO₂H, -CO₂(alkyle en C₁ à C₆), -C(O)NH₂, -C(O)NH(alkyle en C₁ à C₆), -C(O)N(alkyle en C₁ à C₆)₂, -N(alkyle en C₁ à C₆)C(O)(alkyle en C₁ à C₆), -NHCC(O)-(alkyle en C₁ à C₆), -NHSO₂(alkyle en C₁ à C₆), -N(alkyle en C₁ à C₆)SO₂(alkyle en C₁ à C₆), -SO₂NH₂, -SO₂NH(alkyle en C₁ à C₆), -SO₂N(alkyle en C₁ à C₆)₂, -OC(O)NH₂, -OC(O)NH(alkyle en C₁ à C₆), -OC(O)N(alkyle en C₁ à C₆)₂, -NHC(O)NH(alkyle en C₁ à C₆), -NHC(O)N(alkyle en C₁ à C₆)₂, -N(alkyle en C₁ à C₆)C(O)NH(alkyle en C₁ à C₆), -N (alkyle en C₁ à C₆)C(O)N(alkyle en C₁ à C₆)₂, -NHC(O)NH(alkyle en C₁ à C₆), -NHC(O)N(alkyle en C₁ à C₆)₂, -NHC(O)O(alkyle en C₁ à C₆) et -N(alkyle en C₁ à C₆)C(O)O(alkyle en C₁ à C₆) ;
Y' représente indépendamment 0, un groupe NR²² ou S ; et
chaque groupe R²² représente indépendamment H ou un groupe alkyle en C₁ à C₅.

2. Composé suivant la revendication 1, dans lequel X représente un groupe CR².

3. Composé suivant la revendication 2, dans lequel R² représente H.

4. Composé suivant la revendication 2 ou 3, dans lequel Y représente un groupe CR⁴.

5. Composé suivant la revendication 4, dans lequel R⁴ représente H.

6. Composé suivant la revendication 4 ou 5, dans lequel Z représente un groupe C^{7a}.

7. Composé suivant la revendication 6, dans lequel R^{7a} représente H.

8. Composé suivant la revendication 4 ou 5 dans lequel Z représente N.

9. Composé suivant l'une quelconque des revendications 6 à 8, dans lequel W représente un groupe CR^{8a}.

10. Composé suivant la revendication 9, dans lequel R^{8a} représente H.

11. Composé suivant la revendication 9 ou 10, dans lequel R³ représente un groupe Br.

12. Composé suivant la revendication 9 ou 10, dans lequel R³ représente un groupe R⁹.

13. Composé suivant la revendication 12, dans lequel R⁹ représente un groupe alcynyle en C₂ à C₃, aryle en C₆, ou hétéroaryle monocyclique penta- ou hexagonal ou bicyclique octo- à décagonal ayant 1 ou 2 atomes de noyau choisis entre N, 0 et S ; et où chaque membre de R⁹ est substitué indépendamment avec un ou deux groupes R¹⁰.

14. Composé suivant la revendication 13, dans lequel R⁹ représente un groupe propynyle, phényle, pyrazolyle, pyridyle, pyrimidinyle, thiényle, furannyle, imidazolyle ou benzothiényle, où chaque membre de R⁹ est substitué indépendamment avec un ou deux groupes R¹⁰.

15. Composé suivant la revendication 14, dans lequel R⁹ représente un groupe phényle substitué avec un ou deux groupes R¹⁰_{.}

16. Composé suivant la revendication 14 ou 15, dans lequel R¹⁰ représente un groupe halogéno, R¹¹, -OR¹¹, CN, -CF₃, -OCF₃, -NR¹²C(=O)R¹¹, -NR¹²S(O)_{q}R¹¹, -SR¹¹, -NR¹¹R¹², -C(=O)NR¹¹R¹², -S(O)_{q}R¹¹, ou -S(O)₂NR¹¹R¹², dans lequel R¹¹ et R¹² sont facultativement pris conjointement avec l'atome de N fixé pour former un noyau tétra- à heptagonal comprenant 0 à 2 hétéroatomes supplémentaires choisis entre 0, S, et N, ledit noyau étant facultativement substitué avec un à quatre groupes R¹³_{.}

17. Composé suivant la revendication 16, dans lequel R¹⁰ représente un groupe R¹¹.

18. Composé suivant la revendication 17, dans lequel R¹¹ représente un groupe alkyle en C₁ à C₆, ou hétérocyclyle monocyclique penta- ou hexagonal ou bicyclique ortho- à décagonal comprenant un ou deux hétéroatomes choisis entre N et 0, où lesdits groupes alkyle et hétérocyclyle sont facultativement substitués avec un à quatre groupes R¹³, où deux groupes R¹³ géminés sont facultativement pris conjointement avec l'atome auquel ils sont fixés pour former un noyau hexagonal comprenant 0 à 2 hétéroatomes choisis entre 0, S et N, ledit noyau étant facultativement substitué avec un à quatre groupes R¹⁸.

19. Composé suivant la revendication 18, dans lequel R¹¹ représente un groupe alkyle en C₁ à C₆, ledit groupe alkyle étant facultativement substitué avec un ou deux groupes R¹³ et chaque groupe R¹³ représente indépendamment un groupe halogéno, CN, CF₃, -OCF₃, oxo, - (CR¹⁴R¹⁵)ₙC(O)OR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, - (CR¹⁴R¹⁵)ₙNR¹⁶C(O)R¹⁷, -(CR¹⁴R¹⁵)ₙS(O)₂NR¹⁶R¹⁷, ou R¹⁶.

20. Composé suivant la revendication 18, dans lequel R¹¹ représente un groupe hétérocyclyle monocyclique penta ou hexagonal ou bicyclique octo- à décagonal comprenant 1 ou 2 hétéroatomes choisis entre N et 0, où lesdits groupes alkyle et hétérocyclyle sont facultativement substitués avec un ou deux groupes R¹³ et où chaque groupe R¹³ représente indépendamment un groupe halogéno, CN, CF₃, -OCF₃, oxo, -(CR¹⁴R¹⁵)ₙC(O)OR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷, -(CR₁₄R¹⁵)ₙS(O)₂NR¹⁶R¹⁷, ou R¹⁶.

21. Composé suivant la revendication 16, dans lequel R¹⁰ représente un groupe -OR¹¹.

22. Composé suivant la revendication 21, dans lequel R¹¹ représente H, un groupe alkyle en C₁ à C₆, ou hétérocyclyle monocyclique penta ou hexagonal ou bicyclique octo- à décagonal comprenant un ou deux atomes d'azote, où ledit alkyle ou hétérocyclyle est facultativement substitué avec un ou deux groupes R¹³, chaque groupe R¹³ représente indépendamment un groupe halogéno, CN, CF₃, -OCF₃, oxo, -(CR¹⁴R¹⁵)ₙC(O)OR¹⁶, -(CR¹⁴R¹⁵)ₙC(O)NR¹⁶R¹⁷, -(CR¹⁴R¹⁵)ₙNR¹⁶R¹⁷, -(CR¹⁴R¹⁵)ₙOR¹⁶, -(CR¹⁴R¹⁵)ₙNR¹⁶C(O)R¹⁷, -(CR¹⁴R¹⁵)ₙS(O)₂NR¹⁶R¹⁷, ou R¹⁶.

23. Composé suivant l'une quelconque des revendications 11 à 22, dans lequel R⁵ représente H.

24. Composé suivant l'une quelconque des revendications 1 à 22, dans lequel R⁵ représente un groupe

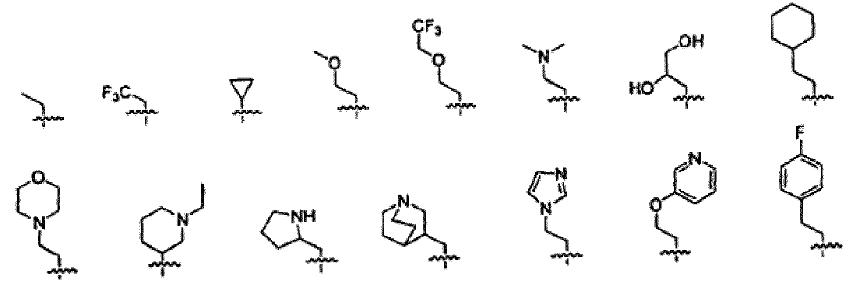

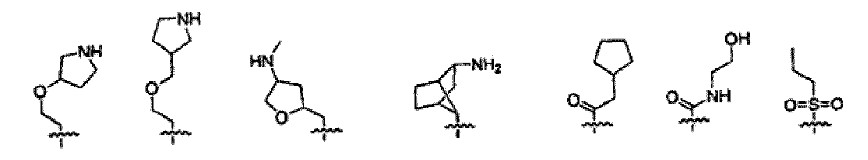

25. Composé suivant la revendication 23, dans lequel R⁶ représente un groupe CN, halogèno, -C(O)NR¹¹R¹², -OR¹¹, - NR¹¹R¹², NR¹²C(O)R¹¹, alkyle en C₁ à C₃, cycloalkyle en C₃ à C₆, hétérocyclyle penta- ou hexagonal comprenant 1 ou 2 hétéroatomes, aryle en C₆, ou hétéroaryle penta- ou hexagonal comprenant 1 ou 2 hétéroatomes ; où ledit groupe alkyle est substitué avec un ou deux groupes R¹³ à l'exception de H ; et ledit groupe cycloalkyle, aryle, hétérocyclyle ou hétérocyclyle est facultativement substitué avec un ou deux groupes R¹³ ; où les hétéroatomes sont choisis entre N, 0 et S ; où chaque groupe R¹² représente H ou un groupe alkyle en C₁ à C₃, et chaque groupe R¹¹ représente indépendamment H ou un groupe alkyle en C₁ à C₃ facultativement substitué avec un ou deux groupes R¹³.

26. Composé suivant la revendication 25, dans laquelle R⁶ représente un groupe CN.

27. Composé suivant la revendication 25, dans lequel R⁶ représente un groupe pyridyle ou pyrazolyle facultativement substitué avec un substituant méthyle.

28. Composés suivant la revendication 1, qui sont choisis dans le groupe consistant en :
3-bromo-6-pyridine-3-yl-7,9-dihydro-1,5,7,9-fluorène-8-one,
3-bromo-6-(1-méthyl-1H-pyrazole-4-yl)-7,9-dihydro-1,5,7,9-dihydro-tétra-aza-fluorène-8-one,
3-bromo-6-[1-(4-méthoxy-benzyl)-1H-pyrazole-4-yl]-7,9-dihydro-1,5,7,9-tétra-aza-fluorène-8-one,
3-bromo-6-(1-méthyl-1H-pyrazole-3-yl)-7,9-dihydro-1,5,7,9-tétra-aza-fluorène-8-one,
3-bromo-6-(2-méthyl-2H-pyrazole-3-yl)-7,9-dihydro-1,5,7,9-tétra-aza-fluorène-8-one,
3-[4-(4-méthyl-pipérazine-1-yl)-phényl]-6-pyridine-3-yl-7,9-dihydro-1,5,7,9-tétra-aza-fluorène-8-one,
3-[4-(4-méthyl-pipérazine-1-yl)-phényl]-6-(1-méthyl-1H-pyrazole-4-yl)-7,9-dihydro-1,5,7,9-tétra-aza-fluorène-8-one,
6-[1-(4-méthoxy-benzyl)-1H-pyrazole-4-yl]-3-[4-(4-méthyl-pipérazine-1-yl)-phényl]-7,9-dihydro-1,5,7,9-tétra-aza-fluorène-8-one,
3-[4-(4-méthyl-pipérazine-1-yl)-phényl]-6-(1-méthyl-1H-pyrazole-3-yl)-7,9-dihydro-1,5,7,9-tétra-aza-fluorène-8-one,
3-[4-(4-méthyl-pipérazine-1-yl)-phényl]-6-(2-méthyl-2H-pyrazole-3-yl)-7,9-dihydro-1,5,7,9-tétra-aza-fluorène-8-one,
6-(1-méthyl-1H-pyrazole-4-yl)-3-(4-pipéridine-1-ylméthyl-phényl)-7,9-dihydro-1,5,7,9-tétra-aza-fluorène-8-one,
8-chloro-3-[4-(4-méthyl-pipérazine-1-yl)phényl]-6-pyridine-3-yl-9H-1,5,7,9-tétra-aza-fluorène,
8-chloro-3-[4-(4-méthyl-pipérazine-1-yl)phényl]-6-(1-méthyl-1H-pyrazole-4-yl)-9H-1,5,7,9-tétra-aza-fluorène,
8-chloro-3-[4-(4-méthyl-pipérazine-1-yl)phényl]-6-(1H-pyrazole-4-yl)-9H-1,5,7,9-tétra-aza-fluorène,
8-chloro-3-[4-(4-méthyl-pipérazine-1-yl)phényl]-6-(2-méthyl-2H-pyrazole-3-yl)-9H-1,5,7,9-tétra-aza-fluorène,
8-chloro-3-[4-(4-méthyl-pipérazine-1-yl)phényl]-6-(2-méthyl-2H-pyrazole-3-yl)-9H-1,5,7,9-tétra-aza-fluorène,
8-chloro-6-(1-méthyl-1H-pyrazole-4-yl)-3-(4-pipéridine-1-ylméthyl-phényl)-9H-1,5,7,9-tétra-aza-fluorène,
3-[4-(4-méthyl-pipérazine-1-yl)-phényl]-6-pyridine-3-yl-9H-1,5,7,9-tétra-aza-fluorène,
3-[4-(4-méthyl-pipérazine-1-yl)-phényl]-6-(1-méthyl-1H-pyrazole-4-yl)-9H-1,5,7,9-tétra-aza-fluorène,
3-[4-(4-méthyl-pipérazine-1-yl)-phényl]-6-(1H-pyrazole-4-yl)-9H-1,5,7,9-tétra-aza-fluorène,
3-[4-(4-méthyl-pipérazine-1-yl)-phényl]-6-(1-méthyl-1H-pyrazole-3-yl)-9H-1,5,7,9-tétra-aza-fluorène,
3-[4-(4-méthyl-pipérazine-1-yl)-phényl]-6-(2-méthyl-2H-pyrazole-3-yl)-9H-1,5,7,9-tétra-aza-fluorène,
6-(1-méthyl-1H-pyrazole-4-yl)-3-(4-pipéridine-1-ylméthyl-phényl)-9H-1,5,7,9-tétra-aza-fluorène,
8-méthyl-3-[4-(4-méthyl-pipérazine-1-yl)phényl]-6-(1-méthyl-1H-pyrazole-4-yl)-9H-1,5,7,9-tétra-aza-fluorène, et
8-[4-(4-méthyl-pipérazine-1-yl)phényl]-5H-dipyrido[2,3-b ;2',3'-d]pyrrole-2-carbonitrile.

29. Composition pharmaceutique comprenant un composé de l'une quelconque des revendications 1 à 28 et un support pharmaceutiquement acceptable.

30. Composition pharmaceutique suivant la revendication 29, comprenant en outre un second agent chimiothérapeutique.

31. Composition pharmaceutique suivant la revendication 30, dans laquelle ledit second agent thérapeutique est un agent d'altération de l'ADN.

32. Composé suivant l'une quelconque des revendications 1 à 28, ou composition pharmaceutique suivant l'une quelconque des revendications 29 et 30, pour une utilisation dans une méthode pour inhiber une croissance cellulaire anormale ou pour traiter un trouble hyperprolifératif chez un mammifère.

33. Composé suivant l'une quelconque des revendications 1 à 28, ou composition pharmaceutique suivant l'une quelconque des revendications 29 et 30, pour une utilisation dans une méthode du traitement du cancer chez un mammifère.

34. Composé suivant la revendication 33, dans lequel le cancer est choisi entre le cancer du sein, le cancer colorectal, le cancer des ovaires, le cancer pulmonaire non à petites cellules, des tumeurs cérébrales malignes, des sarcomes, un mélanome, un lymphome, des myélomes et une leucémie.

35. Composé suivant la revendication 31, dans lequel ledit second agent chimiothérapeutique est administré audit mammifère séquentiellement ou conjointement avec ledit ou lesdits composés.

36. Utilisation d'un composé suivant l'une quelconque des revendications 1 à 28 dans la préparation d'un médicament pour le traitement du cancer.

37. Utilisation suivant la revendication 36, dans laquelle le cancer est choisi entre le cancer du sein, le cancer colorectal, le cancer des ovaires, le cancer pulmonaire non à petites cellules, des tumeurs cérébrales malignes, des sarcomes, un mélanome, un lymphome, des myélomes et une leucémie.
